(19) Europäisches Patentamt
European Patent Office
Office européen des brevets

(11) Veröffentlichungsnummer: **0 252 879 B1**

# (12) EUROPÄISCHE PATENTSCHRIFT

(45) Veröffentlichungstag der Patentschrift: **06.05.92**

(51) Int. Cl.[5]: **C07D 493/22**, C07F 7/08, A01N 43/90, //(C07D493/22, 313:00,311:00,311:00,307:00)

(21) Anmeldenummer: **87810369.6**

(22) Anmeldetag: **26.06.87**

Die Akte enthält technische Angaben, die nach dem Eingang der Anmeldung eingereicht wurden und die nicht in dieser Patentschrift enthalten sind.

## (54) Pestizide.

(30) Priorität: **02.07.86 CH 2668/86**

(43) Veröffentlichungstag der Anmeldung:
**13.01.88 Patentblatt 88/02**

(45) Bekanntmachung des Hinweises auf die Patenterteilung:
**06.05.92 Patentblatt 92/19**

(84) Benannte Vertragsstaaten:
**AT BE CH DE ES FR GB GR IT LI LU NL SE**

(56) Entgegenhaltungen:
**EP-A- 0 184 173**
**GB-A- 2 168 345**

(73) Patentinhaber: **CIBA-GEIGY AG**
**Klybeckstrasse 141**
**CH-4002 Basel(CH)**

(72) Erfinder: **O'Sullivan, Anthony Cornelius, Dr.**
**Habsburgerstrasse 38**
**CH-4055 Basel(CH)**
Erfinder: **Frei, Bruno, Dr.**
**Bündtenstrasse 16**
**CH-4410 Liestal(CH)**

**EP 0 252 879 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue 13β-Milbemycin-Derivate der Formel I, ihre Herstellung und ihre Verwendung zur Bekämpfung von Schädlingen, ferner Schädlingsbekämpfungsmittel, die mindestens eine dieser Verbindungen als Wirkstoff enthalten.

Die neuen Verbindungen entsprechen der allgemeinen Formel I

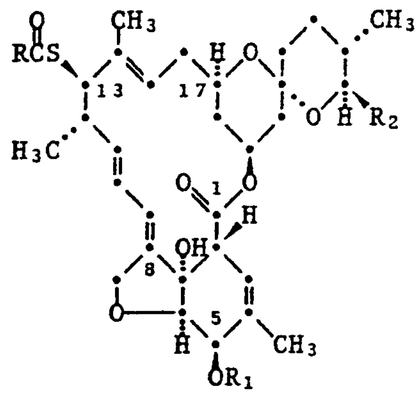

(I)

in welcher

$R_1$ Wasserstoff, eine Silyl- oder Acylgruppe,

$R_2$ Methyl, Ethyl, Isopropyl oder sek.Butyl und

R Wasserstoff,
eine geradkettige oder verzweigte $C_1$-$C_{18}$-Alkylgruppe, welche unsubstituiert oder durch 1 bis 7 Halogenatome, 1 bis 6 $C_1$-$C_6$-Alkoxygruppen, eine unsubstituierte oder halogenierte phenoxygruppe, eine $C_1$-$C_4$-Alkylthiogruppe, eine $C_1$-$C_4$-Alkylcarbonyl-gruppe oder eine unsubstituierte oder durch Methyl ein- oder mehrfach substituierte $C_3$-$C_6$-Cycloalkylgruppe substituiert ist,
eine $C_2$-$C_6$-Alkenylgruppe, welche unsubstituiert oder durch 1 bis 7 Halogenatome oder 1 bis 6 $C_1$-$C_6$-Alkoxygruppen substituiert ist,
eine $C_2$-$C_6$-Alkinylgruppe, welche unsubstituiert oder durch 1 bis 7 Halogenatome oder 1 bis 6 $C_1$-$C_6$-Alkoxygruppen substituiert ist,
eine mono- bis tetracyclische cycloaliphatische Gruppe mit 3 bis 10 Kohlenstoffatomen oder eine $C_3$-$C_{10}$-Cycloalkylgruppe, welche durch 1 bis 7 Halogenatome, 1 bis 6 $C_1$-$C_6$-Alkoxygruppen oder durch $C_1$-$C_4$-Alkylgruppen substituiert ist, oder 2,2-Dimethyl-3-(2,2-dichlorvinyl)-cyclopropyl, eine Phenylgruppe, welche unsubstituiert oder durch 1 bis 3 Substituenten aus der Gruppe bestehend aus Halogenatomen, $C_1$-$C_6$-Alkyl, $C_1$-$C_6$-Alkoxy, $C_1$-$C_4$-Alkylthio, Phenyl, Nitro, Halogen-$C_1$-$C_4$-alkyl und Amin oder durch eine Difluormethylendioxygruppe, deren Sauerstoffatome an zwei benachbarten Kohlenstoffatomen sitzen, substituiert ist, oder eine Benzylgruppe, welche unsubstituiert oder durch 1 bis 3 Substituenten aus der Gruppe bestehend aus Halogenatomen, $C_1$-$C_6$-Alkyl, $C_1$-$C_6$-Alkoxy, $C_1$-$C_4$-Alkylthio, Phenyl, Nitro, Halogen-$C_1$-$C_4$-alkyl und bin substituiert ist, wobei die beiden letztgenannten Substituenten nur an Ringkohlenstoffatomen vorliegen können,

bedeuten.

Unter den vorgenannten Bedeutungen für R sind als bevorzugt anzusehen: unsubstituiertes oder halogeniertes $C_1$-$C_8$-Alkyl, unsubstituiertes oder durch Methyl ein- oder mehrfach substituiertes $C_3$-$C_6$-Cycloalkyl, Adamantyl, unsubstitutiertes oder halogeniertes $C_2$-$C_6$-Alkenyl und $C_2$-$C_6$-Alkinyl.

Als Substituenten der Alkyl-, Cycloalkyl-, Alkenyl- und Alkinyl-Gruppen kommen beispielsweise 1 bis 7 Halogenatome oder 1 bis 6 $C_1$-$C_6$-Alkoxygruppen und als Substituenten der Phenyl- und Benzyl-Gruppen 1 bis 3 Substituenten ausgewählt aus der Gruppe bestehend aus Halogenatomen, $C_1$-$C_6$-Alkyl, $C_1$-$C_6$-Alkoxy, $C_1$-$C_4$-Alkylthio, Phenyl oder Nitro in Betracht. Benzylgruppen können sowohl am Ring wie am aliphatischen Kohlenstoffatom substituiert sein wie beispielsweise α-Methyl- und α,α-Dimethylbenzyl. R kann auch eine Alkylgruppe darstellen, welche einen der nachfolgend genannten Substituenten trägt: Eine unsubstituierte oder substituierte Phenoxygruppe, wie beispielsweise eine halogenierte, insbesondere eine durch 1 bis 3 Halogenatome substituierte Phenoxygruppe, eine $C_1$-$C_4$-Alkylthiogruppe, eine $C_1$-$C_4$-Alkylcarbonylgruppe oder $C_3$-$C_6$-Cycloalkyl, welches durch Methyl ein- oder mehrfach substituiert sein kann. Als Substituenten der unmittelbar mit der Carbonylthiogruppe verknüpften Cycloalkylgruppen können als Substituenten ausser

den weiter oben bereits genannten auch $C_1$-$C_4$-Alkylgruppen vorliegen. Aromatische Ringe können ferner als Substituenten auch Halogen-$C_1$-$C_4$-alkyl und Amin aufweisen, wobei die Substituenten gleich oder verschieden sein können oder nur einer der Substituenten vorliegt, und die Phenylgruppe kann auch durch eine Difluormethylendioxygruppe substituiert sein, wobei die beiden Sauerstoffatome an zwei benachbarten Ringkohlenstoffatomen sitzen.

Unter dem Begriff Alkyl als Substituent oder Teil eines Substituenten sind je nach Zahl der angegebenen Kohlenstoffatome beispeislweise folgende Gruppen zu verstehen: Methyl, Ethyl, Propyl, Butyl, Pentyl, Hexyl, Heptyl, Octyl, Nonyl oder Decyl, sowie die Isomeren, wie beispielsweise Isopropyl, Isobutyl, tert.-Butyl oder Isopentyl. Halogenalkyl steht für einen einfach bis perhalogenierten Alkylsubstituenten, wie beispielsweise $CHCl_2$, $CHF_2$, $CH_2Cl$, $CCl_3$, $CF_3$, $CH_2F$, $CH_2CH_2Cl$, $CHBr_2$. Unter Halogen soll hier und im folgenden Fluor, Chlor, Brom oder Jod, vorzugsweise Fluor, Chlor oder Brom, verstanden werden. Als cycloaliphatische Gruppen kommen mono-bis tetracylische Gruppen in Betracht, wie beispielsweise Cyclopropyl, Cyclobutyl, Cyclopentyl, Cyclohexyl, Decalin, Hydrindan, Bicycloheptan, Bicyclooctan, Norbornan, Bornan oder Adamantyl. Diese cycloaliphatischen Gruppen sind vorzugsweise unsubstituiert oder durch Methyl ein- oder mehrfach substituiert. Alkenyl steht für einen mindestens durch eine C=C-Doppelbindung charakterisierten aliphatischen, acyclischen Kohlenwasserstoffrest, wie beispielsweise für Vinyl, Propenyl-(1), Allyl, Butenyl-(1), Butenyl-(2) oder Butenyl-(3). Halogenalkenyl bedeutet dementsprechend einen derartigen Alkenylrest, der ein- oder mehrfach halogeniert ist. Alkinyl steht für eine gerade oder verzweigte Kohlenstoffkette, die durch mindestens eine C≡C Dreifachbindung charakterisiert ist. Typische Vertreter sind beispielsweise Ethinyl, Propinyl-(1), Propargyl oder Butinyl-(1). Alkoxyalkyl steht für eine unverzweigte oder verzweigte, durch ein Sauerstoffatom unterbrochene. Alkylgruppe, beispielsweise für $CH_2OCH_3$, $CH_2CH_2OCH_3$, $CH_2CH(CH_3)OCH_3$, $CH_2OC_2H_5$, $C(CH_3)_2OCH_3$, $CH_2OC_3H_7$-i oder $CH_2CH_2CH_2OCH_3$. Diese Ethergruppen lassen sich - bei Austausch von O durch S - auch als Vertreter von Alkylthioalkylgruppen anführen.

Als substituiertes Phenyl kommen beispielsweise 2,4-Dichlorphenyl, 2,3,6-Trichlorphenyl, p-Bromphenyl, 2,4-Xylyl, 3-Nitrophenyl, o-(Trifluormethyl)-phenyl, 4-Chlor-2-methylphenyl, 4-Methyl-2-methoxyphenyl, 2,4,6-Trimethylphenyl, p-Aminophenyl oder p-Methylthiophenyl in Betracht.

Als Beispiele für R, die keine Limitierung darstellen, sind zu nennen: Wasserstoff, Methyl, Ethyl, Propyl, Isopropyl, n-Butyl, sek.Butyl, tert.Butyl, Neopentyl, Chlormethyl, Trifluormethyl, Trichlormethyl, Trichlorethyl, Trichlor-tert.Butyl, 1,2,2,2-Tetrachlorethyl, 1,3,3,3-Tetrachlorpropyl, 3-Chlorpropyl, Ethenyl, Propenyl, Propinyl, Methoxymethyl, Isopropoxymethyl, 1-Methyl-1-methoxyethyl, 2,2-Dimethylvinyl, 1,2,2-Trichlorvinyl, 1,3,3,3-Tetrachlorpropyl, 1,1-Dichlor-2,2,2-trifluorethyl, 1,3-Pentadienyl, Ethinyl, 1-Propinyl, 1-Butinyl, Cyclopropyl, 2,2-Dimethylcyclopropyl, 1-Methylcyclopropyl, 2,2-Dimethyl-3-(2,2-dichlorvinyl)-cyclopropyl, Cyclobutyl, Cyclopentyl, Cyclohexyl, Adamantyl, Phenyl, Benzyl, p-Tolyl, p-Chlorphenyl, 2,6-Dichlorphenyl oder 2,4-Dinitrophenyl oder 4-Fluorphenoxymethyl.

Verbindungen der Formel I, worin $R_1$ Wasserstoff bedeutet, sind bevorzugt. Acyl- und Silylgruppen als $R_1$ sind im allgemeinen als Schutzgruppen aufzufassen. Geeignete Acylgruppen sind beispielsweise die Reste $R_5$-C(O)-, wobei $R_5$ für $C_1$-$C_{10}$-Alkyl, $C_1$-$C_{10}$-Haloalkyl oder einen unsubstituierten oder durch Substituenten der Gruppe bestehend aus Halogen, $C_1$-$C_3$-Alkyl, $C_1$-$C_3$-Haloalkyl, $C_1$-$C_3$-Alkoxy, $C_1$-$C_3$-Haloalkoxy, Cyano und Nitro substituierten Rest aus der Gruppe Phenyl und Benzyl steht und vorzugsweise $C_1$-$C_6$-Alkyl, $C_1$-$C_6$-Haloalkyl oder unsubstituiertes oder durch Halogen, $C_1$-$C_3$-Alkyl, $CF_3$ oder Nitro substituiertes Phenyl bedeutet. Besonders bevorzugt ist die Acetylgruppe. Als Acylverbindung ist beispielsweise 5-O-Acetyl-13$\beta$-Formylthio-milbemycin $A_4$ zu nennen. Als geeignete Silylgruppen für $R_1$ kommt der Rest -Si($R_6$)($R_7$)($R_8$) in Frage, wobei $R_6$, $R_7$ und $R_8$ vorzugsweise unabhängig voneinander für $C_1$-$C_4$-Alkyl, Benzyl oder Phenyl stehen und beispielsweise eine der Gruppen Trimethylsilyl, Diphenyl-tert.butylsilyl, bis-(Isopropyl)methylsilyl oder Triphenylsilyl und vorzugsweise tert.Butyldimethylsilyl bilden.

Die vorliegende Erfindung erstreckt sich sowohl auf die einzelnen Diastereomere wie auch auf Diastereomerengemische von Verbindungen der Formel I.

Verbindungen, worin $R_2$ sek.Butyl darstellt, sollen hier und im folgenden gleichfalls zu den Milbemycin-Derivaten gerechnet werden, obwohl sie nach der üblichen Systematik von Avermectin-Derivaten abgeleitet sind. Avermectin-Aglykone (mit einer OH-Gruppe in 13$\alpha$-Position) lassen sich jedoch gemäss US-PS 4,173,571 in Milbemycin-Homologe überführen.

In natürlich vorkommenden Milbemycinen ($R_1$ = H; $R_2$ = $CH_3$, $C_2H_5$ oder iso $C_3H_7$) ist die 13-Position anstelle der Estergruppe der erfindungsgemässen Verbindungen der Formel I ausschliesslich mit Wasserstoff besetzt, wie die nachstehende Formel zeigt:

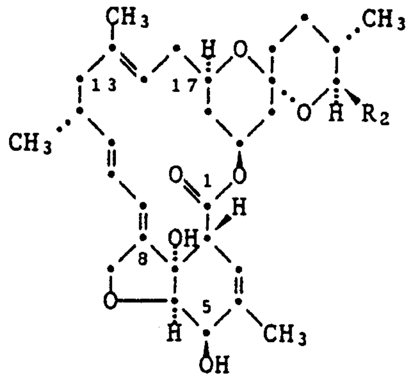

$R_2$ = $CH_3$ Milbemycin $A_3$ (US-PS 3,950,360)
$R_2$ = $C_2H_5$ Milbemycin $A_4$ (US-PS 3,950,360)
$R_2$ = iso$C_3H_7$ Milbemycin D (US-PS 4,346,171)
$R_2$ = sec.$C_4H_9$ 13-Deoxi-22,23-dihydro-C-076-Bla-aglycon.
(US-PS 4,173,571, GB-PS 1,573,955 und DE-OS 2717040).

Bei Avermectinen dagegen steht in der 13-Position ein α-L-Oleandrosyl-α-L-oleandrose-Rest, der über Sauerstoff in α-Konfiguration mit dem Makrolid-Molekül verknüpft ist. Avermectine unterscheiden sich strukturell ausserdem durch eine 23-OH-Gruppe oder $\Delta^{22,23}$-Doppelbindung und in der Regel durch einen Substituenten $R_2$ = sek.$C_4H_9$ von den Milbemycinen. Durch Hydrolyse des Zucker-Restes der Avermectine gelangt man leicht zu den entsprechenden Avermectin-Aglykonen, die eine in Nachbarstellung zu einer C=C-Doppelbindung befindliche 13α-Hydroxy-Gruppe besitzen. Die Avermectin-Aglykone sind, wie vorstehend angegeben, in die Milbemycin-Homologen umwandelbar. Bei den Milbemycin-Derivaten der vorliegenden Anmeldung liegt die $\Delta^{22,23}$-Doppelbindung stets in hydrierter Form vor und der Substituent in 13-Position ist immer β-ständig.

Folgende Untergruppen von Verbindungen der Formel I sind aufgrund ihrer ausgeprägten parasitiziden und insektiziden Wirkung besonders bevorzugt:

Gruppe Ia: Verbindungen der Formel I, worin $R_1$ Wasserstoffdarstellt, $R_2$ für Methyl, Ethyl, Isopropyl oder sek.Butyl steht und R folgende Bedeutungen hat:

- Wasserstoff, unsubstituiertes oder durch 1 bis 4 Halogenatome oder $C_1$-$C_4$-Alkoxy substituiertes $C_1$-$C_6$-Alkyl, $C_2$-$C_4$-Alkenyl, $C_2$-$C_4$-Alkinyl oder $C_3$-$C_6$-Cycloalkyl;
- unsubstituiertes oder ein bis dreifach durch Halogen, $C_1$-$C_4$-Alkyl, $C_1$-$C_4$-Alkoxy, $C_1$-$C_4$-Alkylthio oder Nitro substituiertes Phenyl;

Gruppe Ib: Verbindungen der Formel I, worin $R_1$ Wasserstoff darstellt, $R_2$ für Methyl, Ethyl, Isopropyl oder sek.Butyl steht und R folgende Bedeutungen hat:

- Wasserstoff, unsubstituiertes oder durch 1 bis 4 Chlor- oder Fluoratome oder Methoxy substituiertes $C_1$-$C_4$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxy, $C_1$-$C_2$-Alkylthio oder Nitro substituiertes Phenyl;

Gruppe Ic: Verbindungen der Formel I, worin $R_1$ Wasserstoff darstellt, $R_2$ für Methyl oder Ethyl steht und R folgende Bedeutungen hat:

- Wasserstoff, unsubstituiertes oder durch 1 bis 4 Chlor- oder Fluoratome oder Methoxy substituiertes $C_1$-$C_4$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxy, $C_1$-$C_2$-Alkylthio oder Nitro substituiertes Phenyl;

Gruppe Id: Verbindungen der Formel I, worin $R_1$ Wasserstoff darstellt, $R_2$ für Isopropyl oder sek.Butyl steht und R folgende Bedeutungen hat:

- Wasserstoff, unsubstituiertes oder durch 1 bis 4 Chlor- oder Fluoratome oder Methoxy substituiertes $C_1$-$C_4$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxy, $C_1$-$C_2$-Alkylthio oder Nitro substituiertes Phenyl;

Gruppe Ie: Verbindungen der Formel I, worin $R_1$ Wasserstoff darstellt, $R_2$ für Methyl, Ethyl, Isopropyl oder sek.Butyl steht und R folgende Bedeutungen hat:

- Wasserstoff, unsubstituiertes oder durch 1 bis 3 Chloratome oder Fluoratome oder Methoxy substituiertes $C_1$-$C_4$-Alkyl, $C_2$-$C_3$-Alkenyl, $C_2$-$C_3$-Alkinyl oder $C_3$-$C_6$-Cycloalkyl;
- unsubstituiertes oder durch Chlor, Fluor, $C_1$-$C_2$-Alkyl, $C_1$-$C_2$-Alkoxy, $C_1$-$C_2$-Alkylthio oder Nitro substituiertes Phenyl;

Gruppe If: Verbindungen der Formel I, worin $R_1$ Wasserstoff darstellt, $R_2$ für Methyl oder Ethyl steht und R folgende Bedeutungen hat:

- Wasserstoff, unsubstituiertes oder durch 1 bis 3 Chloratome oder Fluoratome oder Methoxy substituiertes $C_1$-$C_4$-Alkyl, $C_2$-$C_3$-Alkenyl, $C_2$-$C_3$-Alkinyl oder $C_3$-$C_6$-Cycloalkyl;

Gruppe Ig: Verbindungen der Formel I, worin $R_1$ Wasserstoff darstellt, $R_2$ für Isopropyl oder sek.Butyl steht und R folgende Bedeutungen hat:

- Wasserstoff, unsubstituiertes oder durch 1 bis 3 Chloratome oder Fluoratome oder Methoxy substituiertes $C_1$-$C_4$-Alkyl, $C_2$-$C_3$-Alkenyl, $C_2$-$C_3$-Alkinyl oder $C_3$-$C_6$-Cycloalkyl.

Gruppe Ih: Verbindungen der Formel I, worin $R_1$ Wasserstoff darstellt, $R_2$ für Ethyl oder Isopropyl steht und R folgende Bedeutungen hat: Wasserstoff, $C_1$-$C_8$-Alkyl, welches unsubstituiert oder durch $C_1$-$C_4$-Alkoxy oder ein- bis dreifach halogeniertes Phenoxy monosubstituiert oder durch 1 bis 5 Halogenatome substituiert ist, eine unsubstituierte oder durch Substituenten der Gruppe bestehend aus $C_1$-$C_4$-Alkyl und halogeniertem $C_2$-$C_4$-Alkenyl ein- oder mehrfach substituierte, mono- bis tetracyclische, aliphatische Gruppe mit insgesamt 3 bis 10 Kohlenstoffatomen im Ring oder Ringsystem, ein- bis dreifach halogeniertes $C_2$-$C_4$-Alkenyl, $C_3$-$C_4$-Alkinyl oder ein- bis dreifach durch Substituenten der Gruppe bestehend aus Halogen, $C_1$-$C_4$-Alkyl und Nitro substituiertes Phenyl.

Gruppe Ii: Verbindungen der Formel I, worin $R_1$ Wasserstoff darstellt, $R_2$ für Ethyl oder Isopropyl steht und R folgende Bedeutungen hat: Wasserstoff, $C_1$-$C_8$-Alkyl, welches unsubstituiert oder durch Substituenten der Gruppe bestehend aus Chlor und Fluor ein- bis dreifach substituiert ist, Fluorphenoxymethyl, unsubstituiertes oder durch eine Methylgruppe substituiertes $C_3$-$C_4$-Cycloalkyl, Adamantyl, Trichlorvinyl oder Monochlorphenyl.

Gruppe Ik: Verbindungen der Formel I, worin $R_1$ Wasserstoff darstellt, $R_2$ für Methyl oder Ethyl steht und R folgende Bedeutungen hat: $C_1$-$C_7$-Alkyl, durch Chlor, Fluor, Methoxy, Methylthio, Dimethylcyclohexyl oder Acetyl monosubstituiertes $C_1$-$C_4$-Alkyl, Phenyl, welches durch Trifluormethyl oder die Gruppe $-O-CF_2-O-$ monosubstituiert ist, wobei die beiden Sauerstoffatome an zwei benachbarten Ringkohlenstoffatomen sitzen, durch Substituenten der Gruppe bestehend aus Chlor und Methyl 1-3fach substituiertes Phenyl oder unsubstituiertes oder durch 1 bis 3 Substituenten der Gruppe bestehend aus Amino, Halogen, $C_1$-$C_6$-Alkyl, $C_1$-$C_6$-Alkoxy, $C_1$-$C_4$-Alkylthio, Phenyl und Nitro substituiertes $\alpha$-Methyl- oder $\alpha,\alpha$-Dimethylbenzyl.

Gruppe Il: Verbindungen der Formel I, worin $R_1$ Wasserstoff darstellt, $R_2$ für Methyl oder Ethyl, vorzugsweise für Ethyl, steht und R folgende Bedeutungen hat: $C_1$-$C_7$-Alkyl, welches unsubstituiert oder durch Halogen oder $C_1$-$C_4$-Alkoxy monosubstituiert ist, Phenyl, welches durch Trifluormethyl oder die Gruppe $-O-CF_2-O-$ monosubstituiert ist, wobei die beiden Sauerstoffatome an zwei benachbarten Ringkohlenstoffatomen sitzen, $\alpha$-Methyl- oder $\alpha,\alpha$-Dimethylbenzyl.

Gruppe Im: Verbindungen der Formel I, worin $R_1$ Wasserstoff darstellt, $R_2$ für Methyl oder Ethyl, vorzugsweise für Ethyl, steht und R folgende Bedeutungen hat: $C_1$-$C_7$-Alkyl, durch Chlor, Fluor oder Methoxy monosubstituiertes $C_1$-$C_4$-Alkyl, durch Trifluormethyl oder die Gruppe $-O-CF_2-O-$ monosubstituiertes Phenyl, wobei die beiden Sauerstoffatome an zwei benachbarten Ringkohlenstoffatomen sitzen, oder $\alpha$-Methyl- oder $\alpha,\alpha$-Dimethylbenzyl.

Gruppe In: Verbindungen der Formel I, worin $R_1$ uund $R_2$ die für Formel I angegebenen Bedeutungen haben und R für eine $\alpha$-Methylbenzylgruppe steht, die in para-Stellung durch Isobutyl, 1-Cyclohexen-1-yl, Phenyl oder Benzoyl substituiert ist, oder für eine $\alpha$-Methyl-3-fluor-4-phenylbenzylgruppe steht.

Besonders bevorzugte 5-Hydroxy-Derivate der Formel I sind beispielsweise:

13$\beta$-Formylthio-milbemycin D
13$\beta$-Acetylthio-milbemycin D
13$\beta$-Pivaloylthio-milbemycin D
13$\beta$-Formylthio-milbemycin $A_3$
13$\beta$-Acetylthio-milbemycin $A_3$
13$\beta$-Pivaloylthio-milbemycin $A_3$
13$\beta$-Formylthio-milbemycin $A_4$
13$\beta$-Acetylthio-milbemycin $A_4$
13$\beta$-(2'-Methoxy-2'-methylpropionylthio)-milbemycin D
13$\beta$-(2'-Methoxy-2'-methylpropionylthio)-milbemycin $A_4$
13$\beta$-Trichloracetylthio-milbemycin $A_4$

13β-(4′-Chlor-butanoylthio)milbemycin $A_4$
13β-Trichloracryloylthio-milbemycin $A_4$
13β-Cyclopropancarbonylthio-milbemycin $A_4$
13β-Cyclobutancarbonylthio-milbemycin $A_4$
13β-Heptanoylthio-milbemycin $A_4$
13β-(3′-Chlor-2′,2′-dimethyl-propionylthio)-milbemycin $A_4$
13β-(3′-Chlor-2′,2′-dimethyl-propionylthio)-milbemycin $A_3$
13β-(1′-Methyl-cyclopropancarbonylthio)-milbemycin $A_4$
13β-(1′-Methyl-cyclopropancarbonylthio)-milbemycin $A_3$
13β-(1-Adamantancarbonylthio)-milbemycin $A_4$
13β-(p-Fluorphenoxyacetylthio)-milbemycin $A_4$
13β-(2′-Chlor-2′-methyl-propionylthio)-milbemycin $A_4$
13β-(2′,2′-Dichlorpropionylthio)-milbemycin $A_4$
13β-(2′,2′-Dimethylbutanoylthio)-milbemycin $A_4$
13β-(3′,3′-Dimethylbutanoylthio)-milbemycin $A_4$
13β-(2′,2′,3′,3′-Tetramethylbutanoylthio)-milbemycin $A_4$
13β-(p-Chlorbenzoylthio)-milbemycin $A_4$
13β-(3′,3′,3′-Trifluorpropionylthio)-milbemycin $A_4$
13β-Chloracetylthio-milbemycin $A_4$
13β-(2′-Chlor-3′,3′,3′-Trifluorpropionylthio)-milbemycin $A_4$
13β-(3′,3′,3′-Trifluorpropionylthio)-milbemycin $A_4$
13β-[o-(Trifluormethyl)-benzoylthio]-milbemycin $A_4$
13β-(α,α-Dimethylbenzylcarbonylthio)-milbemycin $A_4$
13β-(2-n-Propyl-n-valeroylthio)-milbemycin $A_4$
13β-[(2,3-Difluormethylendioxy)-benzoylthio]-milbemycin $A_4$
und insbesondere
13β-Pivaloylthio-milbemycin $A_4$
13β-(α-Methylbenzylcarbonylthio)-milbemycin $A_4$ und
13β-(Methoxyacetylthio)-milbemycin $A_4$

Bevorzugte an der 5-Hydroxygruppe mit einer Schutzgruppe versehene Verbindungen der Formel I sind z.B. :

5-O-tert.Butyldimethylsilyl-13β-Formylthio-milbemycin D
5-O-tert.Butyldimethylsilyl-13β-Acetylthio-milbemycin D
5-O-tert.Butyldimethylsilyl-13β-Pivaloylthio-milbemycin D
5-O-tert.Butyldimethylsilyl-13β-Formylthio-milbemycin $A_3$
5-O-tert.Butyldimethylsilyl-13β-Acetylthio-milbemycin $A_3$
5-O-tert.Butyldimethylsilyl-13β-Pivaloylthio-milbemycin $A_3$
5-O-tert.Butyldimethylsilyl-13β-Formylthio-milbemycin $A_4$
5-O-tert.Butyldimethylsilyl-13β-Acetylthio-milbemycin $A_4$
5-O-tert.Butyldimethylsilyl-13β-Pivaloylthio-milbemycin $A_4$
5-O-tert.Butyldimethylsilyl-13β-(2′-Methoxy-2′-methylpropionylthio)-milbemycin D
5-O-tert.Butyldimethylsilyl-13β-(2′-Methoxy-2′-methylpropionylthio)-milbemycin $A_4$
5-O-tert.Butyldimethylsilyl-13β-Trichloracetylthio-milbemycin $A_4$
5-O-tert.Butyldimethylsilyl-13β-(4′-Chlor-butanoylthio)milbemycin $A_4$
5-O-tert.Butyldimethylsilyl-13β-Trichloracryloylthio-milbemycin $A_4$
5-O-tert.Butyldimethylsilyl-13β-Cyclopropancarbonylthio-milbemycin $A_4$
5-O-tert.Butyldimethylsilyl-13β-Cyclobutancarbonylthio-milbemycin $A_4$
5-O-tert.Butyldimethylsilyl-13β-Heptanoylthio-milbemycin $A_4$
5-O-tert.Butyldimethylsilyl-13β-(3′-Chlor-2′,2′-dimethyl-propionylthio)-milbemycin $A_4$
5-O-tert.Butyldimethylsilyl-13β-(3′-Chlor-2′,2′-dimethyl-propionylthio)-milbemycin $A_3$
5-O-tert.Butyldimethylsilyl-13β-(1′-Methyl-cyclopropancarbonylthio)-milbemycin $A_4$
5-O-tert.Butyldimethylsilyl-13β-(1′-Methyl-cyclopropancarbonylthio)-milbemycin $A_3$
5-O-tert.Butyldimethylsilyl-13β-(1-Adamantancarbonylthio)-milbemycin $A_4$
5-O-tert.Butyldimethylsilyl-13β-(p-Fluorphenoxyacetylthio)-milbemycin $A_4$
5-O-tert.Butyldimethylsilyl-13β-(2′-Chlor-2′-methyl-propionylthio)-milbemycin $A_4$
5-O-tert.Butyldimethylsilyl-13β-(2′,2′-Dichlorpropionylthio)-milbemycin $A_4$
5-O-tert.Butyldimethylsilyl-13β-(2′,2′-Dimethylbutanoylthio)-milbemycin $A_4$
5-0-tert.Butyldimethylsilyl-13β-(3',3'-Dimethylbutanoylthio)-milbemycin $A_4$

5-0-tert.Butyldimethylsilyl-13β-(2',2',3'3'-Tetramethylbutanoylthio)-milbemycin $A_4$
5-0-tert.Butyldimethylsilyl-13β-(p-Chlorbensoylthio)-milbemycin $A_4$
5-0-tert.Butyldimethylsilyl-13β-(3',3'3'-Trifluorpropionylthio)-milbemycin $A_4$
5-0-tert.Butyldimethylsilyl-13β-Chloracetylthio-milbemycin $A_4$
5-0-tert.Butyldimethylsilyl-13β-(2'-Chlor-3',3',3'-Trifluorpropionylthio)-milbemycin $A_4$
5-0-tert.Butyldimethylsilyl-13β-(3',3'3'-Trifluorpropionylthio)-milbemycin $A_4$
5-0-tert.Butyldimethylsilyl-13β-(α-Methylbenzylcarbonylthio)-milbemycin $A_4$
5-0-tert.Butyldimethylsilyl-13β-(Methoxyacetylthio)-milbemycin $A_4$
5-0-tert.Butyldimethylsilyl-13β-[o-(Trifluormethyl)-benzoylthio]-milbemycin $A_4$
5-0-tert.Butyldimethylsilyl-13β-(α,α-Dimethylbenzylcarbonylthio)-milbemycin $A_4$
5-0-tert.Butyldimethylsilyl-13β-(2-n-Propyl-n-valeroylthio)-milbemycin $A_4$
5-0-tert.Butyldimethylsilyl-13β-[2,3-Difluormethylendioxy)-benzoylthio]-milbemycin $A_4$.

Von besonderem Interesse sind Verbindungen der Formel I, in denen R für tert.Butyl steht und $R_1$ und $R_2$ die für Formel I angegebenen Bedeutungen haben, und von diesen insbesondere diejenigen Verbindungen, in denen $R_1$ Wasserstoff und $R_2$ Methyl, Ethyl oder Isopropyl bedeutet.

Aus der britischen Patentschrift GB-PS 2 168 345 sind 13β-Acyloxy-milbemycin-Derivate mit pestizider Wirkung, insbesondere gegen Ektoparasiten bei Tieren, bekannt. Mit den erfindungsgemässen Verbindungen lässt sich eine ausgezeichnete Wirkung bei geringeren Anwendungskonzentrationen erzielen als mit den aus der GB-PS 2 168 345 bekannten Milbemycin-Derivaten.

Die vorliegende Erfindung betrifft auch Verfahren, die es erlauben, in der 13-Position von Milbemycin- oder 13-Deoxy-22,23-dihydro-Avermectinaglykon-Derivaten eine β-Acylthio-Gruppe gezielt einzuführen und damit zu den hochwirksamen neuen Parasitiziden und Insektiziden der Formel I zu gelangen, die gleichzeitig auch für weitere Derivatisierungen verwendet werden können.

Zur Herstellung von Thiolestern der Formel I, worin RCOS- in der β-Position steht, geht man von einer Verbindung der Formel II

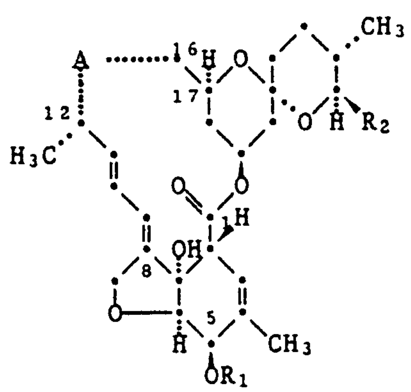

(II)

worin A für eine der Gruppen a, b oder c

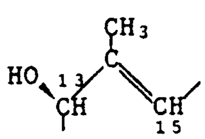
,

(a)

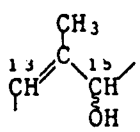

(b)

oder

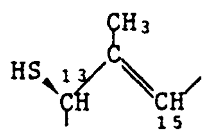

(c)

[= 13β-Hydroxy-$\Delta^{14,15}$] [= $\Delta^{13,14}$-15-Hydroxy] [=13β-Mercapto-$\Delta^{14,15}$

steht, worin $R_1$ und $R_2$ die für Formel I angegebenen Bedeutungen haben, aus.
Eine Verbindung der Formel II, in welcher $R_1$ eine Schutzgruppe bedeutet und $R_2$ die unter Formel I angegebenen Bedeutungen besitzt, wird mit einem zur Einführung oder zum Aufbau einer 13$\beta$-Thiolestergruppe geeigneten Reagenz behandelt. Danach kann die $R_1$-Schutzgruppe, sofern eine freie 5-Hydroxy-Verbindung erwünscht ist, hydrolytisch abgespalten werden.

Verbindungen der Formel II, worin A für die Gruppe a steht, werden hier und im folgenden mit IIa, die Verbindungen mit der Gruppe b mit IIb und die Verbindungen mit der Gruppe c als IIc bezeichnet.

Zur Einführung der 13$\beta$-Thiolestergruppe in Verbindungen der Formeln IIa und IIb geeignete Reagenzien sind beispielsweise:

a) Thiocarbonsäuren der Formel III

RCOSH (III)

b) Thioamide der Formel IV

$RCSN(Alkyl)_2$ (IV)

worin die Alkylreste je 1 bis 4 Kohlenstoffatome umfassen und vorzugsweise Methyl bedeuten.

Ein anderes Verfahren zur Herstellung der Thiolester der Formel I besteht in der Umsetzung einer Verbindung der Formel IIc mit

c) einem Säurehalogenid der Formel V

RCOhal (V),

worin hal Halogen, bevorzugt Chlor oder Brom, bedeutet, oder

d) einem Säureanhydrid der Formel VI

$(RCO)_2O$ (VI),

wobei sich die Thiocarbonsäuren und Thioamide für alle Verbindungen der Formeln IIa und IIb eignen, vorzugsweise aber für Verbindungen der Formel IIb verwendet werden, während Säurehalogenide und Säureanhydride bei Verbindungen der Formel IIc zum Einsatz gelangen.

In den vorstehend angegebenen Formeln III-VI besitzt R die unter Formel I angegebenen Bedeutungen.

Verbindungen der Formel I, worin $R_1$ eine Schutzgruppe darstellt, lassen sich durch einfache, beispielsweise hydrolytische Abspaltung der Schutzfunktion in die hochaktiven freien 5-Hydroxy-derivate ($R_1 = H$) überführen und haben somit Zwischenprodukte-Charakter. Im übrigen wird der biologische Wert dieser Verbindungen durch die Schutzgruppe nicht gemindert.

Das Verfahren wird im allgemeinen in einem reaktions-inerten Lösungsmittel oder in einem der beteiligten Reaktanden, sofern diese flüssig sind, durchgeführt. Geeignete Lösungsmittel sind beispielsweise: Ether und etherartige Verbindungen wie Dialkylether (Diethylether, Diisopropylether, tert.-Butylmethylether, Dimethoxyethan, Dioxan, Tetrahydrofuran oder Anisol); halogenierte Kohlenwasserstoffe wie Chlorbenzol, Methylenchlorid, Ethylenchlorid, Chloroform, Tetrachlorkohlenstoff oder Tetrachlorethylen; oder Sulfoxyde wie Dimethylsulfoxyd; ferner aromatische oder aliphatische Kohlenwasserstoffe, wie Benzol, Toluol, Xylole, Petrolether, Ligroin oder Cyclohexan. In manchen Fällen kann es von Vorteil sein, wenn die Reaktionen unter Schutzgasatmosphäre (beispielsweise Argon, Helium oder Stickstoff) und/oder in absoluten Lösungsmitteln durchgeführt werden. Gewünschtenfalls können die Endprodukte auf übliche Art und Weise gereinigt werden, beispielsweise durch Waschen, Digerieren, Extraktion, Umkristallisation oder Chromatographie.

Die Umsetzung von Verbindungen der Formel IIa oder IIb mit Thiocarbonsäuren oder Thioamiden der Formeln III bzw. IV erfolgt zweckmässigerweise in Gegenwart von Orthoestern sowie in Gegenwart katalytischer Mengen einer weiteren Säure. Als dafür geeignete Säuren können Protonensäuren oder Lewis-Säuren eingesetzt werden. Beispiele derartiger Säuren sind anorganische Säuren: Halogenwasserstoffsäure wie Chlorwasserstoffsäure, Bromwasserstoffsäure oder Jodwasserstoffsäure, Perchlorsäure sowie Schwefelsäure und organische Säuren wie Essigsäure, Trifluoressigsäure, Trichloressigsäure, Propionsäure, Oxalsäure, Ameisensäure, Benzolsulfonsäure, p-Toluolsulfonsäure oder Methansulfonsäure sowie Lewis-Säuren, wie $BF_3$, $AlCl_3$ oder $ZnCl_2$. Besonders bevorzugt sind p-Toluolsulfonsäure (auch als TsOH bezeichnet) und Schwefelsäure.

Die bei dieser Reaktion verwendeten Orthoester haben die Formel VII

$R_3C(OR_4)_3$ (VII),

worin $R_3$ Wasserstoff oder $C_1$-$C_4$-Alkyl, bevorzugt Methyl, und $R_4$ $C_1$-$C_4$-Alkyl, bevorzugt Methyl oder Ethyl bedeuten.

Bei Verwendung von Thiocarbonsäuren oder Thioamiden der Formeln III bzw. IV zur Herstellung von Verbindungen der Formel I liegen die Reaktionstemperaturen im allgemeinen im Bereich von 0° bis 150°C, bevorzugt von 20° bis 130°C.

Die Reaktion von Verbindungen der Formel IIc mit Säurehalogeniden oder Säureanhydriden der Formeln V bzw. VI wird im allgemeinen in den obengenannten reaktionsinerten Lösungsmitteln und bei Temperaturen von -20° bis 100°C, vorzugsweise von 0°C bis 70°C, durchgeführt. Zum Abfangen der dabei als Nebenprodukt gebildeten Säuren findet die Reaktion zweckmässigerweise in Gegenwart eines Neutralisationsmittels statt.

Als solche kommen organische Basen in Betracht, beispielsweise tertiäre Amine wie Trialkylamine (Trimethylamin, Triethylamin, Diisopropylmethylamin oder Tripropylamin), Pyridin und Pyridinbasen (4-Dimethylaminopyridin oder 4-Pyrrolidylaminopyridin), bevorzugt ist Pyridin. Das Neutralisationsmittel wird üblicherweise in mindestens äquimolarer Menge in bezug auf die Ausgangsstoffe eingesetzt.

Bei der Reaktion von Verbindungen der Formel IIb mit Thiocarbonsäuren oder Thioamiden der Formeln II bzw. IV in Gegenwart von Orthoestern der Formel VII sowie einer katalytisch wirksamen Säure können neben den Verbindungen der Formel I als Nebenprodukte auch Verbindungen der Formel VIII

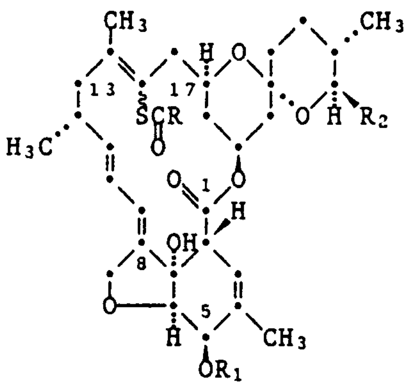

(VIII)

worin $R_1$, $R_2$ und R die unter Formel I angegebenen Bedeutungen besitzen, gebildet werden.

Die erhaltenen Reaktionsprodukte lassen sich durch übliche Trennungsmethoden, wie beispielsweise durch fraktionierte Kristallisation oder durch Chromatographie, voneinander trennen. Unter Chromatographie werden Säulen-, Dickschicht- oder Dünnschicht-Chromatographie sowie bevorzugt Hochdruck-Flüssigkeits-Chromatographie an mineralischen Trägermaterialien wie Silicagel oder an organischen Austauscherharzen verstanden.

Die zur Gewinnung der erfindungsgemässen Verbindungen der Formel I mittels der hierin beschriebenen Verfahren benötigten Ausgangsverbindungen der Formel IIa werden erhalten durch Reaktion von Verbindungen der Formel IIb mit Chromat-, Halochromat- oder Dichromat-Ionen, insbesondere mit Pyridiniumdichromat $[=(Pyr)_2^+ Cr_2O_7]$ oder mit Pyridiniumchlorochromat $[=(Pyr)^+ClCrO_3]$.

Es werden inerte wasserfreie, vorzugsweise polare Lösungsmittel, z.B. Dimethylformamid (=DMF) verwendet. Die Reaktion wird bei Temperaturen von -10°C bis +60°C, bevorzugt +10°C bis +40°C, durchgeführt.

Die Verbindungen der Formel IIb sind in EP 147 852 beschrieben.

Die Herstellung von Verbindungen der Formel IIc kann durch Umsetzung einer Verbindung der Formel IIb mit einem Halothionoformiat der Formel (IX)

$$Hal-\overset{S}{\overset{\|}{C}}-OR_9 \qquad (IX),$$

worin $R_9$ für $C_1$-$C_{10}$-Alkyl, $C_1$-$C_{10}$-Haloalkyl oder eine unsubstituierte oder durch Halogen, $C_1$-$C_3$-Alkyl, $C_1$-$C_3$-Haloalkyl, $C_1$-$C_3$-Alkoxy, $C_1$-$C_3$-Haloalkoxy, Cyano und/oder Nitro substituierte Gruppe aus der Reihe Phenyl und Benzyl steht, und anschliessende Reduktion des erhaltenen Produkts erfolgen.

Die Reaktion von Verbindungen der Formel IIb mit Halothionoformiaten der Formel IX erfolgt üblicherweise in den oben genannten reaktionsinerten Lösungsmitteln oder in dem Halothionoformiat der Formel IX selbst. Man arbeitet zweckmässigerweise in Gegenwart eines Kondensationsmittels. Als solche kommen organische und anorganische Basen in Betracht, z.B. tertiäre Amine wie Trialkylamine (Trimethylamin, Triethylamin, Tripropylamin, usw.), Pyridin und Pyridinbasen (4-Dimethylaminopyridin, 4-Pyrrolidylaminopyridin usw.), bevorzugt ist Pyridin. Das Kondensationsmittel wird üblicherweise in mindestens äquimolarer Menge in Bezug auf die Ausgangsstoffe eingesetzt. Die Reaktiostemperaturen liegen bei dieser Umsetzung im allgemeinen bei -50 °C bis +150 °C, vorzugsweise bei -20° bis +100 °C. Die bei dieser Reaktion entstehenden Thiolcarbonate der Formel I ($R = OR_9$) lassen sich durch einfache Reduktion, z.B. mit Zink in Eisessig, in die 13$\beta$-Mercapto-Verbindungen der Formel IIc überführen. Diese Reduktion erfolgt zweckmässigerweise in einem üblichen, reaktionsinerten, organischen Lösungsmittel bei Temperaturen zwischen 0° und 50 °C, vorzugsweise 20° und 50 °C.

Durch Acylierung oder Silylierung der 5-OH-Gruppe werden alle jene Derivate der Formeln I, IIa, IIb und IIc hergestellt, bei denen $R_1$ eine andere Bedeutung als Wasserstoff ($R_1$ = OH-Schutzgruppe) hat. Die Einführung Acylgruppe erfolgt üblicherweise mit den entsprechenden Acylhalogeniden oder Acylanhydriden und wird vorzugsweise zur Einführung der eingangs definierten $R_5C(O)$- Gruppe benutzt. Zur Silylierung verwendet man zweckmässigerweise ein Silan der Formel Y-Si($R_6$)($R_7$)($R_8$), worin $R_6$, $R_7$ und $R_8$ einen der eingangs genannten Reste darstellen, wobei der Begriff Acylhalogenid für Acylchlorid oder Acylbromid steht und wobei Y eine Silylabgangsgruppe bedeutet. Zu den Silylabgangsgruppen Y zählen beispielsweise Bromid, Chlorid, Cyanid, Azid, Acetamid, Trifluoracetat oder Trifluormethansulfonat. Diese Aufzählung stellt keine Limitierung dar, der Fachman kennt weitere typische Silylabgangsgruppen.

5-O-Acylierungen und 5-O-Silylierungen werden in wasserfreiem Milieu, vorzugsweise in inerten Lösungsmitteln und besonders bevorzugt in aprotischen Lösungsmitteln durchgeführt. Die Reaktion läuft vorteilhaft im Temperaturbereich von 0° bis +80 °C, bevorzugt bei +10° bis +40 °C, ab. Vorzugsweise wird eine organische Base zugegeben. Es kommen als solche beispielsweise tertiäre Amine wie Triethylamin, Triethylendiamin, Triazol und bevorzugt Pyridin, Imidazol oder 1,8-Diazabicyclo[5.4.0]-undec-7-en (DBU) in Frage.

Die Entfernung dieser Silyl- und Acylreste $R_1$ in der 5-Position geschieht durch selektive milde Hydrolyse ($\rightarrow R_1 = H$) beispielsweise mit Arylsulfonsäure in alkoholischer Lösung oder nach einer anderen dem Fachmann geläufigen Methode.

Das beschriebene Verfahren zur Herstellung der Verbindungen der Formel I ist in allen Teilschritten ein Bestandteil vorliegender Erfindung.

Die Verbindungen der Formel I eignen sich ausgezeichnet zur Bekämpfung von Schädlingen an Tieren und Pflanzen, darunter insbesondere von tierparasitären Ekto-Parasiten. Zu letzteren zählen unter der Ordnung Acarina insbesondere Schädlinge der Familien Ixodidae, Dermanyssidae, Sarcoptidae, Psoroptidae; die Ordnungen Mallophaga; Siphonaptera, Anoplura (z.B. Familie der Haemotopinidae); unter der Ordnung Diptera insbesondere Schädlinge der Familien Muscidae, Calliphoridae, Oestridae, Tabanidae, Hippoboscidae, Gastrophilidae.

Die Verbindungen I sind auch einsetzbar gegen Hygiene-Schädlinge, insbesondere der Ordnungen Diptera mit den Familien Sarcophagidae, Anophilidae, Culicidae; der Ordnung Orthoptera, der Ordnung Dictyoptera (z.B. Familie Blattidae) und der Ordnung Hymenoptera (z.B. Familie Formicidae).

Die Verbindungen I besitzen auch nachhaltige Wirksamkeit bei pflanzenparasitären Milben und Insekten. Bei Spinnmilben der Ordnung Acarina sind sie wirksam gegen Eier, Nymphen und Adulte von Tetranychidae (Tetranychus spp. und Panonychus spp.).

Hohe Aktivität besitzen sie bei den saugenden Insekten der Ordnung Homoptera, insbesondere gegen Schädlinge der Familien Aphididae, Delphacidae, Cicadellidae, Psyllidae, Coccidae, Diaspididae und Eriophydidae (z.B. die Rostmilbe auf Citrusfrüchten): der Ordnungen Hemiptera; Heteroptera und Thysanoptera; sowie bei den pflanzenfressenden Insekten der Ordnungen Lepidoptera; Coleoptera; Diptera und Orthoptera.

Sie sind ebenfalls als Bodeninsektizid gegen Schädlinge im Erdboden geeignet.

Die Verbindungen der Formel I sind daher gegen alle Entwicklungsstadien saugender und fressender Insekten an Kulturen wie Getreide, Baumwolle, Reis, Mais, Soja, Kartoffeln, Gemüse, Früchten, Tabak, Hopfen, Citrus, Avocados und anderen wirksam.

Die Verbindungen der Formel I sind auch wirksam gegen Pflanzen-Nematoden der Arten Meloidogyne, Heterodera, Pratylenchus, Ditylenchus, Radopholus, Rhizoglyphus und andere.

Ferner sind die Verbindungen gegen Helminthen wirksam, unter denen die endoparasitären Nematoden die Ursache schwere Erkrankungen an Säugetieren und Geflügel sein können, z.B. an Schafen, Schweinen, Ziegen, Rindern, Pferden, Eseln, Hunden, Katzen, Meerschweinchen, Ziervögeln. Typische Nematoden dieser Indikation sind: Haemonchus, Trichostrongylus, Ostertagia, Nematodirus, Cooperia, Ascaris, Bunostomum, Oesophagostomum, Chabertia, Trichuris, Strongylus, Trichonema, Dictyocaulus, Capillaria, Heterakis, Toxocara, Ascaridia, Oxyuris, Ancylostoma, Uncinaria, Toxascaris und Parascaris. Der besondere Vorteil der Verbindungen der Formel I ist ihre Wirksamkeit gegen solche Parasiten, die gegen Wirkstoffe auf Benzimidazol-Basis resistent sind.

Gewisse Spezies der Arten Nematodirus, Cooperia und Oesophagostomum greifen den Intestinaltrakt des Wirtstiers an, während andere der Arten Haemonchus und Ostertagia im Magen und solche der Art Dictyocaulus im Lungengewebe parasitieren. Parasiten der Familien Filariidae und Setariidae finden sich im internen Zellgewebe und den Organen, z.B. dem Herzen, den Blutgefässen, den Lymphgefässen und dem subcutanen Gewebe. Hier ist vor allem der Herzwurm des Hundes, Dirofilaria immitis, zu nennen. Die Verbindungen der Formel I sind gegen diesen Parasiten hoch wirksam.

Weiter sind die Verbindungen der Formel I zur Bekämpfung von humanpathogenen Parasiten geeignet, unter denen als typische, im Verdauungstrakt vorkommende Vertreter solche der Arten Ancylostoma, Necator, Ascaris, Strongyloides, Trichinella, Capillaria, Trichuris und Enterobius zu nennen sind. Wirksam sind die Verbindungen vorliegender Erfindung auch gegen Parasiten der Arten Wuchereria, Brugia, Onchocerca und Loa aus der Familie der Filariidae, die im Blut, im Gewebe und verschiedenen Organen vorkommen, ferner gegen Dracunculus und Parasiten der Arten Strongyloides und Trichinella, die speziell den Gastro-Intestinalkanal infizieren.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Verbindungen der Formel I werden bei Warmblütern in Aufwandmengen von 0,01 bis 10 mg/kg Körpergewicht dosiert. Ueber geschlossenen Kultur-Anbauflächen werden sie in Mengen von 10 g bis 1000 g pro Hektar angewendet. Sie kommen ferner in Pferchen, Gehegen, Stallungen oder sonstigen Räumen zur Anwendung.

Die Formulierungen, d.h. die den Wirkstoff der Formel I enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon,Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B.

Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxyd-Adduktes oder Phospholipide in Frage.

Die in der Formulierungstechnik gebräuchlichen Tenside sind u.a. in folgender Publikation beschrieben:
"1985 International McCutcheon's Emulsifiers and Detergents"
The Manufacturing Confectioner Publishing Co.,
Glen Rock, New Jersey, USA.

Die pestiziden Zubereitungen enthalten in der Regel 0,01 bis 95 %, insbesondere 0,1 bis 80 %, Wirkstoff der Formel I, 5 bis 99,99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel mit 1-10'000 ppm Wirkstoffgehalt.

Ein weiterer Gegenstand vorliegender Erfindung betrifft daher Schädlingsbekämpfungsmittel, die neben üblichen Trägerstoffen und/oder Verteilungsmitteln als Wirkstoff mindestens eine Verbindung der Formel I enthalten.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## Herstellungsbeispiele

### 1. Herstellung von Ausgangs- und Zwischenprodukten

Beispiel A1: Herstellung von 5-O-tert.Butyldimethylsilyl-13$\beta$-hydroxy-milbemycin D und von 13$\beta$-Hydroxy-milbemycin D (Formel IIa)

Eine Lösung bestehend aus 286 mg (0,41 mmol) 5-O-tert.Butyldimethylsilyl-15-hydroxy-$\Delta^{13,14}$-milbemycin D und 209 mg (0,56 mmol) Pyridiniumdichromat (PDC) in 3 ml Dimethylformamid (DMF) wird 30 min bei Raumtemperatur gerührt. Anschliessend wird 1 ml Isopropanol zugegeben, 5 min weitergerührt und dann mit 50 ml Ether verdünnt. Nach weiteren 10 min wird das Gemisch durch Kieselgel filtriert und eingeengt. Nach chromatographischer Reinigung des Rohproduktes an 20 g Kieselgel (Ether/Hexan 1:2) werden 165 mg (57%) 5-O-t-Butyldimethylsilyl-13$\beta$-hydroxy-Milbemycin D erhalten.
$^1$H-NMR (300 MHz; $CDCl_3$; TMS):
1,59 ppm (br. s)($C_{14}CH_3$)
3,70 ppm (d; J = 10 Hz)($C_{13}H$).

105 mg (0,153 mmol) der so gewonnenen Verbindung werden mit 1 ml einer 1 %igen Lösung von p-Toluolsulfonsäure in Methanol 1 Stunde bei Raumtemperatur gerührt. Das Gemisch wird mit 20 ml Ether verdünnt, durch Kieselgel filtriert, eingeengt. Der Rückstand wird an ca. 10 g Kieselgel chromatographiert (Aceton/Dichlormethan 1:4), wobei 73 mg (83 %) 13β-Hydroxy-Milbemycin D erhalten werden.
$^{1}$H-NMR (300 MHz; $CDCl_3$; TMS):
1,58 ppm (br. s)($C_{14}CH_3$)
3,71 ppm (d; J = 10 Hz)($C_{13}H$).

Beispiel A2: Herstellung von 13β-Mercapto-milbemycin D und von 5-O-tert.Butyldimethylsilyl-13β-mercapto-milbemycin D

a) Zu einer Lösung von 209 mg (0,305 mmol) 5-O-tert.Butyldimethylsilyl-15-hydroxy-$\Delta^{13,14}$-milbemycin D und 0,012 ml (120 mg, 1,52 mmol) Pyridin in 3 ml Dichlormethan werden unter Argon bei -10 °C unter Rühren 0,1 ml (157 mg, 0,689 mmol) Trichlorethylchlorthionoformiat zugetropft. Nach 1 Stunde Rühren bei Raumtemperatur wird mit 5 %iger wässriger $NaHCO_3$-Lösung und Diethylether aufgearbeitet. Die Chromatographie des Rohprodukts [20 g Kieselgel/Laufmittel:Ethylacetat/Hexan (1:4)] liefert 282 mg z.T. noch verunreinigtes 5-O-tert.Butyldimethylsilyl-13β-trichlorethoxycarbonylthio-milbemycin D.
Eine Suspension von 320 mg (4,9 mmol) Zink-Pulver in einer Lösung von 227 mg dieses Rohproduktes in 0,5 ml Diethylether, 2 ml 90 %iger wässriger Essigsäure und 3 Tropfen HCl (1M) werden bei Raumtemperatur 16 Stunden unter Argon gerührt. Das Gemisch wird mit Diethylether verdünnt, durch Kieselgur filtriert, mit $MgSO_4$ getrocknet und eingeengt. Die Chromatographie des Rohprodukts [20 g Kieselgel/Laufmittel:Ethylacetat/Hexan (12:88)] ergibt 72 mg (40 %) 5-O-tert.Butyldimethylsilyl-13β-mercapto-milbemycin D.
b) Dieses gereinigte Produkt wird mit 2 ml einer 1 %igen Lösung von p-Toluolsulfonsäure in Methanol 2 Stunden bei Raumtemperatur gerührt. Nach der Aufarbeitung mit 5 %iger wässriger $NaHCO_3$-Lösung und Diethylether wird das Rohprodukt [20 g Kieselgel/Laufmittel:Ethylacetat/Hexan (2:3)] chromatographiert. Man erhält 54 mg (89 %) 13β-Mercapto-milbemycin D mit folgenden spektroskopischen Daten:
$^{1}$-NMR (300 MHz; $CDCl_3$; TMS)
1,61 ppm ( s)($C_{14}CH_3$)
1,87 ppm (s)($C_4CH_3$)
3,31 ppm (dd; J = 5,4 und 10,9), ($C_{13}H$)
Massenspektrum m/e:588 ($M^+$, $C_{33}H_{48}O_7S$) 460, 309, 277, 209, 181.

Beispiel A3: Herstellung von 5-O-tert.Butyldimethylsilyl-13β-hydroxy-milbemycin $A_4$

Eine Lösung bestehend aus 1,06 g (1,59 mmol) 5-O-tert.Butyldimethylsilyl-15-hydroxy-$\Delta^{13,14}$-milbemycin $A_4$ und 383 mg (1,02 mmol) Pyridiniumdichromat (PDC) in 5 ml Dimethylformamid (DMF) wird 30 min. bei Raumtemperatur gerührt. Anschliessend wird 1 ml Isopropanol zugegeben, 5 min. weitergerührt und dann mit 50 ml Ether verdünnt. Nach weiteren 10 min. wird das Gemisch durch Kieselgel filtriert und eingeengt. Nach chromatographischer Reinigung des Rohproduktes an 20 g Kieselgel (Ether/Hexan 1:2) werden 625 mg (59 %) 5-O-t-Butyldimethylsilyl-13β-hydroxy-Milbemycin $A_4$ erhalten.
$^{1}$H-NMR (300 MHz; $CDCl_3$; TMS):
0,98 ppm (t; J = 7 Hz)($CH_3CH_2$)
1,95 ppm (br. s)($C_{14}CH_3$)
3,69 ppm (d; J = 9 Hz)($C_{13}H$).

Beispiel A4: Herstellung von 5-O-tert.Butyldimethylsilyl-13β-mercapto-milbemycin $A_4$

a) Zu einer Lösung von 100 mg (0,15 mmol) 5-O-tert.Butyldimethylsilyl-15-hydroxy-$\Delta^{13,14}$-milbemycin $A_4$ und 0,060 ml (59 mg, 0,75 mmol) Pyridin in 3 ml Dichlormethan werden unter Argon bei -10 °C unter Rühren 0,13 ml (205 mg, 0,9 mmol) Trichlorethylchlorthionoformiat zugetropft. Nach 30 Min. Rühren bei Raumtemperatur wird mit 5 %iger wässriger $NaHCO_3$-Lösung und Diethylether aufgearbeitet. Die Chromatographie des Rohprodukts [20 g Kieselgel/Laufmittel:Ethylacetat/Hexan (1:12)] liefert 40 mg 5-O-tert.Butyldimethylsilyl-13β-trichlorethoxycarbonylthio-milbemycin $A_4$.
$^{1}$H-NMR (300 MHz; $CDCl_3$; TMS):
3,82 ppm (d; J = 10 Hz)($C_{13}H$)
4,75 ppm (d; J = Hz) und 4,86 (d; J = 14 Hz)($Cl_3CCH_2$)
Massenspektrum (FD) m/e: 862 ($M^+$, $C_{41}H_{61}Cl_3O_9SSi$).

b) Eine Lösung von 2,9 g (3,36 mmol) 5-O-tert.Butyldimethylsilyl-13β-trichlorethoxycarbonylthio-milbemycin $A_4$ in 40 ml Tetrahydrofuran wird mit 1,05 g (16,1 mmol) Zink und 20 ml gesättigtem wässrigem $NH_4Cl$ während 5 Stunden schnell gerührt. Aufgearbeitet wird mit Wasser und Diethylether. Die Chromatographie des Rohprodukts (Kieselgel/Laufmittel: Ethylacetat/Hexan (1:9)] liefert 2,46 g 5-O-tert.Butyldimethylsilyl-13β-mercapto-milbemycin $A_4$.
$^1$H-NMR (300 MHz; $CDCl_3$; TMS):
3,29 ppm (dd; J = 10 und 5 Hz)($C_{13}H$)
Massenspektrum m/e: 688 ($M^+$, $C_{38}H_{60}O_7SSi$).

Beispiel $A_5$: Herstellung von 5-O-tert.Butyldimethylsilyl-13β-mercapto-milbemycin $A_3$

Analog Beispiel A4 wird 5-O-tert.Butyldimethylsilyl-13β-mercapto-milbemycin $A_3$ hergestellt.
a) 5-O-tert.Butyldimethylsilyl-13β-trichlorethoxycarbonylthio-milbemycin $A_3$:
$^1$H-NMR (300 MHz; $CDCl_3$; TMS):
3,84 ppm (d; J = 10 Hz)($C_{13}H$)
4,76 ppm (d; J = 14 Hz) und 4,87 (d; J = 14 Hz)($Cl_3CCH_2$)
Massenspektrum (FD) m/e: 848 ($M^+$, $C_{40}H_{59}Cl_3O_9SSi$).
b) 5-O-tert.Butyldimethylsilyl-13β-mercapto-milbemycin $A_3$;
$^1$H-NMR (300 MHz; $CDCl_3$; TMS):
3,29 ppm (dd; J = 10 und 5 Hz)($C_{13}H$)
Massenspektrum m/e = 674 ($M^+$, $C_{37}H_{58}O_7SSi$).

2. Herstellung von Endprodukten

Beispiel H1: Herstellung von 13β-Pivaloylthio-milbemycin $A_4$

Eine Lösung von 280 mg (0,416 mmol) 5-O-tert.-Butyldimethylsilyl-15-hydroxy-$\Delta^{13,14}$-milbemycin $A_4$, 0,4 ml Orthoessigsäure-trimethylester und 0,4 ml Thiopivalinsäure in 4 ml Toluol wird während 6 Stunden auf 60 °C erhitzt. Aufgearbeitet wird mit Diethylether und 5%iger wässriger $NaHCO_3$-Lösung. Die Chromatographie an Kieselgel (Ethylacetat/Hexan 1:6) liefert 61 mg 5-O-tert.-Butyldimethylsilyl-13β-pivaloylthio-milbemycin $A_4$.

Dieses Material wird mit 2 ml einer 40%igen wässrigen Lösung von HF und Acetonitril (5:95) während 2 Stunden bei Raumtemperatur behandelt. Die Aufarbeitung in Diethylether mit 5%iger wässriger $NaHCO_3$-Lösung und Chromatographie an Kieselgel (Ethylacetat/Hexan 1:2) liefert 21 mg 13β-Pivaloylthio-milbemycin $A_4$.
1H-NMR (250 MHz; $CDCl_3$; TMS):
1,25 ppm (s)[$(CH_3)_3C$]
3,97 ppm (d)(J = 10 Hz)($C_{13}H$)
4,01 ppm (d)(J = 6 Hz)($C_6H$)

Beispiel H2: Herstellung von 13β-Acetylthio-milbemycin $A_4$

Analog Beispiel H1 wird 13β-Acetylthio-milbemycin $A_4$ hergestellt.
$^1$H-NMR (300 MHz; $CDCl_3$; TMS):
2,32 ppm (s)($CH_3COS$]
3,96 ppm (d,J = 6 Hz)($C_6H$)
4,03 ppm (d,J = 10 Hz)($C_{13}H$).

Beispiel H3: Herstellung von 5-O-tert.Butyldimethylsilyl-13β-(2-n-propyl-n-valeroylthio)-milbemycin $A_4$ und von 13β-(2-n-propyl-n-valeroylthio)-milbemycin $A_4$

a) Zu einer Lösung von 110 mg (0,154 mmol) 5-O-tert.Butyldimethylsilyl-13β-mercapto-milbemycin $A_4$ in 5 ml abs. Chloroform und 2 ml Pyridin werden unter Argon bei 0 °C unter Rühren 0,2 ml 2-n-Propyl-n-valeroylchlorid zugegeben. Nach 7 Stunden Rühren bei Raumtemperatur wird mit eiskalter, verdünnter wässriger HCl, verdünnter wässriger $NaHCO_3$-Lösung und Diethylether aufgearbeitet. Die Chromatographie des Rohprodukts [10 g Kieselgel/Laufmittel: Ethylacetat/Hexan (1:5)] liefert 92 mg 5-O-tert.Butyldimethylsilyl-13β-(2-n-propyl-n-valeroylthio)-milbemycin $A_4$.

b) Dieses gereinigte Produkt wird mit 1 ml einer 1 %igen Lösung von p-Toluolsulfonsäure in Methanol 3 Stunden bei Raumtemperatur gerührt. Nach der Aufarbeitung mit 5 %iger wässriger $NaHCO_3$-Lösung und Diethylether wird das Rohprodukt chromatographiert [10 g Kieselgel/Laufmittel: Ethylacetat/Hexan (1:3)]. Man erhält 49 mg 13β-(2-n-propyl-n-valeroylthio)-milbemycin $A_4$ mit folgenden spektroskopischen Daten:
$^1$H-NMR (300 MHz; $CDCl_3$; TMS)
3,95 ppm (d, J = 6 Hz)($C_6H$)
3,97 ppm (d, J = 10 Hz)($C_{13}H$)
MS (FD) m/e: 700 ($M^+$, $C_{40}H_{60}O_8S$).

Die in den folgenden Beispielen H4 bis H16 aufgeführten Verbindungen werden analog dem in Beispiel H3 beschriebenen Verfahren hergestellt:

Beispiel H4: 13β-Acetylthio-milbemycin $A_4$

MS (FD) m/e: 616 ($M^+$, $C_{34}H_{48}O_8S$).

Beispiel H5: 13β-(2-Trifluormethylbenzoyl)-milbemycin $A_4$

$^1$H-NMR (300 MHz; $CDCl_3$; TMS)
3,97 ppm (d, J = 6 Hz)($C_6H$)
4,21 ppm (d, J = 10 Hz)($C_{13}H$)
7,48-7,76 ppm (m)(4 aromat. H)
MS (FD) m/e: 746 ($M^+$, $C_{40}H_{49}O_8SF_3$).

Beispiel H6: 13β-[(R/S)-2-Phenylpropionylthio)-milbemycin $A_4$; Diastereomerengemisch

$^1$H-NMR (300 MHz; $CDCl_3$; TMS)
3,75 und 3,83 ppm (2q, J = 6 Hz)($C'_2H$)
3,82 und 3,93 ppm (2d, J = 6 Hz)($C_6H$)
3,81 und 3,94 ppm (2d, J = 10 Hz)($C_{13}H$)
7,11-7,37 ppm (m)(5 aromat. H).

Beispiel H7: 13β-(2,2-Dimethylbutyrylthio)-milbemycin $A_4$

$^1$H-NMR (300 MHz; $CDCl_3$; TMS)
3,91 ppm (d, J = 10 Hz)($C_{13}H$)
3,95 ppm (d, J = 6 Hz)($C_6H$)
MS (FD) m/e: 672 ($M^+$, $C_{38}H_{56}O_8S$).

Beispiel H8: 13β-(3-Chlor-2,2-dimethylpropionylthio)-milbemycin $A_4$

$^1$H-NMR (300 MHz; $CDCl_3$; TMS)
3,93 ppm (d, J = 10 Hz)($C_{13}H$)
3,96 ppm (d, J = 6 Hz)($C_6H$)
3,60 ppm (AB-System, J = 13 Hz; A-Teil: 3,57 ppm, B-Teil: 3,63 ppm)($CH_2Cl$).

Beispiel H9: 13β-(2-Methyl-2-phenylpropionylthio)-milbemycin $A_4$

$^1$H-NMR (300 MHz; $CDCl_3$; TMS)
3,89 ppm (d, J = 11 Hz)($C_{13}H$)
3,93 ppm (d, J = 6 Hz)($C_6H$)
7,19-7,37 (m)(5 aromat. H).

Beispiel H10: 13β-(3-Fluor-2,2-dimethylpropionylthio)-milbemycin $A_4$

$^1$H-NMR (300 MHz; $CDCl_3$; TMS)
3,94 ppm (d, J = 6 Hz)($C_6H$)
3,98 ppm (d, J = 10 Hz)($C_{13}H$)
4,37 ppm (d, J = 47 Hz)($CH_2F$)
MS (FD) m/e: 676 ($M^+$, $C_{37}H_{53}FO_8S$).

Beispiel H11: 13β-Methoxyacetylthio-milbemycin $A_4$

$^1$H-NMR (300 MHz; $CDCl_3$; TMS)
3,94 ppm (d, J = 6 Hz)($C_6H$)
4,03 ppm (d, J = 10 Hz)($C_{13}H$)
3,45 ppm (s)($CH_3OCH_2$)
4,04 ppm (s)($C\overline{H}_3OCH_2$)
MS (FD) m/e: 646 ($\overline{M^+}$, $C_{35}H_{50}O_9S$).

Beispiel H12: 13β-[(S)-2-Phenylpropionylthio]-milbemycin $A_4$

$^1$H-NMR (300 MHz; $CDCl_3$; TMS)
3,83 ppm (q, J = 6 Hz)($C'_2H$)
3,93 ppm (d, J = 6 Hz)($C_6H$)
3,94 ppm (d, J = 10 Hz)($C_{13}H$)
7,18-7,37 (m)(5 aromat. H).

Beispiel H13: 13β-[(R)-2-Phenylpropionylthio]-milbemycin $A_4$

$^1$H-NMR (300 MHz; $CDCl_3$; TMS)
3,75 ppm (q, J = 6 Hz)($C'_2H$)
3,81 ppm (d, J = 10 Hz)($C_{13}H$)
3,82 ppm (d, J = 6 Hz)($C_6H$)
7,11-7,26 ppm (m)(5 aromat. H).

Beispiel H14: 13β-[2,3-(Difluormethylendioxy)-benzoylthio]-milbemycin $A_4$

$^1$H-NMR (300 MHz; $CDCl_3$; TMS)
3,95 ppm (d, J = 6 Hz)($C_6H$)
4,25 ppm (d, J = 10 Hz)($C_{13}H$)
7,12 ppm (dd, J = 8 und 8 Hz)($C'_5H$)
7,23 ppm (dd, J = 2 und 8 Hz)($C'_4H$)
7,63 ppm (dd, J = 2 und 8 Hz)($C'_6H$).

Beispiel H15: 13β-Chloracetylthio-milbemycin $A_4$

$^1$H-NMR (300 MHz; $CDCl_3$; TMS)
3,94 ppm (d, J = 6 Hz)($C_6H$)
4,02 ppm (d, J = 10 Hz)($C_{13}H$)
4,03 und 4,16 (2s)($CH_2Cl$).

Beispiel H16: 13β-Pivaloylthio-milbemycin $A_3$

$^1$H-NMR (300 MHz; $CDCl_3$; TMS)
1,24 ppm (s)[$(CH_3)_3C$]
3,97 ppm (d, J = 10 Hz)($C_{13}H$)
4,03 ppm (d, J = 6 Hz)($C_6H$).

Analog Zu den beschriebenen Arbeitsweisen werden auch die nachfolgend zusammen mit Verbindungen der vorgängigen Beispiele genannten Verbindungen der Formel I hergestellt:

Tabelle 1

Typische Vertreter von Verbindungen der Formel I, worin $R_1$ Wasserstoff ist.

| Verb. Nr. | $R_2$ | R |
|---|---|---|
| 1.1 | $CH_3$ | H |
| 1.2 | $C_2H_5$ | H |
| 1.3 | $C_3H_7$-iso | H |
| 1.4 | $C_4H_9$-sek | H |
| 1.5 | $CH_3$ | $CH_3$ |
| 1.6 | $C_2H_5$ | $CH_3$ |
| 1.7 | $C_3H_7$-iso | $CH_3$ |
| 1.8 | $C_4H_9$-sek | $CH_3$ |
| 1.9 | $CH_3$ | $C(CH_3)_3$ |
| 1.10 | $C_2H_5$ | $C(CH_3)_3$ |
| 1.11 | $C_3H_7$-iso | $C(CH_3)_3$ |
| 1.12 | $C_4H_9$-sek | $C(CH_3)_3$ |
| 1.13 | $CH_3$ | $CH_3OCH_2$ |
| 1.14 | $C_2H_5$ | $CH_3OCH_2$ |
| 1.15 | $C_3H_7$-iso | $CH_3OCH_2$ |
| 1.16 | $C_4H_9$-sek | $CH_3OCH_2$ |
| 1.17 | $CH_3$ | $CH_3OC(CH_3)_2$ |
| 1.18 | $C_2H_5$ | $CH_3OC(CH_3)_2$ |
| 1.19 | $C_3H_7$-iso | $CH_3OC(CH_3)_2$ |
| 1.20 | $C_4H_9$-sek | $CH_3OC(CH_3)_2$ |
| 1.21 | $CH_3$ | $(CH_3)_2CH$ |
| 1.22 | $C_2H_5$ | $(CH_3)_2CH$ |
| 1.23 | $C_3H_7$-iso | $(CH_3)_2CH$ |
| 1.24 | $C_4H_9$-sek | $(CH_3)_2CH$ |
| 1.25 | $CH_3$ | $CCl_3$ |
| 1.26 | $C_2H_5$ | $CCl_3$ |
| 1.27 | $C_3H_7$-iso | $CCl_3$ |
| 1.28 | $C_4H_9$-sek | $CCl_3$ |
| 1.29 | $CH_3$ | $CF_3$ |
| 1.30 | $C_2H_5$ | $CF_3$ |
| 1.31 | $C_3H_7$-iso | $CF_3CHCl$ |
| 1.32 | $C_4H_9$-sek | $CF_3$ |
| 1.33 | $CH_3$ | $C(Cl_3)CHCl$ |
| 1.34 | $C_2H_5$ | $C(Cl_3)CHCl$ |
| 1.35 | $C_3H_7$-iso | $CF_3CH_2$ |
| 1.36 | $C_4H_9$-sek | $C(Cl_3)CHCl$ |
| 1.37 | $CH_3$ | $ClCH_2CH_2CH_2$ |
| 1.38 | $C_2H_5$ | $ClCH_2CH_2CH_2$ |

Tabelle (Fortsetzung)

| Verb. Nr. | $R_2$ | R |
|---|---|---|
| 1.39 | $C_3H_7$-iso | $ClCH_2CH_2CH_2$ |
| 1.40 | $C_4H_9$-sek | $ClCH_2CH_2CH_2$ |
| 1.41 | $CH_3$ | $CH_2$=CH |
| 1.42 | $C_2H_5$ | $CH_2$=CH |
| 1.43 | $C_3H_7$-iso | $CH_2$=CH |
| 1.44 | $C_4H_9$-sek | $CH_2$=CH |
| 1.45 | $CH_3$ | $CH_2$=CH-$CH_2$ |
| 1.46 | $C_2H_5$ | $CH_2$=CH-$CH_2$ |
| 1.47 | $C_3H_7$-iso | $CH_2$=CH-$CH_2$ |
| 1.48 | $C_4H_9$-sek | $CH_2$=CH-$CH_2$ |
| 1.49 | $CH_3$ | CH≡C-$CH_2$ |
| 1.50 | $C_2H_5$ | CH≡C-$CH_2$ |
| 1.51 | $C_3H_7$-iso | CH≡C-$CH_2$ |
| 1.52 | $C_4H_9$-sek | CH≡C-$CH_2$ |
| 1.53 | $CH_3$ | $(CH_3)_2C$=CH |
| 1.54 | $C_2H_5$ | $(CH_3)_2C$=CH |
| 1.55 | $C_3H_7$-iso | $(CH_3)_2C$=CH |
| 1.56 | $C_4H_9$-sek | $(CH_3)_2C$=CH |
| 1.57 | $CH_3$ | $(Cl)_2C$=C(Cl) |
| 1.58 | $C_2H_5$ | $(Cl)_2C$=C(Cl) |
| 1.59 | $C_3H_7$-iso | $(Cl)_2C$=C(Cl) |
| 1.60 | $C_4H_9$-sek | $(Cl)_2C$=C(Cl) |
| 1.61 | $CH_3$ | $CF_3CCl_2$ |
| 1.62 | $C_2H_5$ | $CF_3CCl_2$ |
| 1.63 | $C_3H_7$-iso | $CF_3CCl_2$ |
| 1.64 | $C_4H_9$-sek | $CF_3CCl_2$ |
| 1.65 | $CH_3$ | Cyclopropyl |
| 1.66 | $C_2H_5$ | Cyclopropyl |
| 1.67 | $C_3H_7$-iso | Cyclopropyl |
| 1.68 | $C_4H_9$-sek | Cyclopropyl |
| 1.69 | $CH_3$ | 2,2-Dimethyl-cyclopropyl |
| 1.70 | $C_2H_5$ | 2,2-Dimethyl-cyclopropyl |
| 1.71 | $C_3H_7$-iso | 2,2-Dimethyl-cyclopropyl |
| 1.72 | $C_4H_9$-sek | 2,2-Dimethyl-cyclopropyl |
| 1.73 | $CH_3$ | 2,2-Dimethyl-3-(2,2-dichlorvinyl)-cyclopropyl |
| 1.74 | $C_2H_5$ | 2,2-Dimethyl-3-(2,2-dichlorvinyl)-cyclopropyl |
| 1.75 | $C_3H_7$-iso | 2,2-Dimethyl-3-(2,2-dichlorvinyl)-cyclopropyl |
| 1.76 | $C_4H_9$-sek | 2,2-Dimethyl-3-(2,2-dichlorvinyl)-cyclopropyl |
| 1.77 | $CH_3$ | Cyclobutyl |
| 1.78 | $C_2H_5$ | Cyclobutyl |
| 1.79 | $C_3H_7$-iso | Cyclobutyl |

Tabelle (Fortsetzung)

| Verb. Nr. | $R_2$ | R |
|---|---|---|
| 1.80 | $C_4H_9$-sek | Cyclobutyl |
| 1.81 | $CH_3$ | Cyclohexyl |
| 1.82 | $C_2H_5$ | Cyclohexyl |
| 1.83 | $C_3H_7$-iso | Cyclohexyl |
| 1.84 | $C_4H_9$-sek | Cyclohexyl |
| 1.85 | $CH_3$ | Phenyl |
| 1.86 | $C_2H_5$ | Phenyl |
| 1.87 | $C_3H_7$-iso | Phenyl |
| 1.88 | $C_4H_9$-sek | Phenyl |
| 1.89 | $CH_3$ | p-Chlorphenyl |
| 1.90 | $C_2H_5$ | p-Chlorphenyl |
| 1.91 | $C_3H_7$-iso | p-Chlorphenyl |
| 1.92 | $C_4H_9$-sek | p-Chlorphenyl |
| 1.93 | $CH_3$ | p-Tolyl |
| 1.94 | $C_2H_5$ | p-Tolyl |
| 1.95 | $C_3H_7$-iso | p-Tolyl |
| 1.96 | $C_4H_9$-sek | p-Tolyl |
| 1.97 | $CH_3$ | p-Nitrophenyl |
| 1.98 | $C_2H_5$ | p-Nitrophenyl |
| 1.99 | $C_3H_7$-iso | p-Nitrophenyl |
| 2.00 | $C_4H_9$-sek | p-Nitrophenyl |
| 2.1 | $CH_3$ | n-Hexyl |
| 2.2 | $C_2H_5$ | n-Hexyl |
| 2.3 | $C_3H_7$-iso | n-Hexyl |
| 2.4 | $C_4H_9$-sek | n-Hexyl |
| 2.5 | $CH_3$ | $ClCH_2C(CH_3)_2$ |
| 2.6 | $C_2H_5$ | $ClCH_2C(CH_3)_2$ |
| 2.7 | $C_3H_7$-iso | $ClCH_2C(CH_3)_2$ |
| 2.8 | $C_4H_9$-sek | $ClCH_2C(CH_3)_2$ |
| 2.9 | $CH_3$ | 1-Methylcyclopropyl |
| 2.10 | $C_2H_5$ | 1-Methylcyclopropyl |
| 2.11 | $C_3H_7$-iso | 1-Methylcyclopropyl |
| 2.12 | $C_4H_9$-sek | 1-Methylcyclopropyl |
| 2.13 | $CH_3$ | Adamantyl |
| 2.14 | $C_2H_5$ | Adamantyl |
| 2.15 | $C_3H_7$-iso | Adamantyl |
| 2.16 | $C_4H_9$-sek | Adamantyl |
| 2.17 | $C_2H_5$ | p-Fluorphenoxymethyl |
| 2.18 | $C_2H_5$ | $ClC(CH_3)_2$ |
| 2.19 | $C_2H_5$ | $CH_3CCl_2$ |
| 2.20 | $C_2H_5$ | $CH_3CH_2C(CH_3)_2$ |
| 2.21 | $C_2H_5$ | $C(CH_3)_3CH_2$ |
| 2.22 | $C_2H_5$ | $C(CH_3)_3C(CH_3)_2$ |
| 2.23 | $C_2H_5$ | $ClCH_2$ |
| 2.24 | $C_2H_5$ | $CF_3CH_2$ |
| 2.25 | $C_2H_5$ | 1-Methylcyclobutyl |
| 2.26 | $C_2H_5$ | 1-Methylcyclopentyl |
| 2.27 | $C_2H_5$ | $FCH_2C(CH_3)_2$ |
| 2.28 | $C_2H_5$ | $CH_2{=}C(CH_3)$ |

Tabelle (Fortsetzung)

| Verb. Nr. | $R_2$ | R |
|---|---|---|
| 2.29 | $C_2H_5$ | $ClCH_2CH_2$ |
| 2.30 | $C_2H_5$ | p-(tert.$C_4H_9$)phenyl |
| 2.31 | $C_2H_5$ | $CH_3CH_2CH_2$ |
| 2.32 | $C_2H_5$ | $CH_3CH_2$ |
| 2.33 | $C_2H_5$ | o-(Trifluormethyl)-phenyl |
| 2.34 | $C_2H_5$ | (R/S)-α-Methylbenzyl |
| 2.35 | $C_2H_5$ | (S)-α-Methylbenzyl |
| 2.36 | $C_2H_5$ | (R)-α-Methylbenzyl |
| 2.37 | $C_2H_5$ | α,α-Dimethylbenzyl |
| 2.38 | $C_2H_5$ | $(CH_3CH_2CH_2)_2CH$ |
| 2.39 | $C_2H_5$ | 2,3-(Difluormethylen-dioxy)-phenyl |

Der Inhalt der Tabelle besitzt illustrativen Charakter und stellt keine Begrenzung dar.

Formulierungsbeispiele für den Wirkstoff der Formel I
(% = Gewichtsprozent)

| F1. Spritzpulver | a) | b) | c) |
|---|---|---|---|
| Wirkstoff aus Tabelle 1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| F2. Emulsions-Konzentrat | |
|---|---|
| Wirkstoff aus Tabelle 1 | 10 % |
| Octylphenolpolyethylenglykolether (4-5 Mol AeO) | 3 % |
| Ca-Dodecylbenzolsulfonat | 3 % |
| Ricinusölpolyglykolether (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| F3. Stäubemittel | a) | b) |
|---|---|---|
| Wirkstoff aus Tabelle 1 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| F4. Extruder Granulat | |
|---|---|
| Wirkstoff aus Tabelle 1 | 10 % |
| Na-Ligninsulfonat | 2 % |
| Carboxethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| F5. Tabletten, Pellets | | |
|---|---|---|
| I | Wirkstoff aus Tabelle 1 | 33,0 % |
| | Methylcellulose | 0,80 % |
| | Kieselsäure hochdispers | 0,80 % |
| | Maisstärke | 8,40 % |

Methylcellulose in Wasser einrühren und quellen lassen; Kieselsäure in die Quellung einrühren und homogen suspendieren. Wirkstoff und Maisstärke mischen. In diese Mischung die wässrige Suspension einarbeiten und zu einem Teig kneten. Diese Masse durch ein Sieb (Maschenweite 12 M) granulieren und dann trocknen.

| | | |
|---|---|---|
| II | Milchzucker krist. | 22,50 % |
| | Maisstärke | 17,00 % |
| | mikrokrist. Cellulose | 16,50 % |
| | Magnesiumstearat | 1,00 % |
| Alle 4 Hilfsstoffe gut mischen<br>Phasen I und II mischen und zu Tabletten oder Pellets verpressen. | | |

Bei einer Verwendung von Verbindungen der Formel I oder entsprechender Mittel zur Bekämpfung von endoparasitären Nematoden, Cestoden und Trematoden bei Haus- und Nutztieren, wie Rindern, Schafen, Ziegen, Katzen und Hunden, können die Verbindungen oder Mittel den Tieren sowohl als Einzeldosis wie auch wiederholt verabreicht werden, wobei die einzelnen Gaben je nach Tierart vorzugsweise zwischen 0,1 und 10 mg pro kg Körpergewicht betragen. Durch eine protrahierte Verabreichung erzielt man in manchen Fällen eine bessere Wirkung oder man kann mit geringeren Gesamtdosen auskommen. Der Wirkstoff bzw. die ihn enthaltenden Mittel können auch dem Futter oder den Tränken zugesetzt werden. Das Fertigfutter enthält die Wirkstoffkombinationen vorzugsweise in einer Konzentration von 0,005 bis 0,1 Gew. %. Die Mittel können in Form von Lösungen, Emulsionen, Suspensionen, Pulver, Tabletten, Pellets, Bolussen oder Kapseln peroral den Tieren verabreicht werden. Soweit die physikalischen und toxikologischen Eigenschaften von Lösungen oder Emulsionen dies zulassen, können die Verbindungen der Formel 1 bzw. die sie enthaltenden Mittel den Tieren auch beispielsweise subcutan injiziert, intraruminal verabreicht oder mittels der Pour-on-Methode auf den Körper der Tiere appliziert werden. Ferner ist eine Verabreichung des Wirkstoffs an die Tiere auch durch Lecksteine (Salz) oder Molasse-Blöcke möglich.

Biologische Beispiele

B1. Wirkung gegen $L_1$- Larven von Lucilia sericata

1 ml einer wässrigen Suspension der zu prüfenden Aktivsubstanz werden so mit 3 ml eines speziellen Larvenzuchtmediums bei ca. 50 °C vermischt, dass ein Homogenisat von wahlweise 250 ppm oder 100 ppm Wirkstoffgehalt entsteht. In jede Reagenzglas-Probe werden ca. 30 Lucilia-Larven ($L_1$) eingesetzt. Nach 4 Tagen wird die Mortalitätsrate bestimmt. Verbindungen der Formel I erzielen mit 250 ppm eine Wirkung

von 100 %. Dieser Effekt lässt sich beispielsweise mit den Verbindungen 1.6, 2.6, 2.20, 2.34, 2.37 und 2.38 auch bei niedrigerer Dosierung erreichen, und diese Verbindungen sind auch mindestens gleich gut wirksam gegen Lucilia cuprina.

B2. Akarizide Wirkung gegen Boophilus microplus (Biarra-Stamm)

Auf einer PVC-Platte wird waagrecht ein Klebstreifen so befestigt, das darauf 10 mit Blut vollgesogene Zecken-Weibchen von Boophilus microplus (Biarra-Stamm) nebeneinander in einer Reihe mit dem Rücken aufgeklebt werden können. Jeder Zecke wird mit einer Injektionsnadel 1 µl einer Flüssigkeit injiziert, die eine 1:1-Mischung von Polyethylenglykol und Aceton darstellt und in der eine bestimmte Wirkstoffmenge von wahlweise 1, 0,5, 0,1 oder 0,01µg pro Zecke gelöst ist. Kontrolltiere erhalten eine Injection der entsprechenden Mischung ohne Wirkstoff. Nach der Behandlung werden die Tiere unter Normalbedingungen in einem Insektarium bei ca. 28° C und 80 % relativer Luftfeuchtigkeit gehalten, bis die Eiablage erfolgt und die Larven aus den Eiern der Kontrolltiere geschlüpft sind.

Die Aktivität einer geprüften Substanz wird mit der $IR_{90}$ bestimmt, d.h. es wird jene Wirkstoffdosis ermittelt, bei der noch nach 30 Tagen 9 von 10 Zeckenweibchen (= 90 %) Eier ablegen, die nicht schlupffähig sind.

Verbindungen der Formeln I erzielen eine $IR_{90}$ von 0,5 µg.

B3. Versuch an mit Nematoden (Haemonchus concortus und Trichostrongylus colubriformis) infizierten Schafen

Der Wirkstoff wird als Suspension formuliert mit einer Magensonde oder durch Injektion in den Pansen eines Schafes gegeben, das mit Haemonchus concortus und Trichostrongylus colubriformis künstlich infiziert worden ist. Pro Dosis werden 1 bis 3 Tiere verwendet. Jedes Schaf wird nur einmal mit einer einzigen Dosis behandelt, und zwar mit 0,5 mg/kg Körpergewicht. Die Evaluierung erfolgt durch Vergleich der Anzahl der vor und nach Behandlung im Kot der Schafe ausgeschiedenen Wurmeier.

Gleichzeitig und gleichartig infizierte aber unbehandelte Schafe dienen als Kontrolle. Schafe, die mit einer der Verbindungen der Formel I bei 0,5 mg/kg behandelt werden, zeigen im Vergleich zu unbehandelten, aber infizierten Vergleichsgruppen keinen Nematodenbefall (= komplette Reduktion der Wurmeier im Kot). Eine weitere Herabsetzung der Dosis auf Bereiche zwischen 0,1 und 0,2 mg/kg ergibt beispielsweise für die Verbindungen 1.6, 1.10 und 2.27 eine über 50 %ige, für die Verbindungen 1.14 und 2.34 eine über 90 %ige Reduktion.

B4. Larvizidwirkung gegen Aedes aegypti

Auf die Oberfläche von 150 ml Wasser, das sich in einem Behälter befindet, wird soviel einer 0,1 %igen acetonischen Lösung des Wrikstoffes pipettiert, dass Konzentrationen von wahlweise 10 ppm, 3,3 ppm und 1,6 ppm erhalten werden. Nach Verdunsten des Acetons wird der Behälter mit ca. 30-40 3 Tage alten Aedes-Larven beschickt. Nach 1, 2 und 5 Tagen wird die Mortalität geprüft.

Verbindungen der Formeln I bewirken in diesem Test bei einer Konzentration von 1,6 ppm bereits nach einem Tag eine vollständige Abtötung sämtlicher Larven.

B5. Milbizide Wirkung gegen Dermanyssus gallinae

2 bis 3 ml einer Testlösung (100, 10, 1 und 0,1 ppm Aktivsubstanz) werden in einen nach oben offenen Glasbehälter gegeben und ca. 200 Milben in unterschiedlichen Entwicklungsstadien in diesen Behälter eingesetzt. Der Glasbehälter wird mit einem Wattebausch verschlossen und 10 Minuten gleichmässig, bis zur vollständigen Benetzung der Milben geschüttelt. Danach wird der Behälter umgekehrt hingestellt, bis die überschüssige Testlösung von der Watte aufgesogen ist. Der Behälter wird erneut gekehrt und die behandelten Zecken zur Evaluierung der Wirksamkeit der Testsubstanzen drei Tage unter Laborbedingungen beobachtet, wobei die Mortalität als Massstab für Wirksamkeit herangezogen wird.

Die Verbindungen der Formel I zeigen in diesem Test eine gute Wirkung. So erhält man beispielsweise mit den Verbindungen 1.6, 2.6, 2.20, 2.27, 2.34, 2.37 und 2.38 bei 100 ppm eine Wirkung von 100 %.

## Patentansprüche

**Patentansprüche für folgende Vertragsstaaten : BE, CH, DE, ES, FR, GB, GR, IT, LI, LU, NL, SE**

1. Verbindungen der Formel I

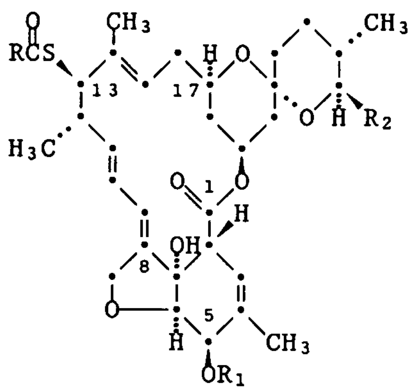

(I)

in welcher

$R_1$ Wasserstoff, eine Silyl- oder Acylgruppe,

$R_2$ Methyl, Ethyl, Isopropyl oder sek.Butyl und

R Wasserstoff,

eine geradkettige oder verzweigte $C_1$-$C_{18}$-Alkylgruppe, welche unsubstituiert oder durch 1 bis 7 Halogenatome, 1 bis 6 $C_1$-$C_6$-Alkoxygruppen, eine unsubstituierte oder halogenierte Phenoxygruppe, eine $C_1$-$C_4$-Alkylthiogruppe, eine $C_1$-$C_4$-Alkylcarbonylgruppe oder eine unsubstituierte oder durch Methyl ein- oder mehrfach substituierte $C_3$-$C_6$-Cycloalkylgruppe substituiert ist,

eine $C_2$-$C_6$-Alkenylgruppe, welche unsubstituiert oder durch 1 bis 7 Halogenatome oder 1 bis 6 $C_1$-$C_6$-Alkoxygruppen substituiert ist,

eine $C_2$-$C_6$-Alkinylgruppe, welche unsubstituiert oder durch 1 bis 7 Halogenatome oder 1 bis 6 $C_1$-$C_6$-Alkoxygruppen substituiert ist,

eine mono- bis tetracyclische cycloaliphatische Gruppe mit 3 bis 10 Kohlenstoffatomen oder eine $C_3$-$C_{10}$-Cycloalkylgruppe, welche durch 1 bis 7 Halogenatome, 1 bis 6 $C_1$-$C_6$-Alkoxygruppen oder durch $C_1$-$C_4$-Alkylgruppen substituiert ist, oder 2,2-Dimethyl-3-(2,2-dichlorvinyl)-cyclopropyl,

eine Phenylgruppe, welche unsubstituiert oder durch 1 bis 3 Substituenten aus der Gruppe bestehend aus Halogenatomen, $C_1$-$C_6$-Alkyl, $C_1$-$C_6$-Alkoxy, $C_1$-$C_4$-Alkylthio, phenyl, Nitro, Halogen-$C_1$-$C_4$-alkyl und Amin oder durch eine Difluormethylendioxygruppe, deren Sauerstoffatome an zwei benachbarten Kohlenstoffatomen sitzen, substituiert ist, oder

eine Benzylgruppe, welche unsubstituiert oder durch 1 bis 3 Substituenten aus der Gruppe bestehend aus Halogenatomen, $C_1$-$C_6$-Alkyl, $C_1$-$C_6$-Alkoxy, $C_1$-$C_4$-Alkylthio, phenyl, Nitro, Halogen-$C_1$-$C_4$-alkyl und Amin substituiert ist, wobei die beiden letztgenannten Substituenten nur an Ringkohlenstoffatomen vorliegen können,

bedeuten.

2. Verbindungen der Formel I nach Anspruch 1, in welchen für R stehende Alkyl-, Cycloalkyl-, Alkenyl- und Alkinyl-Gruppen mit 1 bis 7 Halogenatomen oder 1 bis 6 $C_1$-$C_6$-Alkoxygruppen und die Phenyl-Gruppen mit 1 bis 3 Substituenten ausgewählt aus der Gruppe bestehend aus Halogenatomen, $C_1$-$C_6$-Alkyl, $C_1$-$C_6$-Alkoxy, $C_1$-C-Alkylthio, Phenyl oder Nitro substituiert sind und $R_1$ und $R_2$ die unter Formel I in Anspruch 1 angegebenen Bedeutungen besitzen.

**3.** Verbindungen der Formel I nach Anspruch 1, worin $R_1$ Wasserstoff darstellt, $R_2$ für Methyl, Ethyl, Isopropyl oder sek.Butyl steht und R Wasserstoff, unsubstituiertes oder durch 1 bis 4 Halogenatome oder $C_1$-$C_4$-Alkoxy substituiertes $C_1$-$C_6$-Alkyl, $C_{2-4}$-C-Alkenyl, $C_2$-$C_4$-Alkinyl oder $C_3$-$C_6$-Cycloalkyl; oder unsubstituiertes oder ein- bis dreifach durch Halogen, $C_1$-$C_4$-Alkyl, $C_1$-$C_4$-Alkoxy, $C_1$-$C_4$-Alkylthio oder Nitro substituiertes Phenyl bedeutet.

**4.** Verbindungen der Formel I nach Anspruch 1, worin $R_1$ Wasserstoff darstellt, $R_2$ für Methyl oder Ethyl steht und R $C_1$-$C_7$-Alkyl, welches unsubstituiert oder durch Halogen oder $C_1$-$C_4$-Alkoxy monosubstituiert ist, Phenyl, welches durch Trifluormethyl oder die Gruppe -O-$CF_2$-O- monosubstituiert ist, wobei die beiden Sauerstoffatome an zwei benachbarten Ringkohlenstoffatomen sitzen, oder $\alpha$-Methyl- oder $\alpha,\alpha$-Dimethylbenzyl bedeutet.

**5.** Verbindungen der Formel I nach Anspruch 1, worin $R_1$ Wasserstoff darstellt, $R_2$ für Methyl oder Ethyl steht und R $C_1$-$C_7$-Alkyl, durch Chlor, Fluor oder Methoxy monosubstituiertes $C_1$-$C_4$-Alkyl, durch Trifluormethyl oder die Gruppe -O-$CF_2$-O- monosubstituiertes Phenyl, wobei die beiden Sauerstoffatome an zwei benachbarten Ringkohlenstoffatomen sitzen, oder $\alpha$-Methyl- oder $\alpha,\alpha$-Dimethylbenzyl bedeutet.

**6.** Verbindungen der Formel I nach Anspruch 1, worin R für tert.Butyl steht und $R_1$ und $R_2$ die unter Formel I in Anspruch 1 angegebenen Bedeutungen besitzen.

**7.** Verbindungen der Formel I nach Anspruch 6, worin $R_1$ Wasserstoff darstellt, $R_2$ für Methyl, Ethyl oder Isopropyl steht und R tert.Butyl bedeutet.

**8.** Verbindungen der Formel I nach Anspruch 1, worin $R_1$ tert.Butyl-dimethylsilyl bedeutet.

**9.** Verbindung nach Anspruch 1, ausgewählt aus der Gruppe:
13$\beta$-Formylthio-milbemycin D
13$\beta$-Acetylthio-milbemycin D
13$\beta$-Pivaloylthio-milbemycin D
13$\beta$-Formylthio-milbemycin $A_3$
13$\beta$-Acetylthio-milbemycin $A_3$
13$\beta$-Pivaloylthio-milbemycin $A_3$
13$\beta$-Formylthio-milbemycin $A_4$
13$\beta$-Acetylthio-milbemycin $A_4$
13$\beta$-Pivaloylthio-milbemycin $A_4$
13$\beta$-(2'-Methoxy-2'-methylpropionylthio)milbemycin D
13$\beta$-(2'-Methoxy-2'-methylpropionylthio)-milbemycin $A_4$
13$\beta$-Trichloracetylthio-milbemycin $A_4$
13$\beta$-(4'-Chlor-butanoylthio)milbemycin $A_4$
13$\beta$-Trichloracryloylthio-milbemycin $A_4$
13$\beta$-Cyclopropancarbonylthio-milbemycin $A_4$
13$\beta$-Cyclobutancarbonylthio-milbemycin $A_4$
13$\beta$-Heptanoylthio-milbemycin $A_4$
13$\beta$-(3'-Chlor-2',2'-dimethylpropionylthio)-milbemycin $A_4$
13$\beta$-(3'-Chlor-2',2'-dimethylpropionylthio)-milbemycin $A_3$
13$\beta$-(l'Methylcyclopropancarbonylthio)-milbemycin $A_4$
13$\beta$-(1'-Methyl-cyclopropancarbonylthio)-milbemycin $A_3$
13$\beta$-(1-Adamantancarbonylthio)-milbemycin $A_4$
13$\beta$-(p-Fluorphenoxyacetylthio)-milbemycin $A_4$
13$\beta$-(2′-Chlor-2′-methyl-propionylthio)-milbemycin $A_4$
13$\beta$-(2′,2′-Dichlorpropionylthio)-milbemycin $A_4$
13$\beta$-(2′,2′-Dimethylbutanoylthio)-milbemycin $A_4$
13$\beta$-(3′,3′-Dimethylbutanoylthio)-milbemycin $A_4$
13$\beta$-(2′,2′,3′,3′-Tetramethylbutanoylthio)-milbemycin $A_4$
13$\beta$-(p-Chlorbenzoylthio)-milbemycin $A_4$
13$\beta$-(3′,3′,3′-Trifluorpropionylthio)-milbemycin $A_4$
13$\beta$-Chloracetylthio-milbemycin $A_4$

13$\beta$-(2′-Chlor-3′,3′,3′-Trifluorpropionylthio)-milbemycin $A_4$
13$\beta$-(3′,3′,3′-Trifluorpropionylthio)-milbemycin $A_4$
13$\beta$-[o-(Trifluormethyl)-benzoylthio]-milbemycin $A_4$
13$\beta$-($\alpha$,$\alpha$-Dimethylbenzylcarbonylthio)-milbemycin $A_4$
13$\beta$-(2-n-Propyl-n-valeroylthio)-milbemycin $A_4$
13$\beta$-[(2,3-Difluormethylendioxy)-benzoylthio]-milbemycin $A_4$
13$\beta$-($\alpha$-Methylbenzylcarbonylthio)-milbemycin $A_4$ und
13$\beta$-(Methoxyacetylthio)-milbemycin $A_4$.

**10.** Verbindung nach Anspruch 1, ausgewählt aus der Gruppe:
5-O-tert.Butyldimethylsilyl-13$\beta$-Formylthio-milbemycin D
5-O-tert.Butyldimethylsilyl-13$\beta$-Acetylthio-milbemycin D
5-O-tert.Butyldimethylsilyl-13$\beta$-Pivaloylthio-milbemycin D
5-O-tert.Butyldimethylsilyl-13$\beta$-Formylthio-milbemycin $A_3$
5-O-tert.Butyldimethylsilyl-13$\beta$-Acetylthio-milbemycin $A_3$
5-O-tert.Butyldimethylsilyl-13$\beta$-Pivaloylthio-milbemycin $A_3$
5-O-tert.Butyldimethylsilyl-13$\beta$-Formylthio-milbemycin $A_4$
5-O-tert.Butyldimethylsilyl-13$\beta$-Acetylthio-milbemycin $A_4$
5-O-tert.Butyldimethylsilyl-13$\beta$-Pivaloylthio-milbemycin $A_4$
5-O-tert.Butyldimethylsilyl-13$\beta$-(2′-Methoxy-2′-methylpropionylthio)-milbemycin D
5-O-tert.Butyldimethylsilyl-13$\beta$-(2′-Methoxy-2′-methylpropionylthio)-milbemycin $A_4$
5-O-tert.Butyldimethylsilyl-13$\beta$-Trichloracetylthio-milbemycin $A_4$
5-O-tert.Butyldimethylsilyl-13$\beta$-(4′-Chlor-butanoylthio)milbemycin $A_4$
5-O-tert.Butyldimethylsilyl-13$\beta$-Trichloracryloylthio-milbemycin $A_4$
5-O-tert.Butyldimethylsilyl-13$\beta$-Cyclopropancarbonylthio-milbemycin $A_4$
5-O-tert.Butyldimethylsilyl-13$\beta$-Cyclobutancarbonylthio-milbemycin $A_4$
5-O-tert.Butyldimethylsilyl-13$\beta$-Heptanoylthio-milbemycin $A_4$
5-O-tert.Butyldimethylsilyl-13$\beta$-(3′-Chlor-2′,2′-dimethyl-propionylthio)-milbemycin $A_4$
5-O-tert.Butyldimethylsilyl-13$\beta$-(3′-Chlor-2′,2′-dimethyl-propionylthio)-milbemycin $A_3$
5-O-tert.Butyldimethylsilyl-13$\beta$-(1′-Methyl-cyclopropancarbonylthio)-milbemycin $A_4$
5-O-tert.Butyldimethylsilyl-13$\beta$-(1′-Methyl-cyclopropancarbonylthio)-milbemycin $A_3$
5-O-tert.Butyldimethylsilyl-13$\beta$-(1-Adamantancarbonylthio)-milbemycin $A_4$
5-O-tert.Butyldimethylsilyl-13$\beta$-(p-Fluorphenoxyacetylthio)-milbemycin $A_4$
5-O-tert.Butyldimethylsilyl-13$\beta$-(2′-Chlor-2′-methyl-propionylthio)-milbemycin $A_4$
5-O-tert.Butyldimethylsilyl-13$\beta$-(2′,2′-Dichlorpropionylthio)-milbemycin $A_4$
5-O-tert.Butyldimethylsilyl-13$\beta$-(2′,2′-Dimethylbutanoylthio)-milbemycin $A_4$
5-O-tert.Butyldimethylsilyl-13$\beta$-(3′,3′-Dimethylbutanoylthio)-milbemycin $A_4$
5-O-tert.Butyldimethylsilyl-13$\beta$-(2′,2′,3′,3′-Tetramethylbutanoylthio)-milbemycin $A_4$
5-O-tert.Butyldimethylsilyl-13$\beta$-(p-Chlorbenzoylthio)-milbemycin $A_4$
5-O-tert.Butyldimethylsilyl-13$\beta$-(3′,3′,3′-Trifluorpropionylthio)-milbemycin $A_4$
5-O-tert.Butyldimethylsilyl-13$\beta$-Chloracetylthio-milbemycin $A_4$
5-O-tert.Butyldimethylsilyl-13$\beta$-(2′-Chlor-3′,3′,3′-Trifluorpropionylthio)-milbemycin $A_4$
5-O-tert.Butyldimethylsilyl-13$\beta$-(3′,3′,3′-Trifluorpropionylthio)-milbemycin $A_4$
5-O-tert.Butyldimethylsilyl-13$\beta$-($\alpha$-Methylbenzylcarbonylthio)-milbemycin $A_4$
5-0-tert.Butyldimethylsilyl-13$\beta$-(Methoxyacetylthio)-milbemycin $A_4$
5-0-tert.Butyldimethylsilyl-13$\beta$-[o-(Trifluormethyl)-benzoylthio]-milbemycin $A_4$
5-0-tert.Butyldimethylsilyl-13$\beta$-($\alpha$,$\alpha$-Dimethylbenzylcarbonylthio)-milbemycin $A_4$
5-0-tert.Butyldimethylsilyl-13$\beta$-(2-n-Propyl-n-valeroylthio)-milbemycin $A_4$
5-0-tert.Butyldimethylsilyl-13$\beta$-[(2,3-Difluormethylendioxy)-benzoylthio]-milbemycin $A_4$.

**11.** Verfahren zur Herstellung von Verbindungen der Formel I

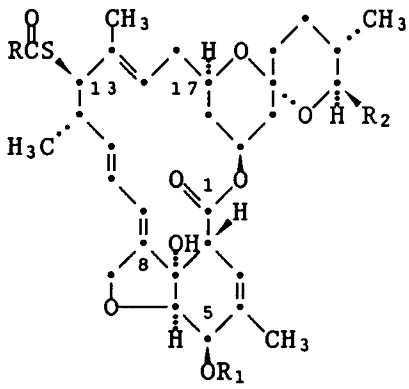

(I)

in welcher

- $R_1$ Wasserstoff, eine Silyl- oder Acylgruppe,
- $R_2$ Methyl, Ethyl, Isopropyl oder sek.Butyl und
- R Wasserstoff,
eine geradkettige oder verzweigte $C_{1-18}$-Alkylgruppe, welche unsubstituiert oder durch 1 bis 7 Halogenatome, 1 bis 6 $C_1$-$C_6$-Alkoxygruppen, eine unsubstituierte oder halogenierte Phenoxygruppe, eine $C_1$-$C_4$-Alkylthiogruppe, eine $C_1$-$C_4$-Alkylcarbonylgruppe oder eine unsubstituierte oder durch Methyl ein- oder mehrfach substituierte $C_3$-$C_6$-Cycloalkylgruppe substituiert ist,
eine $C_2$-$C_6$-Alkenylgruppe, welche unsubstituiert oder durch 1 bis 7 Halogenatome oder 1 bis 6 $C_1$-$C_6$-Alkoxygruppen substituiert ist,
eine $C_2$-$C_6$-Alkinylgruppe, welche unsubstituiert oder durch 1 bis 7 Halogenatome oder 1 bis 6 $C_1$-$C_6$-Alkoxygruppen substituiert ist,
eine mono- bis tetracyclische cycloaliphatische Gruppe mit 3 bis 10 Kohlenstoffatomen oder eine $C_3$-$C_{10}$-Cycloalkylgruppe, welche durch 1 bis 7 Halogenatome, 1 bis 6 $C_1$-$C_6$-Alkoxygruppen oder durch $C_1$-$C_4$-Alkylgruppen substituiert ist, oder 2,2-Dimethyl-3-(2,2-dichlorvinyl)-cyclopropyl,
eine Phenylgruppe, welche unsubstituiert oder durch 1 bis 3 Substituenten aus der Gruppe bestehend aus Halogenatomen, $C_1$-$C_6$-Alkyl, $C_1$-$C_6$-Alkoxy, $C_1$-$C_4$-Alkylthio, Phenyl, Nitro, Halogen-$C_1$-$C_4$-alkyl und Amin oder durch eine Difluormethylendioxygruppe, deren Sauerstoffatome an zwei benachbarten Kohlenstoffatomen sitzen, substituiert ist, oder
eine Benzylgruppe, welche unsubstituiert oder durch 1 bis 3 Substituenten aus der Gruppe bestehend aus Halogenatomen, $C_1$-$C_6$-Alkyl, $C_1$-$C_6$-Alkoxy, $C_1$-$C_4$-Alkylthio, Phenyl, Nitro, Halogen-$C_1$-$C_4$-alkyl und Amin substituiert ist, wobei die beiden letztgenannten Substituenten nur an Ringkohlenstoffatomen vorliegen können, bedeuten, dadurch gekennzeichnet, dass

man Verbindungen der Formel II

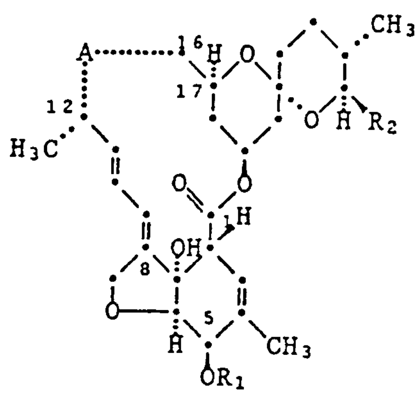

(II)

worin A für eine Gruppe a, b oder c

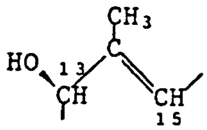 ,

(a)

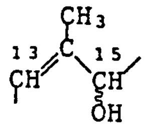

(b)

oder

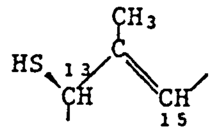 ,

(c)

[= 13β-Hydroxy-$\Delta^{14,15}$] [= $\Delta^{13,14}$-15-Hydroxy] [= 13β-Mercapto-$\Delta^{14,15}$]

steht, $R_1$ eine Schutzgruppe bedeutet und $R_2$ die unter Formel I angegebenen Bedeutungen besitzt, mit einem zur Einführung oder zum Aufbau einer 13β-Thiolestergruppe geeigneten Reagenz behandelt, woraufhin die $R_1$-Schutzgruppe, sofern freie 5-Hydroxy-Verbindungenerwünscht sind, abgespalten wird.

**12.** Verfahren nach Anspruch 11, dadurch gekennzeichnet, dass zur Einführung oder zum Aufbau einer 13β-Thiolestergruppe in Verbindungen der Formel II als Reagenz eingesetzt wird

i) bei Verbindungen der Formeln IIa oder IIb:

a) eine Thiocarbonsäure der Formel III

RCOSH (III)

oder

b) ein Thioamid der Formel IV

$RCSN(Alkyl)_2$ (IV),

worin die Alkylreste 1 bis 4 Kohlenstoffatome umfassen und vorzugsweise Methyl darstellen, oder

ii) bei Verbindungen der Formel IIc:

c) ein Säurehalogenid der Formel V

RCOhal (V)

worin hal Halogen, bevorzugt Chlor oder Brom bedeutet, oder

d) ein Säureanhydrid der Formel VI

$(RCO)_2O$ (VI)

in welchen R die unter Formel I angegebenen Bedeutungen besitzt.

**13.** Verfahren nach Anspruch 12, dadurch gekennzeichnet, dass die Reaktion von Verbindungen der Formel IIa oder IIb mit einer Thiosäure der Formel III oder einem Thioamid der Formel IV in Gegenwart eines Orthoesters der Formel VII

$R_3C(OR_4)_3$ (VII)

worin $R_3$ Wasserstoff oder $C_1$-$C_4$-Alkyl und $R_4$ $C_1$-$C_4$-Alkyl bedeuten und zusätzlich in Gegenwart einer katalytisch wirkenden Säure bei Temperaturen im Bereich von 0° bis 150°C durchgeführt wird.

**14.** Verfahren nach Anspruch 12, dadurch gekennzeichnet, dass die Reaktion von Verbindungen der Formel IIc mit einem Säurechlorid oder Säurebromid der Formel V oder einem Säureanhydrid der Formel VI in einem reaktionsinerten Lösungsmittel bei Temperaturen von -20° bis 100°C durchgeführt wird.

15. Mittel zur Bekämpfung von Schädlingen, dadurch gekennzeichnet, dass es neben Trägermitteln, Verteilungsmitteln oder Träger- und Verteilungsmitteln als Wirkstoff mindestens eine Verbindung der Formel I gemäss Anspruch 1 enthält.

16. Verfahren zur Bekämpfung von Schädlingen, dadurch gekennzeichnet, dass man eine pestizid-wirksame Menge mindestens einer Verbindung der Formel I gemäss Anspruch 1 auf oder in die Wirtspflanzen oder sonstigen Lebensräume der Schädlinge mit Ausnahme menschlicher oder tierischer Körper appliziert.

17. Verfahren nach Anspruch 16, dadurch gekennzeichnet, dass die zu bekämpfenden Schädlinge Tiere befallende Endo- oder Ektoparasiten sind.

18. Verfahren nach Anspruch 16, dadurch gekennzeichnet, dass die zu bekämpfenden Schädlinge pflanzenschädigende Parasiten sind.

19. Verbindungen der Formel I gemäss Anspruch 1 zur Verwendung als antiparasitäres Mittel gegen Tiere befallende Endo- und Ektoparasiten.

20. Verwendung einer Verbindung der Formel I gemäss Anspruch 1 zur Herstellung eines Arzneimittels zur Bekämpfung von Tiere befallenden Endo- und Ektoparasiten.

**Patentansprüche für folgenden Vertragsstaat : AT**

1. Mittel zur Bekämpfung von Schädlingen, dadurch gekennzeichnet, dass es neben Trägermitteln, Verteilungsmitteln oder Träger- und Verteilungsmitteln als Wirkstoff mindestens eine Verbindung der Formel I

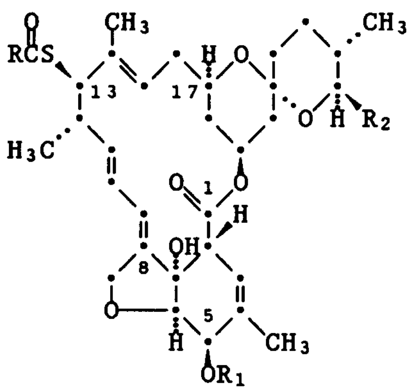

(I)

in welcher

$R_1$ Wasserstoff, eine Silyl- oder Acylgruppe,

$R_2$ Methyl, Ethyl, Isopropyl oder sek.Butyl und

R Wasserstoff,

eine geradkettige oder verzweigte $C_1$-$C_{18}$-Alkylgruppe, welche unsubstituiert oder durch 1 bis 7 Halogenatome, 1 bis 6 $C_1$-$C_6$-Alkoxygruppen, eine unsubstituierte oder halogenierte Phenoxygruppe, eine $C_1$-$C_4$-Alkylthiogruppe, eine $C_1$-$C_4$-Alkylcarbonylgruppe oder eine unsubstituierte oder durch Methyl ein- oder mehrfach substituierte $C_3$-$C_6$-Cycloalkylgruppe substituiert ist,

eine $C_2$-$C_6$-Alkenylgruppe, welche unsubstituiert oder durch 1 bis 7 Halogenatome oder 1 bis 6 $C_1$-$C_6$-Alkoxygruppen substituiert ist,

eine $C_2$-$C_6$-Alkinylgruppe, welche unsubstituiert oder durch 1 bis 7 Halogenatome oder 1 bis 6 $C_1$-$C_6$-Alkoxygruppen substituiert ist,

eine mono- bis tetracyclische cycloaliphatische Gruppe mit 3 bis 10 Kohlenstoffatomen oder eine $C_3$-$C_{10}$-Cycloalkylgruppe, welche durch 1 bis 7 Halogenatome, 1 bis 6 $C_1$-$C_6$-Alkoxygruppen oder durch $C_1$-$C_4$-Alkylgruppen substituiert ist, oder 2,2-Dimethyl-3-(2,2-dichlorvinyl)-cyclopropyl,
eine Phenylgruppe, welche unsubstituiert oder durch 1 bis 3 Substituenten aus der Gruppe bestehend aus Halogenatomen, $C_1$-$C_6$-Alkyl, $C_1$-$C_6$-Alkoxy, $C_1$-$C_4$-Alkylthio, Phenyl, Nitro, Halogen-$C_1$-$C_4$-alkyl und Amin oder durch eine Difluormethylendioxygruppe, deren Sauerstoffatome an zwei benachbarten Kohlenstoffatomen sitzen, substituiert ist, oder
eine Benzylgruppe, welche unsubstituiert oder durch 1 bis 3 Substituenten aus der Gruppe bestehend aus Halogenatomen, $C_1$-$C_6$-Alkyl, $C_1$-$C_6$-Alkoxy, $C_1$-$C_4$-Alkylthio, Phenyl, Nitro, Halogen-$C_1$-$C_4$-alkyl und Amin substituiert ist, wobei die beiden letztgenannten Substituenten nur an Ringkohlenstoffatomen vorliegen können,
bedeuten, enthält.

**2.** Mittel nach Anspruch 1, dadurch gekennzeichnet, dass es eine Verbindung der Formel I enthält, in welcher für R stehende Alkyl-, Cycloalkyl-, Alkenyl- und Alkinyl-Gruppen mit 1 bis 7 Halogenatomen oder 1 bis 6 $C_1$-$C_6$-Alkoxygruppen und die Phenyl-Gruppen mit 1 bis 3 Substituenten ausgewählt aus der Gruppe bestehend aus Halogenatomen, $C_1$-$C_6$-Alkyl, $C_1$-$C_6$-Alkoxy, $C_1$-$C_4$-Alkylthio, Phenyl oder Nitro substituiert sind und $R_1$ und R die unter Formel I in Anspruch 1 angegebenen Bedeutungen besitzen.

**3.** Mittel nach Anspruch 1, dadurch gekennzeichnet, dass es eine Verbindung der Formel I enthält, in welcher $R_1$ Wasserstoff darstellt, $R_2$ für Methyl, Ethyl, Isopropyl oder sek.Butyl steht und R Wasserstoff, unsubstituiertes oder durch 1 bis 4 Halogenatome oder $C_1$-$C_4$-Alkoxy substituiertes $C_1$-$C_6$-Alkyl, $C_2$-$C_4$-Alkenyl, $C_2$-$C_4$-Alkinyl oder $C_3$-$C_6$-Cycloalkyl; oder unsubstituiertes oder ein- bis dreifach durch Halogen, $C_1$-$C_4$-Alkyl, $C_1$-$C_4$-Alkoxy, $C_1$-$C_4$-Alkylthio oder Nitro substituiertes Phenyl bedeutet.

**4.** Mittel nach Anspruch 1, dadurch gekennzeichnet, dass es eine Verbindung der Formel I enthält, in welcher $R_1$ Wasserstoff darstellt, $R_2$ für Methyl oder Ethyl steht und R $C_1$-$C_7$-Alkyl, welches unsubstituiert oder durch Halogen oder $C_1$-$C_4$-Alkoxy monosubstituiert ist, Phenyl, welches durch Trifluormethyl oder die Gruppe -O-$CF_2$-O- monosubstituiert ist, wobei die beiden Sauerstoffatome an zwei benachbarten Ringkohlenstoffatomen sitzen, oder $\alpha$-Methyl- oder $\alpha,\alpha$-Dimethylbenzyl bedeutet.

**5.** Mittel nach Anspruch 1, dadurch gekennzeichnet, dass es eine Verbindung der Formel I enthält, in welcher $R_1$ Wasserstoff darstellt, $R_2$ für Methyl oder Ethyl steht und R $C_1$-$C_7$-Alkyl, durch Chlor, Fluor oder Methoxy monosubstituiertes $C_1$-$C_4$-Alkyl, durch Trifluormethyl oder die Gruppe -O-$CF_2$-O- monosubstituiertes Phenyl, wobei die beiden Sauerstoffatome an zwei benachbarten Ringkohlenstoffatomen sitzen, oder $\alpha$-Methyl- oder $\alpha,\alpha$-Dimethylbenzyl bedeutet.

**6.** Mittel nach Anspruch 1, dadurch gekennzeichnet, dass es eine Verbindung der Formel I enthält, in welcher R für tert.Butyl steht und $R_1$ und $R_2$ die unter Formel I in Anspruch 1 angegebenen Bedeutungen besitzen.

**7.** Mittel nach Anspruch 1, dadurch gekennzeichnet, dass es eine Verbindung der Formel I enthält, in welcher $R_1$ Wasserstoff darstellt, $R_2$ für Methyl, Ethyl oder Isopropyl steht und R tert.Butyl bedeutet.

**8.** Mittel nach Anspruch 1, dadurch gekennzeichnet, dass es eine Verbindung der Formel I enthält, in welcher $R_1$ tert.Butyl-dimethylsilyl bedeutet.

**9.** Mittel nach Anspruch 1, dadurch gekennzeichnet, dass es eine Verbindung der Formel I, ausgewählt aus der Gruppe:
13$\beta$-Formylthio-milbemycin D
13$\beta$-Acetylthio-milbemycin D
13$\beta$-Pivaloylthio-milbemycin D
13$\beta$-Formylthio-milbemycin $A_3$
13$\beta$-Acetylthio-milbemycin $A_3$
13$\beta$-Pivaloylthio-milbemycin $A_3$
13$\beta$-Formylthio-milbemycin $A_4$
13$\beta$-Acetylthio-milbemycin $A_4$
13$\beta$-Pivaloylthio-milbemycin $A_4$.
13$\beta$-(2'-Methoxy-2'-methylpropionylthio)-milbemycin D

13β-(2'-Methoxy-2'-methylpropionylthio)-milbemycin $A_4$
13β-Trichloracetylthio-milbemycin $A_4$
13β-(4'-Chlor-butanoylthio)milbemycin $A_4$
13β-Trichloracryloylthio-milbemycin $A_4$
13β-Cyclopropancarbonylthio-milbemycin $A_4$
13β-Cyclobutancarbonylthio-milbemycin $A_4$
13β-Heptanoylthio-milbemycin $A_4$
13β-(3'-Chlor-2'2'-dimethyl-proplonylthio-milbemycin $A_4$
13β-(3'-Chlor-2',2'-dimethyl-propionylthio)-milbemycin $A_3$
13β-(1'-Methyl-cyclopropancarbonylthio)-milbemycin $A_4$
13β-(1'-Methyl-cyclopropancarbonylthio)-milbemycin $A_3$
13β-(1-Adamantancarbonylthio)-milbemycin $A_4$
13β-(p-Fluorphenoxyacetylthio)-milbemycin $A_4$
13β-(2'-Chlor-2'-methyl-propionylthio)-milbemycin $A_4$
13β-(2',2'-Dichlorpropionylthio)-milbemycin $A_4$
13β-(2',2'-Dimethylbutanoylthio)-milbemycin $A_4$
13β-(3',3'-Dimethylbutanoylthio)-milbemycin $A_4$
13β-(2',2',3',3'-Tetramethylbutanoylthio)-milbemycin $A_4$
13β-(p-Chlorbenzoylthio)-milbemycin $A_4$
13β-(3',3',3'-Trifluorpropionylthio)-milbemycin $A_4$
13β-Chloracetylthio-milbemycin $A_4$
13β-(2'-Chlor-3',3',3'-Trifluorpropionylthio)-milbemycin $A_4$
13β-(3',3',3'-Trifluorpropionylthio)-milbemycin $A_4$
13β-[o-(Trifluormethyl)-benzoylthio]-milbemycin $A_4$
13β-(α,α-Dimethylbenzylcarbonylthio)-milbemycin $A_4$
13β-(2-n-Propyl-n-valeroylthio)-milbemycin $A_4$
13β-[2,3-Difluormethylendioxy)-benzoylthio]-milbemycin $A_4$
13β-(α-Methylbenzylcarbonylthio)-milbemycin $A_4$ und
13β-(Methoxyacetylthio)-milbemycin $A_4$,
enthält.

**10.** Mittel nach Anspruch 1, dadurch gekennzeichnet, dass es eine Verbindung der Formel I, ausgewählt aus der Gruppe:
5-O-tert.Butyldimethylsilyl-13β-Formylthio-milbemycin D
5-O-tert.Butyldimethylsilyl-13β-Acetylthio-milbemycin D
5-O-tert.Butyldimethylsilyl-13β-Pivaloylthio-milbemycin D
5-O-tert.Butyldimethylsilyl-13β-Formylthio-milbemycin $A_3$
5-O-tert.Butyldimethylsilyl-13β-Acetylthio-milbemycin $A_3$
5-O-tert.Butyldimethylsilyl-13β-Pivaloylthio-milbemycin $A_3$
5-O-tert.Butyldimethylsilyl-13β-Formylthio-milbemycin $A_4$
5-O-tert.Butyldimethylsilyl-13β-Acetylthio-milbemycin $A_4$
5-O-tert.Butyldimethylsilyl-13β-Pivaloylthio-milbemycin $A_4$
5-O-tert.Butyldimethylsilyl-13β-(2'-Methoxy-2'-methylpropionylthio)-milbemycin D
5-O-tert.Butyldimethylsilyl-13β-(2'-Methoxy-2'-methylpropionylthio)-milbemycin $A_4$
5-O-tert.Butyldimethylsilyl-13β-Trichloracetylthio-milbemycin $A_4$
5-O-tert.Butyldimethylsilyl-13β-(4'-Chlor-butanoylthio)milbemycin $A_4$
5-O-tert.Butyldimethylsilyl-13β-Trichloracryloylthio-milbemycin $A_4$
5-O-tert.Butyldimethylsilyl-13β-Cyclopropancarbonylthio-milbemycin $A_4$
5-O-tert.Butyldimethylsilyl-13β-Cyclobutancarbonylthio-milbemycin $A_4$
5-O-tert.Butyldimethylsilyl-13β-Heptanoylthio-milbemycin $A_4$
5-O-tert.Butyldimethylsilyl-13β-(3'-Chlor-2',2'-dimethyl-propionylthio)-milbemycin $A_4$
5-O-tert.Butyldimethylsilyl-13β-(3'-Chlor-2',2'-dimethyl-propionylthio)-milbemycin $A_3$
5-O-tert.Butyldimethylsilyl-13β-(1'-Methyl-cyclopropancarbonylthio)-milbemycin $A_4$
5-O-tert.Butyldimethylsilyl-13β-(1'-Methyl-cyclopropancarbonylthio)-milbemycin $A_3$
5-O-tert.Butyldimethylsilyl-13β-(1-Adamantancarbonylthio)-milbemycin $A_4$
5-O-tert.Butyldimethylsilyl-13β-(p-Fluorphenoxyacetylthio)-milbemycin $A_4$
5-O-tert.Butyldimethylsilyl-13β-(2'Chlor-2'methyl-propionylthio)-milbemycin $A_4$
5-O-tert.Butyldimethylsilyl-13β-(2',2'-Dichlorpropionylthio)-milbemycin $A_4$

5-O-tert.Butyldimethylsilyl-13β-(2',2'-Dimethylbutanoylthio)-milbemycin $A_4$
5-O-tert.Butyldimethylsilyl-13β-(3',3'-Dimethylbutanoylthio)-milbemycin $A_4$
5-O-tert.Butyldimethylsilyl-13β-(2',2',3',3'-Tetramethylbutanoylthio)-milbemycin $A_4$
5-O-tert.Butyldimethylsilyl 13β-(p-Chlorbenzoylthio)-milbemycin $A_4$
5-O-tert.Butyldimethylsilyl-13β-(3',3',3'-Trifluorpropionylthio)-milbemycin $A_4$
5-O-tert.Butyldimethylsilyl-13β-Chloracetylthio-milbemycin $A_4$
5-O-tert.Butyldimethylsilyl-13β-(2'Chlor-3',3',3'-Trifluorpropionylthio)-milbemycin $A_4$
5-O-tert.Butyldimethylsilyl-13β-(3',3'3'-Trifluorpropionylthio)-milbemycin $A_4$
5-O-tert.Butyldimethylsilyl-13β-(α-Methylbenzylcarbonylthio)-milbemycin $A_4$
5-O-tert.Butyldimethylsilyl-13β-(Methoxyacetylthio)-milbemycin $A_4$
5-O-tert.Butyldimethylsilyl-13β-[o-(Trifluormethyl)-benzoylthio]-milbemycin $A_4$
5-O-tert.Butyldimethylsilyl-13β-(α,α-Dimethylbenzylcarbonylthio)-milbemycin $A_4$
5-O-tert.Butyldimethylsilyl-13β-(2-n-Propyl-n-valeroylthio)-milbemycin $A_4$
und
5-O-tert.Butyldimethylsilyl-13β-[(2,3-Difluormethylendioxy)-benzoylthio]-milbemycin $A_4$, enthält.

**11.** Verfahren zur Herstellung von Verbindungen der Formel I

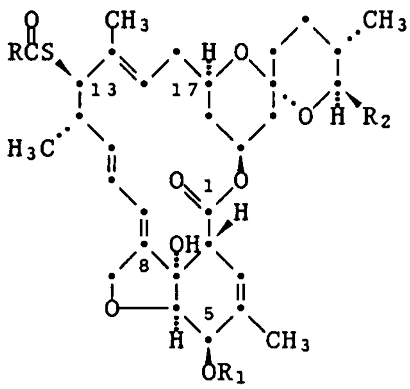

(I)

in welcher

$R_1$ Wasserstoff, eine Silyl- oder Acylgruppe,

$R_2$ Methyl, Ethyl, Isopropyl oder sek.Butyl und

R Wasserstoff,
eine geradkettige oder verzweigte $C_1$-$C_{18}$-Alkylgruppe, welche unsubstituiert oder durch 1 bis 7 Halogenatome, 1 bis 6 $C_1$-$C_6$-Alkoxygruppen, eine unsubstituierte oder halogenierte Phenoxygruppe, eine $C_1$-$C_4$-Alkylthiogruppe, eine $C_1$-$C_4$-Alkylcarbonylgruppe oder eine unsubstituierte oder durch Methyl ein- oder mehrfach substituierte $C_3$-$C_6$-Cycloalkylgruppe substituiert ist,
eine $C_2$-$C_6$-Alkenylgruppe, welche unsubstituiert oder durch 1 bis 7 Halogenatome oder 1 bis 6 $C_1$-$C_6$-Alkoxygruppen substituiert ist,
eine $C_2$-$C_6$-Alkinylgruppe, welche unsubstituiert oder durch 1 bis 7 Halogenatome oder 1 bis 6 $C_1$-$C_6$-Alkoxygruppen substituiert ist,
eine mono- bis tetracyclische cycloaliphatische Gruppe mit 3 bis 10 Kohlenstoffatomen oder eine $C_3$-$C_{10}$-Cycloalkylgruppe, welche durch 1 bis 7 Halogenatome, 1 bis 6 $C_1$-$C_6$-Alkoxygruppen oder durch $C_1$-$C_4$-Alkylgruppen substituiert ist, oder 2,2-Dimethyl-3-(2,2-dichlorvinyl)-cyclopropyl,
eine Phenylgruppe, welche unsubstituiert oder durch 1 bis 3 Substituenten aus der Gruppe bestehend aus Halogenatomen, $C_1$-$C_6$-Alkyl, $C_1$-$C_6$-Alkoxy, $C_1$-$C_4$-Alkylthio, Phenyl, Nitro, Halogen-$C_1$-$C_4$-alkyl und Amin oder durch eine Difluormethylendioxygruppe, deren Sauerstoffatome an zwei benachbarten Kohlenstoffatomen sitzen, substituiert ist, oder
eine Benzylgruppe, welche unsubstituiert oder durch 1 bis 3 Substituenten aus der Gruppe bestehend aus Halogenatomen, $C_1$-$C_6$-Alkyl, $C_1$-$C_6$-Alkoxy, $C_1$-$C_4$-Alkylthio, Phenyl, Nitro, Halogen-$C_1$-$C_4$-alkyl und Amin substituiert ist, wobei die beiden letztgenannten Substituenten nur an Ringkohlenstoffatomen vorliegen können,, bedeuten, dadurch gekennzeichnet, dass

man Verbindungen der Formel II

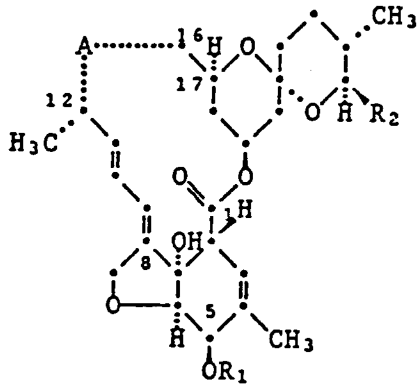

(II)

worin A für eine Gruppe a, b oder c

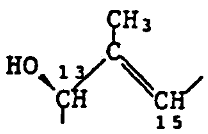 , 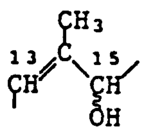 oder 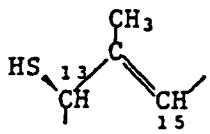 ,

(a) (b) (c)

[= 13β-Hydroxy-$\Delta^{14,15}$] [= $\Delta^{13,14}$-15-Hydroxy] [= 13β-Mercapto-$\Delta^{14,15}$]

steht, $R_1$ eine Schutzgruppe bedeutet und $R_2$ die unter Formel I angegebenen Bedeutungen besitzt, mit einem zur Einführung oder zum Aufbau einer 13β-Thiolestergruppe geeigneten Reagenz behandelt, woraufhin die $R_1$-Schutzgruppe, sofern freie 5-Hydroxy-Verbindungenerwünscht sind, abgespalten wird.

**12.** Verfahren nach Anspruch 11, dadurch gekennzeichnet, dass zur Einführung oder zum Aufbau einer 13β-Thiolestergruppe in Verbindungen der Formel II als Reagenz eingesetzt wird

i) bei Verbindungen der Formeln IIa oder IIb:

a) eine Thiocarbonsäure der Formel III

RCOSH (III)

oder

b) ein Thioamid der Formel IV

$RCSN(Alkyl)_2$ (IV) ,

worin die Alkylreste 1 bis 4 Kohlenstoffatome umfassen und vorzugsweise Methyl darstellen,
oder

ii) bei Verbindungen der Formel IIc:

c) ein Säurehalogenid der Formel V

RCOhal (V)

worin hal Halogen, bevorzugt Chlor oder Brom bedeutet,
oder

d) ein Säureanhydrid der Formel VI

$(RCO)_2O$ (VI)

in welchen R die unter Formel I angegebenen Bedeutungen besitzt.

**13.** Verfahren nach Anspruch 12, dadurch gekennzeichnet, dass die Reaktion von Verbindungen der Formel IIa oder IIb mit einer Thiosäure der Formel III oder einem Thioamid der Formel IV in Gegenwart eines Orthoesters der Formel VII

$R_3C(OR_4)_3$ (VII)

worin $R_3$ Wasserstoff oder $C_1$-$C_4$-Alkyl und $R_4$ $C_1$-$C_4$-Alkyl bedeuten und zusätzlich in Gegenwart einer katalytisch wirkenden Säure bei Temperaturen im Bereich von 0° bis 150°C durchgeführt wird.

**14.** Verfahren nach Anspruch 12, dadurch gekennzeichnet, dass die Reaktion von Verbindungen der Formel IIc mit einem Säurechlorid oder Säurebromid der Formel V oder einem Säureanhydrid der Formel VI in einem reaktionsinerten Lösungsmittel bei Temperaturen von -20° bis 100°C durchgeführt wird.

**15.** Verfahren zur Bekämpfung von Schädlingen, dadurch gekennzeichnet, dass man eine pestizid-wirksame Menge mindestens einer Verbindung der Formel I gemäss Anspruch 1 auf oder in die Wirtspflanzen oder sonstigen Lebensräume der Schädlinge mit Ausnahme menschlicher oder tierischer Körper appliziert.

**16.** Verfahren nach Anspruch 15, dadurch gekennzeichnet, dass die zu bekämpfenden Schädlinge Tiere befallende Endo- oder Ektoparasiten sind.

**17.** Verfahren nach Anspruch 15, dadurch gekennzeichnet, dass die zu bekämpfenden Schädlinge pflanzenschädigende Parasiten sind.

**18.** Verbindungen der Formel I gemäss Anspruch 1 zur Verwendung als antiparasitäres Mittel gegen Tiere befallende Endo- und Ektoparasiten.

**19.** Verwendung einer Verbindung der Formel I gemäss Anspruch 1 zur Herstellung eines Arzneimittels zur Bekämpfung von Tiere befallenden Endo- und Ektoparasiten.

**Claims**

**Claims for the following Contracting States : BE, CH, DE, ES, FR, GB, GR, IT, LI, LU, NL, SE**

**1.** A compound of the formula I

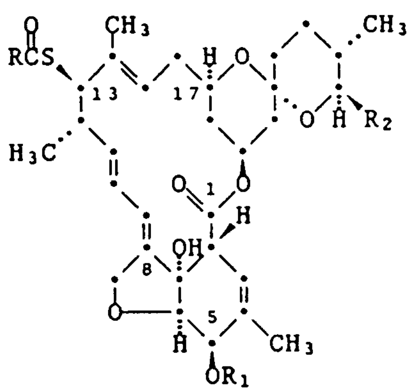

in which $R_1$ is hydrogen or a silyl or acyl group, $R_2$ is methyl, ethyl, isopropyl or sec-butyl and R is hydrogen, a straight-chain or branched $C_1$-$C_{18}$alkyl group which is unsubstituted or substituted by 1 to

7 halogen atoms, 1 to 6 $C_1$-$C_6$alkoxy groups, an unsubstituted or halogenated phenoxy group, a $C_1$-$C_4$alkylthio group, a $C_1$-$C_4$alkylcarbonyl group or an unsubstituted or mono- or polymethyl-substituted $C_3$-$C_6$cycloalkyl group, or is a $C_2$-$C_6$alkenyl group which is unsubstituted or substituted by 1 to 7 halogen atoms or 1 to 6 $C_1$-$C_6$alkoxy groups, or is a $C_2$-$C_6$alkynyl group which is unsubstituted or substituted by 1 to 7 halogen atoms or 1 to 6 $C_1$-$C_6$alkoxy groups, or is a mono- to tetracyclic cycloaliphatic group having 3 to 10 carbon atoms, or a $C_3$-$C_{10}$cycloalkyl group which is substituted by 1 to 7 halogen atoms, 1 to 6 $C_1$-$C_6$alkoxy groups or by $C_1$-$C_4$alkyl groups, or is 2,2-dimethyl-3-(2,2-dichlorovinyl)cyclopropyl, a phenyl group which is unsubstituted or substituted by 1 to 3 substituents from the group comprising halogen atoms, $C_1$-$C_6$alkyl, $C_1$-$C_6$alkoxy, $C_1$-$C_4$alkylthio, phenyl, nitro, halo-$C_1$-$C_4$alkyl and amino or by a difluoromethylenedioxy group whose oxygen atoms are located on two adjacent carbon atoms, or is a benzyl group which is unsubstituted or substituted by 1 to 3 substituents from the group comprising halogen atoms, $C_1$-$C_6$alkyl, $C_1$-$C_6$alkoxy, $C_1$-$C_4$alkylthio, phenyl, nitro, halo-$C_1$-$C_4$alkyl and amino, where the two last-mentioned substituents can only be present on ring carbon atoms.

**2.** A compound of the formula I, according to claim 1, in which alkyl, cycloalkyl, alkenyl and alkynyl groups represented by R are substituted by 1 to 7 halogen atoms or 1 to 6 $C_1$-$C_6$alkoxy groups, and the phenyl groups are substituted by 1 to 3 substituents selected from the group consisting of halogen atoms, $C_1$-$C_6$alkyl, $C_1$-$C_6$alkoxy, $C_1$-$C_4$alkylthio, phenyl or nitro, and $R_1$ and $R_2$ are as defined under formula I in claim 1.

**3.** A compound of the formula I, according to claim 1, in which $R_1$ is hydrogen, $R_2$ is methyl, ethyl, isopropyl or sec-butyl and R is hydrogen or $C_1$-$C_6$alkyl, $C_2$-$C_4$alkenyl, $C_2$-$C_4$alkynyl or $C_3$-$C_6$cycloalkyl, each of which is unsubstituted or substituted by 1 to 4 halogen atoms or by $C_1$-$C_4$alkoxy; or R is phenyl which is unsubstituted or monosubstituted to trisubstituted by halogen, $C_1$-$C_4$alkyl, $C_1$-$C_4$alkoxy, $C_1$-$C_4$alkylthio or nitro.

**4.** A compound of the formula I, according to claim 1, in which $R_1$ is hydrogen, $R_2$ is methyl or ethyl and R is $C_1$-$C_7$alkyl which is unsubstituted or monosubstituted by halogen or $C_1$-$C_4$alkoxy, phenyl which is monosubstituted by trifluoromethyl or the group $-O-CF_2-O-$ in which the two oxygen atoms are located on two adjacent ring carbon atoms, or $\alpha$-methylbenzyl or $\alpha,\alpha$-dimethylbenzyl.

**5.** A compound of the formula I, according to claim 1, in which $R_1$ is hydrogen, $R_2$ is methyl or ethyl and R is $C_1$-$C_7$alkyl, $C_1$-$C_4$alkyl which is monosubstituted by chlorine, fluorine or methoxy, phenyl which is monosubstituted by trifluoromethyl or the group $-O-CF_2-O-$ in which the two oxygen atoms are located on two adjacent ring carbon atoms, or $\alpha$-methylbenzyl or $\alpha,\alpha$-dimethylbenzyl.

**6.** A compound of the formula I, according to claim 1, in which R is tert-butyl and $R_1$ and $R_2$ are as defined under formula I in claim 1.

**7.** A compound of the formula I, according to claim 6, in which $R_1$ is hydrogen, $R_2$ is methyl, ethyl or isopropyl and R is tert-butyl.

**8.** A compound of the formula I, according to claim 1, in which $R_1$ is tert-butyldimethylsilyl.

**9.** A compound according to claim 1, selected from the group comprising:
13$\beta$-Formylthiomilbemycin D
13$\beta$-Acetylthiomilbemycin D
13$\beta$-Pivaloylthiomilbemycin D
13$\beta$-Formylthiomilbemycin $A_3$
13$\beta$-Acetylthiomilbemycin $A_3$
13$\beta$-Pivaloylthiomilbemycin $A_3$
13$\beta$-Formylthiomilbemycin $A_4$
13$\beta$-Acetylthiomilbemycin $A_4$
13$\beta$-Pivaloylthiomilbemycin $A_4$
13$\beta$-(2'-Methoxy-2'-methylpropionylthio)-milbemycin D
13$\beta$-(2'Methoxy-2'-methylpropionylthio)-milbemycin $A_4$
13$\beta$-Trichloroacetylthiomilbemycin $A_4$

13β-(4'-Chlorobutanoylthio)-milbemycin $A_4$
13β-Trichloroacryloylthiomilbemycin $A_4$
13β-Cyclopropanecarbonylthiomilbemycin $A_4$
13β-Cyclobutanecarbonylthiomilbemycin $A_4$
13β-Heptanoylthiomilbemycin $A_4$
13β-(3'-Chloro-2',2'-dimethylpropionylthio)-milbemycin $A_4$
13β-(3'-Chloro-2',2'-dimethylpropionylthio)-milbemycin $A_3$
13β-(1'-Methylcyclopropanecarbonylthio)-milbemycin $A_4$
13β-(1'-Methylcyclopropanecarbonylthio)-milbemycin $A_3$
13β-(1-Adamantanecarbonylthio)-milbemycin $A_4$
13β-(p-Fluorophenoxyacetylthio)-milbemycin $A_4$
13β-(2'-Chloro-2'-methylpropionylthio)-milbemycin $A_4$
13β-(2',2'-Dichloropropionylthio)-milbemycin $A_4$
13β-(2',2'-Dimethylbutanoylthio)-milbemycin $A_4$
13β-(3',3'-Dimethylbutanoylthio)-milbemycin $A_4$
13β-(2',2',3',3'-Tetramethylbutanoylthio)-milbemycin $A_4$
13β-(p-Chlorobenzoylthio)-milbemycin $A_4$
13β-(3',3',3'-Trifluoropropionylthio)-milbemycin $A_4$
13β-Chloroacetylthiomilbemycin $A_4$
13β-(2'-Chloro-3',3',3'-trifluoropropionylthio)-milbemycin $A_4$
13β-(3',3',3'-Trifluoropropionylthio)-milbemycin $A_4$
13β-[o-(Trifluoromethyl)-benzoylthio]-milbemycin $A_4$
13β-(α,α-Dimethylbenzylcarbonylthio)-milbemycin $A_4$
13β-(2-n-Propyl-n-valeroylthio)-milbemycin $A_4$
13β-[2,3-Difluoromethylenedioxy)-benzoylthio]-milbemycin $A_4$
13β-(α-Methylbenzylcarbonylthio)-milbemycin $A_4$ and
13β-(Methoxyacetylthio)-milbemycin $A_4$.

**10.** A compound according to claim 1, selected from the group comprising:
5-O-tert-Butyldimethylsilyl-13β-formylthiomilbemycin D
5-O-tert-Butyldimethylsilyl-13β-acetylthiomilbemycin D
5-O-tert-Butyldimethylsilyl-13β-pivaloylthiomilbemycin D
5-O-tert-Butyldimethylsilyl-13β-formylthiomilbemycin $A_3$
5-O-tert-Butyldimethylsilyl-13β-acetylthiomilbemycin $A_3$
5-O-tert-Butyldimethylsilyl-13β-pivaloylthiomilbemycin $A_3$
5-O-tert-Butyldimethylsilyl-13β-formylthiomilbemycin $A_4$
5-O-tert-Butyldimethylsilyl-13β-alacetylthiomilbemycin $A_4$
5-O-tert-Butyldimethylsilyl-13β-pivaloylthiomilbemycin $A_4$
5-O-tert-Butyldimethylsilyl-13β-(2'-methoxy-2'-methylpropionylthio)-milbemycin D
5-O-tert-Butyldimethylsilyl-13β-(2'-methoxy-2'-methylpropionylthio)-milbemycin $A_4$
5-O-tert-Butyldimethylsilyl-13β-trichloroacetylthiomilbemycin $A_4$
5-O-tert-Butyldimethylsilyl-13β-(4'-chlorobutanoylthio)-milbemycin $A_4$
5-O-tert-Butyldimethylsilyl-13β-trichloroacryloylthiomilbemycin $A_4$
5-O-tert-Butyldimethylsilyl-13β-cyclopropanecarbonylthiomilbemycin $A_4$
5-O-tert-Butyldimethylsilyl-13β-cyclobutanecarbonylthiomilbemycin $A_4$
5-O-tert-Butyldimethylsilyl-13β-heptanoylthiomilbemycin $A_4$
5-O-tert-Butyldimethylsilyl-13β-(3'-chloro-2',2'-dimethylpropionylthio) milbemycin $A_4$
5-O-tert-Butyldimethylsilyl-13β-(3'-chloro-2',2'-dimethylpropionylthio)-milbemycin $A_3$
5-O-tert-Butyldimethylsilyl-13β-(1'-methylcyclopropanecarbonylthio)-milbemycin $A_4$
5-O-tert-Butyldimethylsilyl-13β-(1'-methylcyclopropanecarbonylthio). milbemycin $A_3$
5-O-tert-Butyldimethylsilyl-13β-(1-adamantanecarbonylthio)-milbemycin $A_4$
5-O-tert-Butyldimethylsilyl-13β-(p-fluorophenoxyacetylthio)-milbemycin $A_4$
5-O-tert-Butyldimethylsilyl-13β-(2'-chloro-2'-methylpropionylthio)-milbemycin $A_4$
5-O-tert-Butyldimethylsilyl-13β-(2',2'-dichloropropionylthio)-milbemycin $A_4$
5-O-tert-Butyldimethylsilyl-13β-(2',2'-dimethylbutanoylthio)-milbemycin $A_4$
5-O-tert-Butyldimethylsilyl-13β-(3',3'-dimethylbutanoylthio)-milbemycin $A_4$
5-O-tert-Butyldimethylsilyl-13β-(2',2',3',3'-tetramethylbutanoylthio)-milbemycin $A_4$
5-O-tert-Butyldimethylsilyl-13β-(p-chlorobenzoylthio)-milbemycin $A_4$

5-O-tert-Butyldimethylsilyl-13β-(3',3',3',-trifluoropropionylthio)-milbemycin $A_4$
5-O-tert-Butyldimethylsilyl-13β-chloroacetylthiomilbemycin $A_4$
5-O-tert-Butyldimethylsilyl-13β-(2'-chloro-3',3',3',-trifluoropropionylthio)-milbemycin $A_4$
5-O-tert-Butyldimethylsilyl-13β-(3',3',3'-trifluoropropionylthio)-milbemycin $A_4$
5-O-tert-Butyldimethylsilyl-13β-(α-methylbenzylcarbonylthio)-milbemycin $A_4$
5-O-tert-Butyldimethylsilyl-13β-(methoxyacetylthio)-milbemycin $A_4$
5-O-tert-Butyldimethylsilyl-13β-[o-(trifluoromethyl)-benzoylthio]-milbemycin $A_4$
5-O-tert-Butyldimethylsilyl-13β-(α,α-dimethylbenzylcarbonylthio)-milbemycin $A_4$
5-O-tert-Butyldimethylsilyl-13β-(2-n-propyl-n-valeroylthio)-milbemycin $A_4$
5-O-tert-Butyldimethylsilyl-13β-[2,3-difluoromethylenedioxy)-benzoylthio]-milbemycin $A_4$.

**11.** A process for the preparation of compounds of the formula I

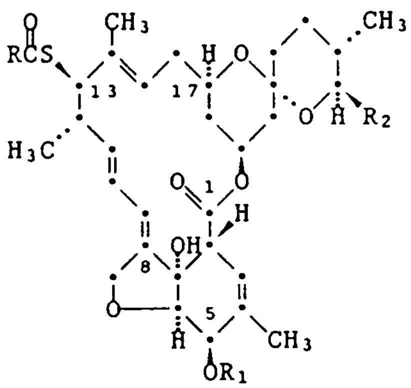

(I)

in which $R_1$ is hydrogen or a silyl or acyl group, $R_2$ is methyl, ethyl, isopropyl or sec-butyl and R is hydrogen, a straight-chain or branched $C_1$-$C_{18}$alkyl group which is unsubstituted or substituted by 1 to 7 halogen atoms, 1 to 6 $C_1$-$C_6$alkoxy groups, an unsubstituted or halogenated phenoxy group, a $C_1$-$C_4$alkylthio group, a $C_1$-$C_4$alkylcarbonyl group or an unsubstituted or mono- or polymethyl-substituted $C_3$-$C_6$cycloalkyl group, or is a $C_2$-$C_6$alkenyl group which is unsubstituted or substituted by 1 to 7 halogen atoms or 1 to 6 $C_1$-$C_6$alkoxy groups, or is a $C_2$-$C_6$alkynyl group which is unsubstituted or substituted by 1 to 7 halogen atoms or 1 to 6 $C_1$-$C_6$alkoxy groups, or is a mono- to tetracyclic cycloaliphatic group having 3 to 10 carbon atoms, or a $C_3$-$C_{10}$cycloalkyl group which is substituted by 1 to 7 halogen atoms, 1 to 6 $C_1$-$C_6$alkoxy groups or by $C_1$-$C_4$alkyl groups, or is 2,2-dimethyl-3-(2,2-dichlorovinyl)cyclopropyl, a phenyl group which is unsubstituted or substituted by 1 to 3 substituents from the group comprising halogen atoms, $C_1$-$C_6$alkyl, $C_1$-$C_6$alkoxy, $C_1$-$C_4$alkylthio, phenyl, nitro, halo-$C_1$-$C_4$alkyl and amino or by a difluoromethylenedioxy group whose oxygen atoms are located on two adjacent carbon atoms, or is a benzyl group which is unsubstituted or substituted by 1 to 3 substituents from the group comprising halogen atoms, $C_1$-$C_6$alkyl, $C_1$-$C_6$alkoxy, $C_1$-$C_4$alkylthio, phenyl, nitro, halo-$C_1$-$C_4$alkyl and amino, where the two last-mentioned substituents can only be present on ring carbon atoms, wherein compounds of the formula II

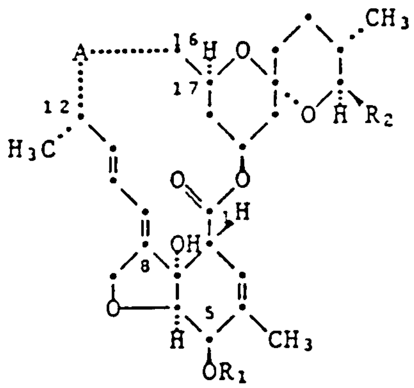

(II)

in which A is a group a, b or c

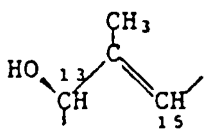 ,

(a)

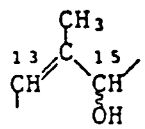 or

(b)

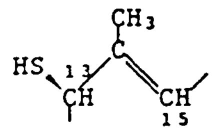 ,

(c)

$R_1$ is a protective group and $R_2$ is as defined under formula I, is treated with a reagent suitable for introducing or building up a 13β-thiol ester group, after which, if free 5-hydroxy compounds are desired, the $R_1$-protective group is detached.

**12.** A process according to claim 11, wherein the reagent employed to introduce or to build up a 13β-thiol ester group in compounds of the formula II is:

i) in the case of compounds of the formulae IIa or IIb:

a) a thiocarboxylic acid of the formula III

RCOSH (III)

or

b) a thioamide of the formula IV

$RCSN(alkyl)_2$ (IV)

in which the alkyl radicals contain 1 to 4 carbon atoms and are preferably methyl, or

ii) in the case of compounds of the formula IIc:

c) an acid halide of the formula V

RCOhal (V)

in which hal is halogen, preferably chlorine or bromine, or

d) an acid anhydride of the formula VI

$(RCO)_2O$ (VI)

in which R is as defined under formula I.

**13.** A process according to claim 12, wherein the reaction of compounds of the formula 11a or IIb with a thioacid of the formula III or a thioamide of the formula IV is carried out at temperatures within the range from 0° to 150°C, in the presence of an orthoester of the formula VII

$R_3C(OR_4)_3$ (VII)

in which $R_3$ is hydrogen or $C_1$-$C_4$alkyl and $R_4$ is $C_1$-$C_4$alkyl, and, additionally, in the presence of a catalytically active acid.

**14.** A process according to claim 12, wherein the reaction of compounds of the formula IIc with an acid chloride or acid bromide of the formula V or an acid anhydride of the formula VI is carried out in a solvent inert towards the reaction at temperatures from -20° to 100°C.

**15.** An agent for the control of pests, which, in addition to carriers, distribution agents or agents for carrying and distribution, contains at least one compound of the formula I according to claim 1 as the active substance.

**16.** A process for the control of pests, wherein a pesticidally effective amount of at least one compound of the formula I according to claim 1 is applied onto or into the host plants or other habitats of the pests with the exception of human or animal bodies.

**17.** A process according to claim 16, wherein the pests to be combated are endoparasites or ectoparasites which infest animals.

**18.** A process according to claim 16, wherein the pests to be combated are parasites which damage plants.

**19.** A compound of the formula I according to claim 1 for use as an antiparasitic agent against endoparasites and ectoparasites which infest animals.

**20.** The use of a compound of the formula I according to claim 1 for the production of a pharmaceutical for combating endoparasites and ectoparasites which infest animals.

**Claims for the following Contracting State : AT**

**1.** An agent for the control of pests, which, in addition to carriers, distribution agents or agents for carrying and distribution, contains at least one compound of the formula I

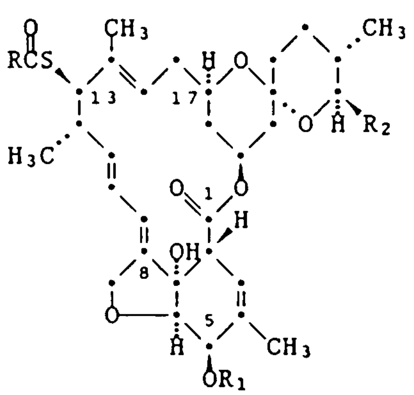

(I)

in which $R_1$ is hydrogen or a silyl or acyl group, $R_2$ is methyl, ethyl, isopropyl or sec-butyl and R is hydrogen, a straight-chain or branched $C_1$-$C_{18}$alkyl group which is unsubstituted or substituted by 1 to 7 halogen atoms, 1 to 6 $C_1$-$C_6$alkoxy groups, an unsubstituted or halogenated phenoxy group, a $C_1$-$C_4$alkylthio group, a $C_1$-$C_4$alkylcarbonyl group or an unsubstituted or mono- or polymethyl-substituted $C_3$-$C_6$cycloalkyl group, or is a $C_2$-$C_6$alkenyl group which is unsubstituted or substituted by 1 to 7 halogen atoms or 1 to 6 $C_1$-$C_6$alkoxy groups, or is a $C_2$-$C_6$alkynyl group which is unsubstituted or substituted by 1 to 7 halogen atoms or 1 to 6 $C_1$-$C_6$alkoxy groups, or is a mono- to tetracyclic cycloaliphatic group having 3 to 10 carbon atoms, or a $C_3$-$C_{10}$cycloalkyl group which is substituted by

1 to 7 halogen atoms, 1 to 6 $C_1$-$C_6$alkoxy groups or by $C_1$-$C_4$alkyl groups, or is 2,2-dimethyl-3-(2,2-dichlorovinyl)cyclopropyl, a phenyl group which is unsubstituted or substituted by 1 to 3 substituents from the group comprising halogen atoms, $C_1$-$C_6$alkyl, $C_1$-$C_6$alkoxy, $C_1$-$C_4$alkylthio, phenyl, nitro, halo-$C_1$-$C_4$alkyl and amino or by a difluoromethylenedioxy group whose oxygen atoms are located on two adjacent carbon atoms, or is a benzyl group which is unsubstituted or substituted by 1 to 3 substituents from the group comprising halogen atoms, $C_1$-$C_6$alkyl, $C_1$-$C_6$alkoxy, $C_1$-$C_4$alkylthio, phenyl, nitro, halo-$C_1$-$C_4$alkyl and amino, where the two last-mentioned substituents can only be present on ring carbon atoms.

**2.** An agent according to claim 1, which contains a compound of the formula I in which alkyl, cycloalkyl, alkenyl and alkynyl groups represented by R are substituted by 1 to 7 halogen atoms or 1 to 6 $C_1$-$C_6$alkoxy groups, and the phenyl groups are substituted by 1 to 3 substituents selected from the group consisting of halogen atoms, $C_1$-$C_6$alkyl, $C_1$-$C_6$alkoxy, $C_1$-$C_4$alkylthio, phenyl or nitro, and $R_1$ and $R_2$ are as defined under formula I in claim 1.

**3.** An agent according to claim 1, which contains a compound of the formula I in which $R_1$ is hydrogen, $R_2$ is methyl, ethyl, isopropyl or secbutyl and R is hydrogen or $C_1$-$C_6$alkyl, $C_2$-$C_4$alkenyl, $C_2$-$C_4$alkynyl or $C_3$-$C_6$cycloalkyl, each of which is unsubstituted or substituted by 1 to 4 halogen atoms or by $C_1$-$C_4$alkoxy; or R is phenyl which is unsubstituted or monosubstituted to trisubstituted by halogen, $C_1$-$C_4$alkyl, $C_1$-$C_4$alkoxy, $C_1$-$C_4$alkylthio or nitro.

**4.** An agent according to claim 1, which contains a compound of the formula I in which $R_1$ is hydrogen, $R_2$ is methyl or ethyl and R is $C_1$-$C_7$alkyl which is unsubstituted or monosubstituted by halogen or $C_1$-$C_4$alkoxy, phenyl which is monosubstituted by trifluoromethyl or the group -O-$CF_2$-O- in which the two oxygen atoms are located on two adjacent ring carbon atoms, or $\alpha$-methylbenzyl or $\alpha,\alpha$-dimethylbenzyl.

**5.** An agent according to claim 1, which contains a compound of the formula I in which $R_1$ is hydrogen, $R_2$ is methyl or ethyl and R is $C_1$-$C_7$alkyl, $C_1$-$C_4$alkyl which is monosubstituted by chlorine, fluorine or methoxy, phenyl which is monosubstituted by trifluoromethyl or the group -O-$CF_2$-O- in which the two oxygen atoms are located on two adjacent ring carbon atoms, or $\alpha$-methylbenzyl or $\alpha,\alpha$-dimethylbenzyl.

**6.** An agent according to claim 1, which contains a compound of the formula I in which $R_1$ is tert-butyl and $R_1$ and $R_2$ are as defined under formula I in claim 1.

**7.** An agent according to claim 1, which contains a compound of the formula I in which $R_1$ is hydrogen, $R_2$ is methyl, ethyl or isopropyl and R is tert-butyl.

**8.** An agent according to claim 1, which contains a compound of the formula I in which $R_1$ is tert-butyldimethylsilyl.

**9.** An agent according to claim 1, which contains a compound of the formula I selected from the group comprising:
13$\beta$-Formylthiomilbemycin D
13$\beta$-Acetylthiomilbemycin D
13$\beta$-Pivaloylthiomilbemycin D
13$\beta$-Formylthiomilbemycin $A_3$
13$\beta$-Acetylthiomilbemycin $A_3$
13$\beta$-Pivaloylthiomilbemycin $A_3$
13$\beta$-Formylthiomilbemycin $A_4$
13$\beta$-Acetylthiomilbemycin $A_4$
13$\beta$-Pivaloylthiomilbemycin $A_4$
13$\beta$-(2'-Methoxy-2'-methylpropionylthio)-milbemycin D
13$\beta$-(2'-Methoxy-2'-methylpropionylthio)-milbemycin $A_4$
13$\beta$-Trichloroacetylthiomilbemycin $A_4$
13$\beta$-(4'-Chlorobutanoylthio)-milbemycin $A_4$
13$\beta$-Trichloroacryloylthiomilbemycin $A_4$
13$\beta$-Cyclopropanecarbonylthiomilbemycin $A_4$
13$\beta$-Cyclobutanecarbonylthiomilbemycin $A_4$

13β-Heptanoylthiomilbemycin $A_4$
13β-(3'-Chloro-2',2'-dimethylpropionylthio)-milbemycin $A_4$
13β-(3'Chloro-2',2'-dimethylpropionylthio)-milbemycin $A_3$
13β-(1'-Methylcyclopropanecarbonylthio)-milbemycin $A_4$
13β-(1'-Methylcyclopropanecarbonylthio)-milbemycin $A_3$
13β-(1-Adamantanecarbonylthio)-milbemycin $A_4$
13β-(p-Fluorophenoxyacetylthio)-milbemycin $A_4$
13β-(2'-Chloro-2'-methylpropionylthio)-milbemycin $A_4$
13β-(2',2'-Dichloropropionylthio)-milbemycin $A_4$
13β-(2',2'-Dimethylbutanoylthio)-milbemycin $A_4$
13β-(3',3'-Dimethylbutanoylthio)-milbemycin $A_4$
13β-(2',2',3',3'-Tetramethylbutanoylthio)-milbemycin $A_4$
13β-(p-Chlorobenzoylthio)-milbemycin $A_4$
13β-(3',3',3'-Trifluoropropionylthio)-milbemycin $A_4$
13β-Chloroacetylthiomilbemycin $A_4$
13β-(2'-Chloro-3',3',3'-trifluoropropionylthio)-milbemycin $A_4$
13β-(3',3',3'-Trifluoropropionylthio)-milbemycin $A_4$
13β-[o-(Trifluoromethyl)-benzoylthio]-milbemycin $A_4$
13β-(α,α-Dimethylbenzylcarbonylthio)-milbemycin $A_4$
13β-(2-n-Propyl-n-valeroylthio)-milbemycin $A_4$
13β-[(2,3,-Difluoromethylenedioxy)-benzoylthio]-milbemycin $A_4$
13β-(α-Methylbenzylcarbonylthio)-milbemycin $A_4$ and
13β-(Methoxyacetylthio)-milbemycin $A_4$.

**10.** An agent according to claim 1, which contains a compound of the formula I selected from the group comprising:
5-O-tert-Butyldimethylsilyl-13β-formylthiomilbemycin D
5-O-tert-Butyldimethylsilyl-13β-acetylthiomilbemycin D
5-O-tert-Butyldimethylsilyl-13β-pivaloylthiomilbemycin D
5-O-tert-Butyldimethylsilyl-13β-formylthiomilbemycin $A_3$
5-O-tert-Butyldimethylsilyl-13β-acetylthiomilbemycin $A_3$
5-O-tert-Butyldimethylsilyl-13β-pivaloylthiomilbemycin $A_3$
5-O-tert-Butyldimethylsilyl-13β-formylthiomilbemycin $A_4$
5-O-tert-Butyldimethylsilyl-13β-acetylthiomilbemycin $A_4$
5-O-tert-Butyldimethylsilyl-13β-pivaloylthiomilbemycin $A_4$
5-O-tert-Butyldimethylsilyl-13β-(2'-methoxy-2'-methylpropionylthio)-milbemycin D
5-O-tert-Butyldimethylsilyl-13β-(2'-methoxy-2'-methylpropionylthio)-milbemycin $A_4$
5-O-tert-Butyldimethylsilyl-13β-trichloroacetylthiomilbemycin $A_4$
5-O-tert-Butyldimethylsilyl-13β-(4'-chlorobutanoylthio)-milbemycin $A_4$
5-O-tert-Butyldimethylsilyl-13β-trichloroacryloylthiomilbemycin $A_4$
5-O-tert-Butyldimethylsilyl-13β-cyclopropanecarbonylthiomilbemycin $A_4$
5-O-tert-Butyldimethylsilyl-13β-cyclobutanecarbonylthiomilbemycin $A_4$
5-O-tert-Butyldimethylsilyl-13β-heptanoylthiomilbemycin $A_4$
5-O-tert-Butyldimethylsilyl-13β-(3'-chloro-2',2'-dimethylpropionylthio)-milbemycin $A_4$
5-O-tert-Butyldimethylsilyl-13β-(3'-chloro-2',2'-dimethylpropionylthio)-milbemycin $A_3$
5-O-tert-Butyldimethylsilyl-13β-(1'-methylcyclopropanecarbonylthio)-milbemycin $A_4$
5-O-tert-Butyldimethylsilyl-13β-(1'-methylcyclopropanecarbonylthio)-milbemycin $A_3$
5-O-tert-Butyldimethylsilyl-13β-(1-adamantanecarbonylthio)-milbemycin $A_4$
5-O-tert-Butyldimethylsilyl-13β-(p-fluorophenoxyacetylthio)-milbemycin $A_4$
5-O-tert-Butyldimethylsilyl-13β-(2'-chloro-2'-methylpropionylthio)-milbemycin $A_4$
5-O-tert-Butyldimethylsilyl-13β-(2',2'-dichloropropionylthio)-milbemycin $A_4$
5-O-tert-Butyldimethylsilyl-13β-(2',2'-dimethylbutanoylthio)-milbemycin $A_4$
5-O-tert-Butyldimethylsilyl-13β-(3',3'-dimethylbutanoylthio)-milbemycin $A_4$
5-O-tert-Butyldimethylsilyl-13β-(2'2',3',3'-tetramethylbutanoylthio)-milbemycin $A_4$
5-O-tert-Butyldimethylsilyl-13β-(p-chlorobenzoylthio)-milbemycin $A_4$
5-O-tert-Butyldimethylsilyl-13β-(3',3',3'-trifluoropropionylthio)-milbemycin $A_4$
5-O-tert-Butyldimethylsilyl-13β-chloroacetylthiomilbemycin $A_4$
5-O-tert-Butyldimethylsilyl-13β-(2'-chloro-3',3',3'-trifluoropropionylthio)-milbemycin $A_4$

5-O-tert-Butyldimethylsilyl-13β-(3',3',3'-trifluoropropionylthio)-milbemycin $A_4$
5-O-tert-Butyldimethylsilyl-13β-(α-methylbenzylcarbonylthio)-milbemycin $A_4$
5-O-tert-Butyldimethylsilyl-13β-(methoxyacetylthio)milbemycin $A_4$
5-O-tert-Butyldimethylsilyl-13β-[o-(trifluoromethyl)-benzoylthio]-milbemycin $A_4$
5-O-tert-Butyldimethylsilyl-13β-(α,α-dimethylbenzylcarbonylthio)-milbemycin $A_4$
5-O-tert-Butyldimethylsilyl-13β-(2-n-propyl-n-valeroylthio)-milbemycin $A_4$
and
5-O-tert-Butyldimethylsilyl-13β-[(2,3-difluoromethylenedioxy)-benzoylthio]-milbemycin $A_4$.

**11.** A process for the preparation of compounds of the formula I

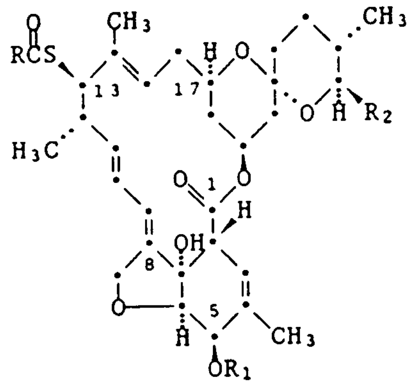

(I)

in which $R_1$ is hydrogen or a silyl or acyl group, $R_2$ is methyl, ethyl, isopropyl or sec-butyl and R is hydrogen, a straight-chain or branched $C_1$-$C_{18}$alkyl group which is unsubstituted or substituted by 1 to 7 halogen atoms, 1 to 6 $C_1$-$C_6$alkoxy groups, an unsubstituted or halogenated phenoxy group, a $C_1$-$C_4$alkylthio group, a $C_1$-$C_4$alkylcarbonyl group or an unsubstituted or mono- or polymethyl-substituted $C_3$-$C_6$cycloalkyl group, or is a $C_2$-$C_6$alkenyl group which is unsubstituted or substituted by 1 to 7 halogen atoms or 1 to 6 $C_1$-$C_6$alkoxy groups, or is a $C_2$-$C_6$alkynyl group which is unsubstituted or substituted by 1 to 7 halogen atoms or 1 to 6 $C_1$-$C_6$alkoxy groups, or is a mono- to tetracyclic cycloaliphatic group having 3 to 10 carbon atoms, or a $C_3$-$C_{10}$cycloalkyl group which is substituted by 1 to 7 halogen atoms, 1 to 6 $C_1$-$C_6$alkoxy groups or by $C_1$-$C_4$alkyl groups, or is 2,2-dimethyl-3-(2,2-dichlorovinyl)cyclopropyl, a phenyl group which is unsubstituted or substituted by 1 to 3 substituents from the group comprising halogen atoms, $C_1$-$C_6$alkyl, $C_1$-$C_6$alkoxy, $C_1$-$C_4$alkylthio, phenyl, nitro, halo-$C_1$-$C_4$alkyl and amino or by a difluoromethylenedioxy group whose oxygen atoms are located on two adjacent carbon atoms, or is a benzyl group which is unsubstituted or substituted by 1 to 3 substituents from the group comprising halogen atoms, $C_1$-$C_6$alkyl, $C_1$-$C_6$alkoxy, $C_1$-$C_4$alkylthio, phenyl, nitro, halo-$C_1$-$C_4$alkyl and amino, where the two last-mentioned substituents can only be present on ring carbon atoms, wherein compounds of the formula II

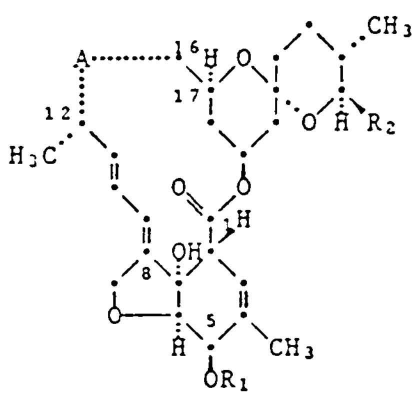

(II)

in which A is a group a, b or c

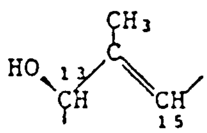 ,

(a)

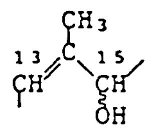 or

(b)

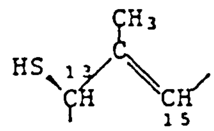 ,

(c)

$R_1$ is a protective group and $R_2$ is as defined under formula I, is treated with a reagent suitable for introducing or building up a 13$\beta$-thiol ester group, after which, if free 5-hydroxy compounds are desired, the $R_1$-protective group is detached.

**12.** A process according to claim 11, wherein the reagent employed to introduce or to build up a 13$\beta$-thiol ester group in compounds of the formula II is:

i) in the case of compounds of the formulae IIa or IIb:

a) a thiocarboxylic acid of the formula III

RCOSH (III)

or

b) a thioamide of the formula IV

$RCSN(alkyl)_2$ (IV)

in which the alkyl radicals contain 1 to 4 carbon atoms and are preferably methyl, or

ii) in the case of compounds of the formula IIc:

c) an acid halide of the formula V

RCOhal (V)

in which hal is halogen, preferably chlorine or bromine, or

d) an acid anhydride of the formula VI

$(RCO)_2O$ (VI)

in which R is as defined under formula I.

**13.** A process according to claim 12, wherein the reaction of compounds of the formula IIa or IIb with a thioacid of the formula III or a thioamide of the formula IV is carried out at temperatures within the range from 0° to 150°C, in the presence of an orthoester of the formula VII

$R_3C(OR_4)_3$ (VII)

in which $R_3$ is hydrogen or $C_1$-$C_4$alkyl and $R_4$ is $C_1$-$C_4$alkyl, and, additionally, in the presence of a catalytically active acid.

**14.** A process according to claim 12, wherein the reaction of compounds of the formula IIc with an acid chloride or acid bromide of the formula V or an acid anhydride of the formula VI is carried out in a solvent inert towards the reaction at temperatures from -20° to 100°C.

**15.** A process for the control of pests, wherein a pesticidally effective amount of at least one compound of the formula I according to claim 1 is applied onto or into the host plants or other habitats of the pests with the exception of human or animal bodies.

**16.** A process according to claim 15, wherein the pests to be combated are endoparasites or ectoparasites which infest animals.

**17.** A process according to claim 15, wherein the pests to be combated are parasites which damage plants.

**18.** A compound of the formula I according to claim 1 for use as an antiparasitic agent against endoparasites and ectoparasites which infest animals.

**19.** The use of a compound of the formula I according to claim 1 for the production of a pharmaceutical for combating endoparasites and ectoparasites which infest animals.

**Revendications**

**Revendications pour les Etats contractants suivants : BE, CH, DE, ES, FR, GB, GR, IT, LI, LU, NL, SE**

**1.** Composés de formule I

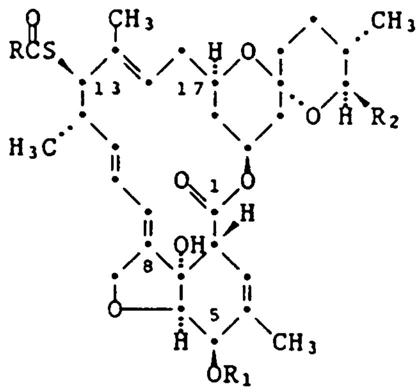

(I)

dans laquelle

$R_1$ représente l'hydrogène, un groupe silyle ou acyle,

$R_2$ représente un groupe méthyle, éthyle, isopropyle ou sec-butyle, et

R représente l'hydrogène,

un groupe alkyle à chaîne droite ou ramifiée en C1-C18, non substitué ou substitué par 1 à 7 atomes d'halogènes, 1 à 6 groupes alcoxy en C1-C6, un groupe phénoxy no substitué ou halogéné, un groupe alkylthio en C1-C4, un groupe (alkyle en C1-C4)-carbonyle ou un groupe cycloalkyle en C3-C6 non substitué ou mono- ou poly-substitué par des groupes méthyle,

un groupe alcényle en C2-C6 non substitué ou substitué par 1 à 7 atomes d'halogènes ou un à six groupes alcoxy en C1-C6,

un groupe alcynyle en C2-C6 non substitué ou substitué par 1 à 7 atomes d'halogènes ou un à six groupes alcoxy en C1-C6,

un groupe cycloaliphatique mono- à tétra-cyclique en C3-C10 ou un groupe cycloalkyle en C3-C10 qui est substitué par 1 à 7 atomes d'halogènes, un à six groupes alcoxy en C1-C6 ou par des groupes alkyle en C1-C4, ou un groupe 2,2-diméthyl-3-(2,2-dichlorovinyl)-cyclopropyle,

un groupe phényle non substitué ou portant un à trois substituants choisis parmi les atomes d'halogènes, les groupes alkyle en C1-C6, alcoxy en C1-C6, alkylthio en C1-C4, phényle, nitro, halogénoalkyle en C1-C4 et amino ou par un groupe difluorométhylène-dioxy dont les atomes d'oxygène sont situés sur deux atomes de carbone voisins, ou bien un groupe benzyle non substitué ou portant un à trois substituants choisis parmi les atomes d'halogènes, les groupes alkyle en C1-C6, alcoxy en C1-C6, alkylthio en C1-C4, phényle, nitro, halogénoalkyle en C1-C4 et amino, ces deux derniers substituants ne pouvant se trouver que sur des atomes de carbone cycliques.

**2.** Composés de formule I de la revendication 1, dans lesquels les groupes alkyle, cycloalkyle, alcényle et alcynyle mentionnés en référence à R sont substitués par 1 à 7 atomes d'halogènes ou un à six groupes alcoxy en C1-C6, et les groupes phényle portent un à trois substituants choisis parmi les atomes d'halogènes, les groupes alkyle en C1-C6, alcoxy en C1-C6, alkylthio en C1-C4, phényle ou nitro, et $R_1$ et $R_2$ ont les significations indiquées en référence à la formule I dans la revendication 1.

**3.** Composés de formule I de la revendication 1, dans lesquels $R_1$ représente l'hydrogène, $R_2$ représente un groupe méthyle, éthyle, isopropyle ou sec-butyle et R représente l'hydrogène, un groupe alkyle en C1-C6, alcényle en C2-C4, alcynyle en C2-C4 ou cycloalkyle en C3-C6 non substitué ou substitué par 1 à 4 atomes d'halogènes ou groupes alcoxy en C1-C4 ; ou un groupe phényle non substitué ou portant un à trois substituants choisis parmi les halogènes, les groupes alkyle en C1-C4, alcoxy en C1-C4, alkylthio en C1-C4 ou nitro.

**4.** Composés de formule I de la revendication 1, dans lesquels $R_1$ représente l'hydrogène, $R_2$ un groupe méthyle ou éthyle et R un groupe alkyle en C1-C7 non substitué ou monosubstitué par un halogène ou un groupe alcoxy en C1-C4, un groupe phényle monosubstitué par un groupe trifluorométhyle ou le groupe $-O-CF_2-O-$ dont les deux atomes d'oxygène se trouvent sur deux atomes de carbone cycliques voisins, ou un groupe alpha-méthyl-ou alpha,alpha-diméthyl-benzyle.

**5.** Composés de formule I de la revendication 1, dans lesquels $R_1$ représente l'hydrogène, $R_2$ un groupe méthyle ou éthyle et R un groupe alkyle en C1-C7, un groupe alkyle en C1-C4 monosubstitué par le chlore, le fluor ou un groupe méthoxy, un groupe phénylé monosubstitué par un groupe trifluorométhyle ou par le groupe $-O-CF_2-O-$ dont les deux atomes d'oxygène sont situés sur deux atomes de carbone cycliques voisins, ou un groupe alpha-méthyl- ou alpha,alpha-diméthyl-benzyle.

**6.** Composés de formule I de la revendication 1, dans lesquels R représente un groupe tert-butyle et $R_1$ et $R_2$ ont les significations indiquées en référence à la formule I dans la revendication 1.

**7.** Composés de formule I de la revendication 6, dans lesquels $R_1$ représente l'hydrogène, $R_2$ un groupe méthyle, éthyle ou isopropyle et R un groupe tert-butyle.

**8.** Composés de formule I de la revendication 1, dans lesquels $R_1$ représente le groupe tert-butyl-diméthyl-silyle.

**9.** Composé de la revendication 1, choisi dans le groupe suivant :
13bêta-formylthio-milbémycine D
13bêta-acétylthio-milbémycine D
13bêta-pivaloylthio-milbémycine D
13bêta-formylthio-milbémycine $A_3$
13bêta-acétylthio-milbémycine $A_3$
13bêta-pivaloylthio-milbémycine $A_3$
13bêta-formylthio-milbémycine $A_4$
13bêta-acétylthio-milbémycine $A_4$
13bêta-pivaloylthio-milbémycine $A_4$
13bêta-(2'-méthoxy-2'-méthylpropionylthio)-milbémycine D
13bêta-(2'-méthoxy-2'-méthylpropionylthio)-milbémycine $A_4$
13bêta-trichloracétylthio-milbémycine $A_4$
13bêta-(4'-chloro-butanoylthio)milbémycine $A_4$
13bêta-trichloracryloylthio-milbémycine $A_4$
13bêta-cyclopropane-carbonylthio-milbémycine $A_4$
13-bêta-cyclobutane-carbonylthio-milbémycine $A_4$
13bêta-heptanoylthio-milbémycine $A_4$
13bêta-(3'-chloro-2',2'-diméthyl-propionylthio)-milbémycine $A_4$
13bêta-(3'-chloro-2',2'-diméthyl-propionylthio)-milbémycine $A_3$
13bêta-(1'-méthyl-cyclopropane-carbonylthio)-milbémycine $A_4$
13bêta-(1'-méthyl-cyclopropane-carbonylthio)-milbémycine $A_3$
13bêta-(1-adamantane-carbonylthio)-milbémycine $A_4$
13bêta-(p-fluorophénoxyacétylthio)-milbémycine $A_4$

13bêta-(2'-chloro-2'-méthyl-propionylthio)-milbémycine $A_4$
13bêta-(2',2'-dichloropropionylthio)-milbémycine $A_4$
13bêta-(2',2'-diméthylbutanoylthio)-milbémycine $A_4$
13bêta-(3',3'-diméthylbutanoylthio)-milbémycine $A_4$
13bêta-(2',2',3',3'-tétraméthylbutanoylthio)-milbémycine $A_4$
13bêta-(p-chlorobenzoylthio)-milbémycine $A_4$
13bêta-(3',3',3'-trifluoropropionylthio)-milbémycine $A_4$
13bêta-chloroacétylthio-milbémycine $A_4$
13bêta-(2'-chloro-3',3',3'-trifluoropropionylthio)-milbémycine $A_4$
13bêta-(3',3',3'-trifluoropropionylthio)-milbémycine $A_4$
13bêta-[o-(trifluorométhyl)-benzoylthio]-milbémycine $A_4$
13bêta-(alpha,alpha-diméthylbenzylcarbonylthio)-milbémycine $A_4$
13bêta-(2-n-propyl-n-valéroylthio)-milbémycine $A_4$
13bêta-[(2,3-difluorométhylènedioxy)-benzoylthio]-milbémycine $A_4$
13bêta-(alpha-méthylbenzylcarbonylthio)-milbémycine $A_4$ et
13bêta-(méthoxyacétylthio)-milbémycine $A_4$.

**10.** Composé de la revendication 1, choisi dans le groupe suivant :
5-O-tert-butyldiméthylsilyl-13bêta-formylthio-milbémycine D
5-O-tert-butyldiméthylsilyl-13bêta-acétylthio-milbémycine D
5-O-tert-butyldiméthylsilyl-13bêta-pivaloylthio-milbémycine D
5-O-tert-butyldiméthylsilyl-13bêta-formylthio-milbémycine $A_3$
5-O-tert-butyldiméthylsilyl-13bêta-acétylthio-milbémycine $A_3$
5-O-tert-butyldiméthylsilyl-13bêta-pivaloylthio-milbémycine $A_3$
5-O-tert-butyldiméthylsilyl-13bêta-formylthio-milbémycine $A_4$
5-O-tert-butyldiméthylsilyl-13bêta-acétylthio-milbémycine $A_4$
5-O-tert-butyldiméthylsilyl-13bêta-pivaloylthio-milbémycine $A_4$
5-O-tert-butyldiméthylsilyl-13bêta-(2'-méthoxy-2'-méthyl-propionylthio)-milbémycine D
5-O-tert-butyldiméthylsilyl-13bêta-(2'-méthoxy-2'-méthylpropionylthio)-milbémycine $A_4$
5-O-tert-butyldiméthylsilyl-13bêta-trichloroacétylthio-milbémycine $A_4$
5-O-tert-butyldiméthylsilyl-13bêta-(4'-chloro-butanoylthio)milbémycine $A_4$
5-O-tert-butyldiméthylsilyl-13bêta-trichloracryloylthio-milbémycine $A_4$
5-O-tert-butyldiméthylsilyl-13bêta-cyclopropane-carbonylthio-milbémycine $A_4$
5-O-tert-butyldiméthylsilyl-13bêta-cyclobutane-carbonylthio-milbémycine $A_4$
5-O-tert-butyldiméthylsilyl-13bêta-heptanoylthio-milbémycine $A_4$
5-O-tert-butyldiméthylsilyl-13bêta-(3'-chloro-2',2'-diméthyl-propionylthio)-milbémycine $A_4$
5-O-tert-butyldiméthylsilyl-13bêta-(3'-chloro-2',2'-diméthyl-propionylthio)-milbémycine $A_3$
5-O-tert-butyldiméthylsilyl-13bêta-(1'-méthyl-cyclopropane-carbonylthio)-milbémycine $A_4$
5-O-tert-butyldiméthylsilyl-13bêta-(1'-méthyl-cyclopropane-carbonylthio)-milbémycine $A_3$
5-O-tert-butyldiméthylsilyl-13bêta-(1-adamantane-carbonylthio)-milbémycine $A_4$
5-O-tert-butyldiméthylsilyl-13bêta-(p-fluorophénoxyacétylthio)-milbémycine $A_4$
5-O-tert-butyldiméthylsilyl-13bêta-(2'-chloro-2'-méthylpropionylthio)-milbémycine $A_4$
5-O-tert-butyldiméthylsilyl-13bêta-(2',2'-dichloropropionylthio)-milbémycine $A_4$
5-O-tert-butyldiméthylsilyl-13bêta-(2',2'-diméthylbutanoylthio)-milbémycine $A_4$
5-O-tert-butyldiméthylsilyl-13bêta-(3,3'-diméthylbutanoylthio)-milbémycine $A_4$
5-O-tert-butyldiméthylsilyl-13bêta-(2',2',3',3'-tétraméthylbutanoylthio)-milbémycine $A_4$
5-O-tert-butyldiméthylsilyl-13bêta-(p-chlorobenzoylthio)-milbémycine$A_4$
5-O-tert-butyldiméthylsilyl-13bêta-(3',3',3'-trifluoropropionylthio)-milbémycine $A_4$
5-O-tert-butyldiméthylsilyl-13bêta-chloracétylthio-milbémycine $A_4$
5-O-tert-butyldiméthylsilyl-13bêta-(2'-chloro-3',3',3'-trifluoro-propionylthio)-milbémycine $A_4$
5-O-tert-butyldiméthylsilyl-13bêta-(3',3',3'-trifluoropropionylthio)-milbémycine $A_4$
5-O-tert-butyldiméthylsilyl-13bêta-(alpha-méthylbenzylcarbonylthio)-milbémycine $A_4$
5-O-tert-butyldiméthylsilyl-13bêta-(méthoxyacétylthio)-milbémycine $A_4$
5-O-tert-butyldiméthylsilyl-13bêta-[o-(trifluorométhyl)-benzoylthio]-milbémycine $A_4$
5-O-tert-butyldiméthylsilyl-13bêta-(alpha,alpha-diméthylbenzylcarbonylthio)-milbémycine $A_4$
5-O-tert-butyldiméthylsilyl-13bêta-(2-n-propyl-n-valéroylthio)-milbémycine $A_4$
5-O-tert-butyldiméthylsilyl-13bêta-[(2,3-difluorométhylène-dioxy)-benzoylthio]-milbémycine $A_4$.

**11.** Procédé de préparation des composés de formule I

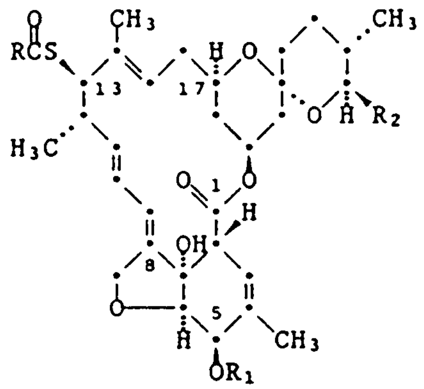

(I)

dans laquelle

$R_1$ représente l'hydrogène, un groupe silyle ou acyle,

$R_2$ représente un groupe méthyle, éthyle, isopropyle ou sec-butyle, et

R représente l'hydrogène,
un groupe alkyle à chaîne droite ou ramifiée en C1-C18, non substitué ou substitué par 1 à 7 atomes d'halogènes, un à six groupes alcoxy en C1-C6, un groupe phénoxy non substitué ou halogéné, un groupe alkylthio en C1-C4, un groupe (alkyle en C1-C4)-carbonyle ou un groupe cycloalkyle en C3-C6 non substitué ou portant un ou plusieurs substituants méthyle,
un groupe alcényle en C2-C6 non substitué ou substitué par 1 à 7 atomes d'halogènes ou un à six groupes alcoxy en C1-C6,
un groupe alcynyle en C2-C6 non substitué ou substitué par 1 à 7 atomes d'halogènes ou un à six groupes alcoxy en C1-C6,
un groupe cycloaliphatique mono- à tétra-cyclique en C3-C10 ou un groupe cycloalkyle en C3-C10 substitué par 1 à 7 atomes d'halogènes, un à six groupes alcoxy en C1-C6 ou par des groupes alkyle en C1-C4, ou le groupe 2,2-diméthyl-3-(2,2-dichlorovinyl)-cyclopropyle,
un groupe phényle non substitué ou portant un à trois substituants choisis parmi les atomes d'halogènes, les groupes alkyle en C1-C6, alcoxy en C1-C6, alkylthio en C1-C4, phényle, nitro, halogénoalkyle en C1-C4, et amino ou par un groupe difluorométhylène-dioxy dont les atomes d'oxygène sont situés sur deux atomes de carbone voisins, ou bien un groupe benzyle non substitué ou portant un à trois substituants choisis parmi les atomes d'halogènes, les groupes alkyle en C1-C6, alcoxy en C1-C6, alkylthio en C1-C4, phényle, nitro, halogénoalkyle en C1-C4 et amino, ces deux derniers substituants ne pouvant se trouver que sur des atomes de carbone cycliques, caractérisé en ce que :

on traite des composés de formule II

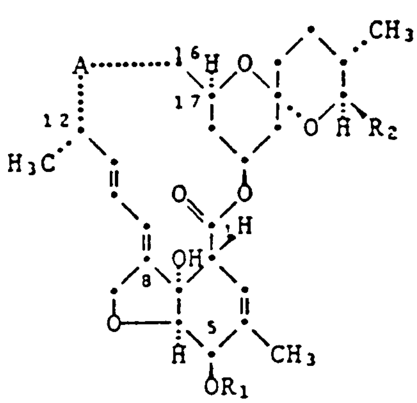

(II)

dans laquelle A représente un groupe a, b ou c

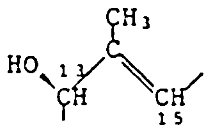 , 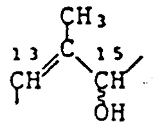 ou 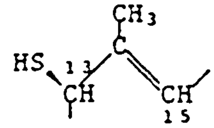 ,

(a) (b) (c)

[= 13β-Hydroxy-$\Delta^{14,15}$] [= $\Delta^{13,14}$-15-Hydroxy] [= 13β-Mercapto-$\Delta^{14,15}$]

$R_1$ représente un groupe protecteur et $R_2$ a les significations indiquées en référence à la formule I, par un réactif approprié à l'introduction ou a la formation d'un groupe 13 bêta-thiolester, après quoi, si l'on désire des composés à groupe 5-hydroxy libres, on élimine le groupe protecteur $R_1$.

**12.** Procédé selon la revendication 11, caractérisé en ce que pour l'introduction ou la formation d'un groupe 13 bêta-thiolester dans les composés de formule II, on utilise en tant que réactif
i) dans le cas des composés de formules IIa ou IIb :
a) un acide thiocarboxylique de formule III

RCOSH (III)

ou bien
b) un thioamide de formule IV

$RCSN(alkyl)_2$ (IV)

dans laquelle les groupes alkyle contiennent 1 à 4 atomes de carbone et consistent de préférence en groupes méthyle,
ou bien
ii) dans le cas des composés de formule IIc :
c) un halogénure d'acide de formule V

RCOhal (V)

dans laquelle hal représente un halogène, de préférence le chlore ou le brome,
ou bien
d) un anhydride de formule VI

$(RCO)_2O$ (VI)

dans laquelle R a les significations indiquées en référence à la formule I.

**13.** Procédé selon la revendication 12, caractérisé en ce que la réaction des composés de formule IIa ou IIb avec un thioacide de formule III ou un thioamide de formule IV est effectuée en présence d'un orthoester de formule VII

$R_3C(OR_4)_3$ (VII)

dans laquelle $R_3$ représente l'hydrogène ou un groupe alkyle en C1-C4 et $R_4$ représente un groupe alkyle en C1-C4, et également en présence d'un acide ayant une activité catalytique, à des températures dans l'intervalle de 0 à 150 ° C.

**14.** Procédé selon la revendication 12, caractérisé en ce que la réaction des composés de formule IIc avec un chlorure d'acide ou un bromure d'acide de formule V ou un anhydride de formule VI est effectuée dans un solvant inerte dans la réaction à des températures de -20 à 100 ° C.

**15.** Produit pour combattre les parasites, caractérisé en ce qu'il contient, avec des véhicules, des diluants ou des matières de charge, au moins une substance active consistant en un composé de formule I de la revendication 1.

**16.** Procédé pour combattre les parasites, caractérisé en ce que l'on applique une quantité pesticide efficace d'au moins un composé de formule I de la revendication 1 sur ou dans la plante hôte ou autres habitats des parasites, à l'exception du corps humain ou animal.

**17.** Procédé selon la revendication 16, caractérisé en ce que les parasites qu'on combat sont des endo- ou des ecto-parasites infestant des animaux.

**18.** Procédé selon la revendication 16, caractérisé en ce que les parasites qu'on combat sont des parasites des végétaux.

**19.** Composés de formule I de la revendication 1 pour l'utilisation en tant qu'agents anti-parasitaires contre les endo- et ecto-parasites infectant les animaux.

**20.** Utilisation des composés de formule I de la revendication 1, pour la préparation d'un médicament pour la lutte contre les endo- et ecto-parasites infectant les animaux.

**Revendications pour l'Etat contractant suivant : AT**

**1.** Produit pour combattre les parasites, caractérisé en ce qu'il contient, avec des véhicules, des matières de charge ou des matières à la fois véhicules et matières de charge, au moins une substance active consistant en un composé de formule I

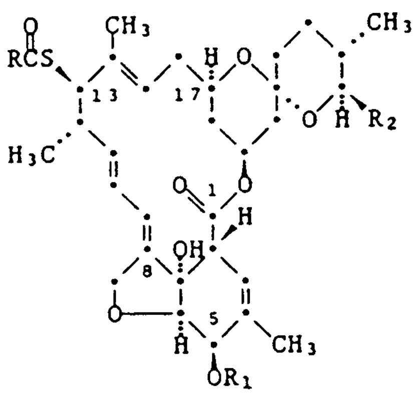

(I)

dans laquelle

$R_1$ représente l'hydrogène, un groupe silyle ou acyle,

$R_2$ représente un groupe méthyle, éthyle, isopropyle ou sec-butyle, et

R représente l'hydrogène,

un groupe alkyle à chaîne droite ou ramifiée en C1-C18 non substitué ou substitué par 1 à 7 atomes d'halogènes, un à six groupes alcoxy en C1-C6, un groupe phénoxy non substitué ou halogéné, un groupe alkylthio en C1-C4, un groupe (alkyle en C1-C4)-carbonyle ou un groupe cycloalkyle en C3-C6 non substitué ou portant un ou plusieurs substituants méthyle,

un groupe alcényle en C2-C6 non substitué ou substitué par 1 à 7 atomes d'halogènes ou un à six groupes alcoxy en C1-C6,

un groupe alcynyle en C2-C6 non substitué ou substitué par 1 à 7 atomes d'halogènes ou un à six groupes alcoxy en C1-C6,

un groupe cycloaliphatique mono- à tétra-cyclique en C3-C10 ou un groupe cycloalkyle en C3-C10, substitué par 1 à 7 atomes d'halogènes, un à six groupes alcoxy en C1-C6 ou par des groupes alkyle en C1-C4, ou un groupe 2,2-diméthyl-3-(2,2-dichlorovinyl)-cyclopropyle,

un groupe phényle non substitué ou portant un à trois substituants choisis parmi les atomes d'halogènes, les groupes alkyle en C1-C6, alcoxy en C1-C6, alkylthio en C1-C4, phényle,

nitro, halogénoalkyle en C1-C4 et amino ou par un groupe difluorométhylène-dioxy dont les atomes d'oxygène sont situés sur deux atomes de carbone voisins, ou bien
un groupe benzyle non substitué ou portant un à trois substituants choisis parmi les atomes d'halogènes, les groupes alkyle en C1-C6, alcoxy en C1-C6, alkylthio en C1-C4, phényle, nitro, halogénoalkyle en C1-C4 et amino, ces deux derniers substituants ne pouvant se trouver que sur des atomes de carbone cycliques.

**2.** Produit selon la revendication 1, caractérisé en ce qu'il contient un composé de formule I dans laquelle les groupes alkyle, cycloalkyle, alcényle et alcynyle mentionnés en référence à R sont substitués par 1 à 7 atomes d'halogènes ou un à six groupes alcoxy en C1-C6 et les groupes phényle portent un à trois substituants choisis parmi les atomes d'halogènes, les groupes alkyle en C1-C6, alcoxy en C1-C6, alkylthio en C1-C4, phényle ou nitro, et $R_1$ et $R_2$ ont les significations indiquées en référence à la formule I dans la revendication 1.

**3.** Produit selon la revendication 1, caractérisé en ce qu'il contient un composé de formule I dans laquelle $R_1$ représente l'hydrogène, $R_2$ représente un groupe méthyle, éthyle, isopropyle ou sec-butyle et R représente l'hydrogène, un groupe alkyle en C1-C6, alcényle en C2-C4, alcynyle en C2-C4 ou cycloalkyle en C3-C6 non substitué ou substitué par 1 à 4 atomes d'halogènes ou groupes alcoxy en C1-C4 ; ou un groupe phényle non substitué ou portant un à trois substituants choisis parmi les halogènes, les groupes alkyle en C1-C4, alcoxy en C1-C4, alkylthio en C1-C4 ou nitro.

**4.** Produit selon la revendication 1, caractérisé en ce qu'il contient un composé de formule I dans laquelle $R_1$ représente l'hydrogène, $R_2$ un groupe méthyle ou éthyle, et R un groupe alkyle en C1-C7 non substitué ou monosubstitué par un halogène ou un groupe alcoxy en C1-C4, un groupe phényle monosubstitué par un groupe trifluorométhyle ou par le groupe $-O-CF_2-O-$ dont les deux atomes d'oxygène sont situés sur deux atomes de carbone cycliques voisins, ou un groupe alpha-méthyl-ou alpha,alpha-diméthyl-benzyle.

**5.** Produit selon la revendication 1, caractérisé en ce qu'il contient un composé de formule I dans laquelle $R_1$ représente l'hydrogène, $R_2$ représente un groupe méthyle ou éthyle et R un groupe alkyle en C1-C7, un groupe alkyle en C1-C4 monosubstitué par le chlore, le fluor ou un groupe méthoxy, un groupe phényle monosubstitué par un groupe trifluorométhyle ou par le groupe $-O-CF_2-O-$ dont les deux atomes d'oxygène sont situés sur deux atomes de carbone cycliques voisins, ou un groupe alpha-méthyl ou alpha,alpha-diméthyl-benzyle.

**6.** Produit selon la revendication 1, caractérisé en ce qu'il contient un composé de formule I dans laquelle R représente un groupe tert-butyle et $R_1$ et $R_2$ ont les significations indiquées en référence à la formule I dans la revendication 1.

**7.** Produit selon la revendication 1, caractérisé en ce qu'il contient un composé de formule I dans laquelle $R_1$ représente l'hydrogène, $R_2$ un groupe méthyle, éthyle ou isopropyle et R un groupe tert-butyle.

**8.** Produit selon la revendication 1, caractérisé en ce qu'il contient un composé de formule I dans laquelle $R_1$ représente le groupe tert-butyl-diméthylsilyle.

**9.** Produit selon la revendication 1, caractérisé en ce qu'il contient un composé de formule I choisi dans le groupe suivant :
13bêta-formylthio-milbémycine D
13bêta-acétylthio-milbémycine D
13bêta-pivaloylthio-milbémycine D
13bêta-formylthio-milbémycine $A_3$
13bêta-acétylthio-milbémycine $A_3$
13bêta-pivaloylthio-milbémycine $A_3$
13bêta-formylthio-milbémycine $A_4$
13bêta-acétylthio-milbémycine $A_4$
13bêta-pivaloylthio-milbémycine $A_4$
13bêta-(2'-méthoxy-2'-méthylpropionylthio)-milbémycine D
13bêta-(2'-méthoxy-2'-méthylpropionylthio)-milbémycine $A_4$

13bêta-trichloracétylthio-milbémycine $A_4$
13bêta-(4'-chloro-butanoylthio)milbémycine $A_4$
13bêta-trichloracryloylthio-milbémycine $A_4$
13bêta-cyclopropane-carbonylthio-milbémycine $A_4$
13bêta-cyclobutane-carbonylthio-milbémycine $A_4$
13bêta-heptanoylthio-milbémycine $A_4$
13bêta-(3'-chloro-2',2'-diméthyl-propionylthio)-milbémycine $A_4$
13bêta-(3'-chloro-2',2'-diméthyl-propionylthio)-milbémycine $A_3$
13bêta-(1'-méthyl-cyclopropane-carbonylthio)-milbémycine $A_4$
13bêta-(1'-méthyl-cyclopropane-carbonylthio)-milbémycine $A_3$
13bêta-(1-adamantane-carbonylthio)-milbémycine $A_4$
13bêta-(p-fluorophénoxyacétylthio)-milbémycine $A_4$
13bêta-(2'-chloro-2'-méthyl-propionylthio)-milbémycine $A_4$
13bêta-(2',2'-dichloropropionylthio)-milbémycine $A_4$
13bêta-(2',2'-diméthylbutanoylthio)-milbémycine $A_4$
13bêta-(3',3'-diméthylbutanoylthio)-milbémycine $A_4$
13bêta-(2',2',3',3'-tétraméthylbutanoylthio)-milbémycine $A_4$
13bêta-(p-chlorobenzoylthio)-milbémycine $A_4$
13bêta-(3',3',3'-trifluoropropionylthio)-milbémycine $A_4$
13bêta-chloroacétylthio-milbémycine $A_4$
13bêta-(2'-chloro-3',3',3'-trifluoropropionylthio)-milbémycine $A_4$
13bêta-(3',3',3'-trifluoropropionylthio)-milbémycine $A_4$
13bêta-[o-(trifluorométhyl)-benzoylthio]-milbémycine $A_4$
13bêta-(alpha,alpha-diméthylbenzylcarbonylthio)-milbémycine $A_4$
13bêta-(2-n-propyl-n-valéroylthio)-milybémcyine $A_4$
13bêta-[(2,3-difluorométhylène-dioxy)-benzoylthio]-milbémycine $A_4$
13bêta-(alpha-méthylbenzylcarbonylthio)-milbémycine $A_4$ et
13bêta-(méthoxyacétylthio)-milbémycine $A_4$.

**10.** Produit selon la revendication 1, caractérisé en ce qu'il contient un composé de formule I choisi dans le groupe suivant :
5-O-tert-butyldiméthylsilyl-13bêta-formylthio-milbémycine D
5-O-tert-butyldiméthylsilyl-13bêta-acétylthio-milbémycine D
5-O-tert-butyldiméthylsilyl-13bêta-pivaloylthio-milbémycine D
5-O-tert-butyldiméthylsilyl-13bêta-formylthio-milbémycine $A_3$
5-O-tert-butyldiméthylsilyl-13bêta-acétylthio-milbémycine $A_3$
5-O-tert-butyldiméthylsilyl-13bêta-pivaloylthio-milbémycine $A_3$
5-O-tert-butyldiméthylsilyl-13bêta-formylthio-milbémycine $A_4$
5-O-tert-butyldiméthylsilyl-13bêta-acétylthio-milbémycine $A_4$
5-O-tert-butyldiméthylsilyl-13bêta-pivaloylthio-milbémycine $A_4$
5-O-tert-butyldiméthylsilyl-13bêta-(2'-méthoxy-2'-méthylpropionylthio)-milbémycine D
5-O-tert-butyldiméthylsilyl-13bêta-(2'-méthoxy-2'-méthylpropionylthio)-milbémycine $A_4$
5-O-tert-butyldiméthylsilyl-13bêta-trichloracétylthio-milbémycine $A_4$
5-O-tert-butyldiméthylsilyl-13bêta-(4'-chloro-butanoylthio)-milbémycine $A_4$
5-O-tert-butyldiméthylsilyl-13bêtatrichloracryloylthio-milbémycine $A_4$
5-O-tert-butyldiméthylsilyl-13bêta-cyclopropane-carbonylthio-milbémycine $A_4$
5-O-tert-butyldiméthylsilyl-13bêta-cyclobutane-carbonylthio-milbémycine $A_4$
5-O-tert-butyldiméthylsilyl-13bêta-heptanoylthio-milbémycine $A_4$
5-O-tert-butyldiméthylsilyl-13bêta-(3'-chloro-2',2', diméthyl-propionylthio)-milbémycine $A_4$
5-O-tert-butyldiméthylsilyl-13bêta-(3'-chloro-2',2'-diméthyl-propionylthio)-milbémycine $A_3$
5-O-tert-butyldiméthylsilyl-13bêta-(1'-méthyl-cyclopropane-carbonylthio)-milbémycine $A_4$
5-O-tert-butyldiméthylsilyl-13bêta-(1'-méthyl-cyclopropane-carbonylthio)-milbémycine $A_3$
5-O-tert-butyldiméthylsilyl-13bêta-(1-adamantanecarbonylthio)-milbémycine $A_4$
5-O-tert-butyldiméthylsilyl-13bêta-(p-fluorophénoxyacétylthio)-milbémycine $A_4$
5-O-tert-butyldiméthylsilyl-13bêta-(2'-chloro-2'-méthylpropionylthio)-milbémycine $A_4$
5-O-tert-butyldiméthylsilyl-13bêta-(2',2'-dichloropropionylthio)-milbémycine $A_4$
5-O-tert-butyldiméthylsilyl-13bêta-(2',2'-diméthylbutanoylthio)-milbémycine $A_4$
5-O-tert-butyldiméthylsilyl-13bêta-(3',3'-diméthylbutanoylthio)-milbémycine $A_4$

5-O-tert-butyldiméthylsilyl-13bêta-(2',2',3',3'-tétraméthylbutanoylthio)-milbémycine $A_4$
5-O-tert-butyldiméthylsilyl-13bêta-(p-chlorobenzoylthio)-milbémycine$A_4$
5-O-tert-butyldiméthylsilyl-13bêta-(3',3',3'-trifluoropropionylthio)-milbémycine $A_4$
5-O-tert-butyldiméthylsilyl-13bêta-chloracétylthio-milbémycine $A_4$
5-O-tert-butyldiméthylsilyl-13bêta-(2'-chloro-3',3',3'-trifluoro-propionylthio)-milbémycine $A_4$
5-O-tert-butyldiméthylsilyl-13bêta-(3',3',3'-trifluoropropionylthio)-milbémycine $A_4$
5-O-tert-butyldiméthylsilyl-13bêta-(alpha-méthylbenzylcarbonylthio)-milbémycine $A_4$
5-O-tert-butyldiméthylsilyl-13bêta-(méthoxyacétylthio)-milbémycine $A_4$
5-O-tert-butyldiméthylsilyl-13bêta-[o-(trifluorométhyl)-benzoylthio]-milbémycine $A_4$
5-O-tert-butyldiméthylsilyl-13bêta-(alpha,alpha-diméthylbenzylcarbonylthio)-milbémycine $A_4$
5-O-tert-butyldiméthylsilyl-13bêta-(2-n-propyl-n-valeroylthio)-milbémycine $A_4$ et
5-O-tert-butyldiméthylsilyl-13bêta-[(2,3-difluorométhylène-dioxy)-benzoylthio]-milbémycine $A_4$.

**11.** Procédé de préparation des composés de formule I

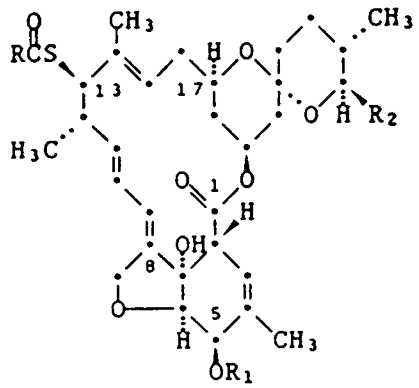

(I)

dans laquelle

$R_1$ représente l'hydrogène, un groupe silyle ou acyle,

$R_2$ représente un groupe méthyle, éthyle, isopropyle ou sec-butyle, et

R représente l'hydrogène,
un groupe alkyle à chaîne droite ou ramifiée en C1-C18, non substitué ou substitué par 1 à 7 atomes d'halogènes, un à six groupes alcoxy en C1-C6, un groupe phénoxy non substitué ou halogéné, un groupe alkylthio en C1-C4, un groupe (alkyle en C1-C4)-carbonyle ou un groupe cycloalkyle en C3-C6 non substitué ou portant un ou plusieurs substituants méthyle,
un groupe alcényle en C2-C6 non substitué ou substitué par 1 à 7 atomes d'halogènes ou un à six groupes alcoxy en C1-C6,
un groupe alcynyle en C2-C6 non substitué ou substitué par 1 à 7 atomes d'halogènes ou un à six groupes alcoxy en C1-C6,
un groupe cycloaliphatique mono- à tétra-cyclique en C3-C10 ou un groupe cycloalkyle en C3-C10 substitué par 1 à 7 atomes d'halogènes, un à six groupes alcoxy en C1-C6 ou par des groupes alkyle en C1-C4, ou le groupe 2,2-diméthyl-3-(2,2-dichlorovinyl)-cyclopropyle,
un groupe phényle non substitué ou portant un à trois substituants choisis parmi les atomes d'halogènes, les groupes alkyle en C1-C6, alcoxy en C1-C6, alkylthio en C1-C4, phényle, nitro, halogénoalkyle en C1-C4, et amino ou par un groupe difluorométhylène-dioxy dont les atomes d'oxygène sont situés sur deux atomes de carbone voisins, ou bien
un groupe benzyle non substitué ou portant un à trois substituants choisis parmi les atomes d'halogènes, les groupes alkyle en C1-C6, alcoxy en C1-C6, alkylthio en C1-C4, phényle, nitro, halogénoalkyle en C1-C4 et amino, ces deux derniers substituants ne pouvant se trouver que sur des atomes de carbone cycliques, caractérisé en ce que :

on traite des composés de formule II

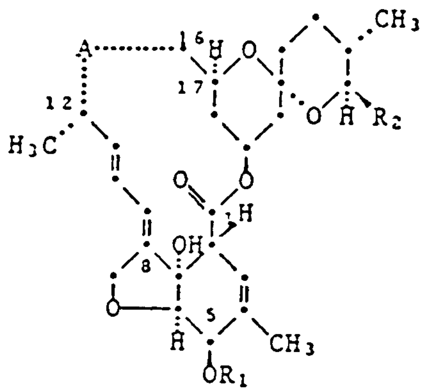

(II)

dans laquelle A représente un groupe a, b ou c

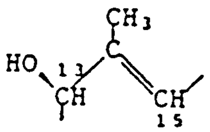 , 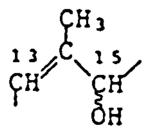 ou 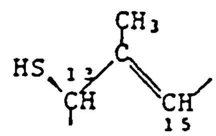 ,

(a) (b) (c)

[= 13β-Hydroxy-$\Delta^{14,15}$] [= $\Delta^{13,14}$-15-Hydroxy] [= 13β-Mercapto-$\Delta^{14,15}$]

$R_1$ représente un groupe protecteur et $R_2$ a les significations indiquées en référence à la formule I, par un réactif approprié à l'introduction ou à la formation d'un groupe 13 bêta-thiolester, après quoi, si l'on désire des composés à groupe 5-hydroxy libres, on élimine le groupe protecteur $R_1$.

**12.** Procédé selon la revendication 11, caractérisé en ce que pour l'introduction ou la formation d'un groupe 13 bêta-thiolester dans les composés de formule II, on utilise en tant que réactif

i) dans le cas des composés de formules IIa ou II b :

a) un acide thiocarboxylique de formule III

RCOSH (III)

ou bien

b) un thioamide de formule IV

$RCSN(alkyl)_2$ (IV)

dans laquelle les groupes alkyle contiennent 1 à 4 atomes de carbone et consistent de préférence en groupes méthyle,

ou bien

ii) dans le cas des composés de formule IIc :

c) un halogénure d'acide de formule V

RCOhal (V)

dans laquelle hal représente un halogène, de préférence le chlore ou le brome,

ou bien

d) un anhydride de formule VI

$(RCO)_2O$ (VI)

dans laquelle R a les significations indiquées en référence à la formule I.

**13.** Procédé selon la revendication 12, caractérisé en ce que la réaction des composés de formule IIa ou IIb avec un thioacide de formule III ou un thioamide de formule IV est effectuée en présence d'un orthoester de formule VII

$R_3C(OR_4)_3$ (VII)

dans laquelle $R_3$ représente l'hydrogène ou un groupe alkyle en C1-C4 et $R_4$ représente un groupe alkyle en C1-C4, et également en présence d'un acide ayant une activité catalytique, à des températures dans l'intervalle de 0 à 150°C.

**14.** Procédé selon la revendication 12, caractérisé en ce que la réaction des composés de formule IIc avec un chlorure d'acide ou un bromure d'acide de formule V ou un anhydride de formule VI est effectuée dans un solvant inerte dans la réaction à des températures de -20 à 100°C.

**15.** Procédé pour combattre les parasites, caractérisé en ce que l'on applique une quantité pesticide efficace d'au moins un composé de formule I de la revendication 1 sur ou dans la plante hôte ou autres habitats des parasites, à l'exception du corps humain ou animal.

**16.** Procédé selon la revendication 15, caractérisé en ce que les parasites qu'on combat sont des endo- ou des ecto-parasites infestant des animaux.

**17.** Procédé selon la revendication 15, caractérisé en ce que les parasites qu'on combat sont des parasites des végétaux.

**18.** Composés de formule I de la revendication 1 pour l'utilisation en tant qu'agents anti-parasitaires contre les endo- et ecto-parasites infectant les animaux.

**19.** Utilisation des composés de formule I de la revendication 1, pour la préparation d'un médicament pour la lutte contre les endo- et ecto-parasites infectant les animaux.